# EUROPEAN PATENT APPLICATION

(11) **EP 1 323 726 A1**
(43) Date of publication of application: **02.07.2003**
(21) Application number: 01970229.9
(22) Date of filing: 26.09.2001
(51) Int. Cl.: C07H 17/08, A61K 31/7048, A61P 31/04

(54) **ERYTHROMYCIN DERIVATIVES AND MEDICINES CONTAINING THE SAME**

(30) Priority: 27.09.2000 JP 2000293754; 07.03.2001 JP 2001063034
(71) Applicant: HOKURIKU SEIYAKU CO., LTD., Katsuyama-shi, Fukui 911-0813 (JP)
(72) Inventor: KATO, Hideo, Katsuyama-shi Fukui 911-0813 (JP); YOSHIDA, Toshihiko, Katsuyama-shi Fukui 911-0813 (JP); NISHIMOTO, Akemi, Katsuyama-shi Fukui 911-0813 (JP); OHMOTO, Shinobu, Katsuyama-shi Fukui 911-0813 (JP)
(74) Representative: Sternagel, Fleischer, Godemeyer & Partner Patentanwälte
(86) International application number: JP0108388
(87) International publication number: WO02026753

(57) **Abstract**

Erythromycin derivatives or salt thereof having excellent antibacterial activity against atypical acid-fast mycobacteria and useful as antibacterial agent, which is represented by the following general formula: 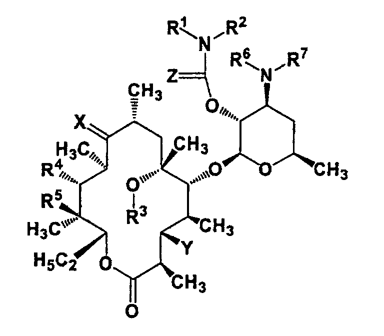 wherein, R¹ and R² represent hydrogen atom, an alkyl group, a homocyclic or heterocyclic group, an alkyl group substituted with a homocyclic group, or an alkyl group substituted with a heterocyclic group or the like, or R¹ and R² may combine to form a homocycle or a heterocycle, R³ represents hydrogen atom or methyl group, R⁴ represents hydroxyl group or the like; R⁵ represents hydrogen atom, hydroxyl group or the like, R⁶ and R⁷ represent hydrogen atom, an alkyl group or the like; X represents NH or a group represented by N-O-R⁸, Y represents a group represented by O-C(=O)-R⁹, a cladinose derivative or the like, Z represents oxygen atom or sulfur atom, R⁸ and R⁹ represents an alkyl group, a homocyclic or heterocyclic group, an alkyl group substituted with a homocyclic group, or an alkyl group substituted with a heterocyclic group.

## Description

### Field of the Invention

The present invention relates to novel erythromycin derivatives or salts thereof as antibacterial agents, which have excellent antibacterial activity especially against atypical acid-fast mycobacteria including multiple drug-resistant bacteria. The present invention also relates to medicaments comprising the same as an active ingredient.

### Related Art

Atypical acid-fast mycobacteria have low sensitivity to available antibacterial agents, and for this reason, atypical acid-fast mycobacteriosis is extremely intractable diseases. Rifampicin (The Merck Index, 12th edition, 8382) and the like are known as compounds that can be applied to similar disease to those treated by the compounds of the present invention. Furthermore, as macrolide derivatives that have a similar chemical structure to that of the compounds of the present invention, clarithromycin (The Merck Index, 12th edition, 2400), roxithromycin (The Merck Index, 12th edition, 8433), and compounds disclosed in Japanese Patent Unexamined Publication (KOKAI) No. 11-236395 are known. Clinical application of clarithromycin has been approved in the United State and other countries, which is considered as the most promising agent for the treatment of atypical acid-fast mycobacteriosis among macrolide derivatives at present. However, antibacterial activity of clarithromycin is also not sufficient as an agent for treatment of atypical acid-fast mycobacteriosis. Therefore, development of more excellent antibacterial agents has been desired.

In recent years, increase of opportunistic infections has become a big social problem. Due to increase of compromised hosts with degraded biophylaxis mechanism such as patients infected by human immunodeficiency virus (HIV), patients of cancer and diabetes, and elderly persons, increase of multiple drug-resistant bacteria whose typical examples are Methicillin-resistant Staphylococcus aureus and the like, microbial substitution of patients by these bacteria and so forth, which are causes of the increase of the opportunistic infections, chemotherapy of opportunistic infections become more difficult. Atypical acid-fast mycobacteriosis is one of the opportunistic infections which have become a problem. Atypical acid-fast mycobacteria, the causal bacteria of the atypical acid-fast mycobacteriosis, proliferate slowly, and even when they are captured by phagocytes, they can survive in the cells for a long period of time. Therefore, prolonged chemotherapy is required to treat infections by these bacteria. In particular, among the atypical acid-fast mycobacteria, almost no effective antibacterial agent is available against *Mycobacterium avium* complex (MAC), and accordingly, surgical treatment for the therapeutic treatment of this infection has also been studied at present. Moreover, even the aforementioned clarithromycin lacks selectivity to the atypical acid-fast mycobacteria, and clarithromycin resistant MACs have already been known. As explained above, various problems arise in chemotherapy of atypical acid-fast mycobacteriosis, for example, low sensitivity to known antibacterial agents, and conditions of high possibility of microbial substitution or emergence of resistant bacteria.

### Disclosure of the Invention

An object of the present invention is to provide a compound that has selective and excellent antibacterial activity against atypical acid-fast mycobacteria.

The inventors of the present invention eagerly conducted researches to achieve the aforementioned object. As a result, they found that the novel erythromycin derivatives or salts thereof according to the present invention were useful as antibacterial agents, and that they had excellent antibacterial activity particularly against atypical acid-fast mycobacteria. The present invention was achieved on the basis of the findings.

The present invention thus relates to novel erythromycin derivatives represented by the following general formula (I) or salts thereof: 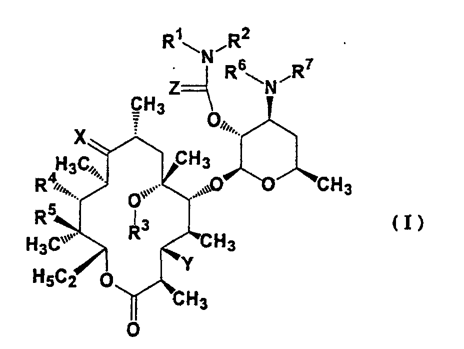 wherein, R¹ and R² independently represent hydrogen atom, an alkyl group which may be substituted, an alkenyl group which may be substituted, an alkynyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, or R¹ and R² may combine together with the nitrogen atom to which they bind to form a saturated or unsaturated heterocyclic group which may further contain one or more heteroatoms selected from the group consisting of oxygen atom, sulfur atom, and nitrogen atom and which may be substituted, R³ represents hydrogen atom or methyl group, R⁵ represents hydrogen atom or hydroxyl group when R⁴ represents hydroxyl group, or R⁴ and R⁵ may combine together with two carbon atoms on the ring to which each of them binds to form a heterocyclic group represented by the following formula (II): 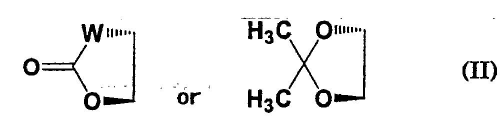 R⁶ and R⁷ independently represent hydrogen atom, an alkyl group which may be substituted, an alkenyl group which may be substituted, an alkynyl group which may be substituted, a cycloalkyl group which may be substituted, an alkyl group substituted with a saturated or unsaturated homocyclic group which may be substituted, or an alkyl group substituted with a saturated or unsaturated heterocyclic group which may be substituted, or R⁶ and R⁷ may bind together with the nitrogen atom to which they bind to form a saturated heterocyclic group which may further contain one or more heteroatoms selected from the group consisting of oxygen atom, sulfur atom, and nitrogen atom and which may be substituted, X represents NH or a group represented by N-O-R⁸, Y represents a group represented by O-C(=O)-R⁹ or O-C(=O)-U-R¹⁰, or represents an oxy group substituted with a heterocyclic group represented by the following formula (III): 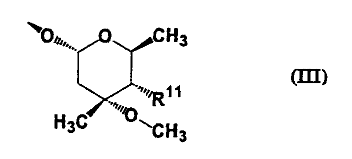 Z represents oxygen atom or sulfur atom, W represents oxygen atom or nitrogen atom which may be substituted, R⁸ and R⁹ independently represents hydrogen atom, an alkyl group which may be substituted, an alkenyl group which may be substituted, an alkynyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, R¹⁰ represents an alkyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, R¹¹ represents hydrogen atom, hydroxyl group, or a group represented by O-C(=O)-R¹² or O-C(=O)-V-R¹³, R¹² and R¹³ independently represent an alkyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, U and V independently represent oxygen atom or a group represented by NH.

According to the second aspect of the present invention, provided is the compound or the salt thereof wherein R³ is hydrogen atom among the compounds represented by the aforementioned general formula (I).

According to the third aspect of the present invention, provided is the compound or the salt thereof wherein Y is the oxy group substituted by the heterocyclic group represented by the aforementioned formula (III) among the compounds represented by the aforementioned general formula (I).

According to the forth aspect of the present invention, provided is the compound or the salt thereof wherein R¹ is hydrogen atom among the compounds represented by the aforementioned general formula (I). It is apparent to one of ordinary skill in the art that the compounds provided may exist as tautomers, and therefore, the compounds may be present in the form represented by the aforementioned general formula (I), or may be present as isomers represented by the following general formula (IV): 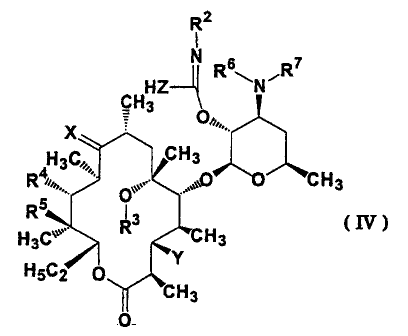 wherein R², R³, R⁴, R⁵, R⁶, R⁷, X, Y, and Z have the same meaning as those defined above.

According to further aspect of the present invention, provided is a medicament which comprises a compound represented by the aforementioned general formula (I) or a salt thereof as an active ingredient. The medicament provided by the present invention can be suitably used as, for example, an antibacterial agent, in particular, an agent for therapeutic or preventive treatment of atypical acid-fast mycobacteriosis.

The present invention further provides a use of the compound represented by the aforementioned general formula (I) or a pharmacologically acceptable salt thereof for the manufacture of the aforementioned medicament; and a method for therapeutic treatment of infectious diseases, in particular a method for therapeutic treatment of atypical acid-fast mycobacteriosis which comprises the step of administering to a mammal including a human a therapeutically effective amount of a compound represented by the aforementioned general formula (I) or a pharmacologically acceptable salt thereof.

### Best Mode for Carrying out the Invention

In the aforementioned general formula (I) according to the present invention, the alkyl group, defined as "an alkyl group which may be substituted" represented by R¹, R², R⁶, R⁷, R⁸, R⁹, R¹⁰, R¹², and R¹³, means a linear or branched alkyl group having 1 to 14 carbon atoms. Examples include methyl group, ethyl group, n-propyl group, isopropyl group, n-butyl group, isobutyl group, sec-butyl group, tert-butyl group, n-pentyl group, isopentyl group, neopentyl group, tert-pentyl group, 1-methylbutyl group, 2-methylbutyl group, 1-ethylpropyl group, n-hexyl group, 1-methylpentyl group, 2-methylpentyl group, 3-methylpentyl group, 4-methylpentyl group, 2,3-dimethylbutyl group, 1,3-dimethylbutyl group, 1,2-dimethylbutyl group, 1-ethylbutyl group, 2-ethylbutyl group, 1-isopropylpropyl group, n-heptyl group, n-octyl group, n-nonyl group, n-decyl group, n-undecyl group, n-dodecyl group, n-tridecyl group, n-tetradecyl group and the like.

In the aforementioned general formula (I) according to the present invention, the alkenyl group, defined as "an alkenyl group which may be substituted" represented by R¹, R², R⁶, R⁷, R⁸, and R⁹, means a linear or branched alkenyl group or alkapolyenyl group having 2 to 14 carbon atoms and one or more double bonds at any positions. Examples include vinyl group, allyl group, 1-methylethenyl group, propenyl group, butenyl group, butadienyl group, pentenyl group, isoprenyl group, 4-methylpentenyl group, hexenyl group, hexadienyl group, hexatrienyl group, heptenyl group, octenyl group, nonenyl group, decenyl group, undecenyl group, dodecenyl group, dodecadienyl group, tridecenyl group, tetradecenyl group, geranyl group, myrcenyl group, ocimenyl group, neryl group, linaloyl group, citronellyl group and the like. The alkynyl group, defined as "an alkynyl group which may be substituted" represented by R¹, R², R⁶, R⁷, R⁸, and R⁹, means a linear or branched alkynyl group or alkapolyynyl group having 2 to 14 carbon atoms and one or more triple bonds at any positions. Examples include ethynyl group, propynyl group, butynyl group, 1-methyl-2-propynyl group, pentynyl group, hexynyl group, hexadiynyl group, heptynyl group, octynyl group, nonynyl group, decynyl group, undecynyl group, dodecynyl group, tridecynyl group, tetradecynyl group and the like.

In the aforementioned general formula (I) according to the present invention, the saturated or unsaturated homocyclic group or saturated or unsaturated heterocyclic group represented by R¹, R², R⁸, R⁹, R¹⁰, R¹², and R¹³ may be monocyclic or polycyclic group. Examples include cyclopropyl group, cyclobutyl group, cyclopentyl group, cyclohexyl group, bicyclo[3.2.1]octyl group, bicyclo[5.2.0]nonyl group, aziridinyl group, azetidinyl group, pyrrolidinyl group, piperidinyl group, hexahydro-1H-azepinyl group, piperazinyl group, pyrazolidinyl group, imidazolidinyl group, morpholinyl group, thiomorpholinyl group, tetrahydropyranyl group, tetrahydrothiopyranyl group, phenyl group, naphthyl group, pyridyl group, pyrimidyl group, pyrazinyl group, imidazolyl group, oxazolyl group, isoxazolyl group, thiazolyl group, isothiazolyl group, furyl group, thienyl group, pyrrolyl group, benzofuranyl group, benzo[b]thienyl group, benzimidazolyl group, indolyl group, quinolyl group, isoquinolyl group, 1,2,3,4-tetrahydronaphthyl group, benzopyrrolidinyl group, cyclohexenyl group and the like. The saturated or unsaturated homocyclic group or saturated or unsaturated heterocyclic group represented by R¹, R², R⁸, R⁹, R¹⁰, R¹², and R¹³ may be substituted.

In the aforementioned general formula (I) according to the present invention, the alkyl group substituted with a homocyclic group or the alkyl group substituted with a heterocyclic group represented by R¹, R², R⁶, R⁷, R⁸, R⁹, R¹⁰, R¹², and R¹³ means a group formed by substitution of the aforementioned saturated or unsaturated homocyclic group or saturated or unsaturated heterocyclic group (the homocyclic group or the heterocyclic group may be substituted) on the aforementioned linear or branched alkyl group having 1 to 14 carbon atoms. Examples include cyclopropylmethyl group, cyclobutylmethyl group, cyclopentylmethyl group, cyclohexylmethyl group, cyclohexylethyl group, cyclohexylpropyl group, cyclohexylbutyl group, cyclohexylpentyl group, cyclohexylhexyl group, cyclohexylheptyl group, cyclohexyloctyl group, cyclohexylnonyl group, cyclohexyldecyl group, cyclohexylundecyl group, cyclohexyldodecyl group, cyclohexyltridecyl group, cyclohexyltetradecyl group, bicyclo[3.2.1]octylmethyl group, bicyclo[5.2.0]nonylmethyl group, aziridinylmethyl group, azetidinylmethyl group, pyrrolidinylmethyl group, pyrrolidinylethyl group, pyrrolidinylhexyl group, pyrrolidinyltetradecyl group, piperidinylmethyl group, piperidinylethyl group, piperidinylpropyl group, piperidinylhexyl group, piperidinyltetradecyl group, hexahydro-1H-azepinylmethyl group, piperazinylmethyl group, piperazinylethyl group, piperazinylpropyl group, morpholinylmethyl group, morpholinylethyl group, morpholinylpropyl group, thiomorpholinylmethyl group, thiomorpholinylethyl group, tetrahydropyranylmethyl group, tetrahydropyranylethyl group, tetrahydrothiopyranylmethyl group, tetrahydrothiopyranylethyl group, (2,3-dihydrobenzofuran-2-yl)methyl group, (2,3-dihydrobenzofuran-2-yl)ethyl group, (3,4-dihydrobenzo[b]pyran-2-yl)methyl group, (3,4-dihydrobenzo[b]pyran-2-yl)ethyl group, (2,3-dihydro-1,4-benzodioxin-2-yl)methyl group, (2,3-dihydro-1,4-benzodioxin-2-yl)ethyl group, benzyl group, phenethyl group, α-methylbenzyl group, phenylpropyl group, phenylbutyl group, phenylpentyl group, phenylhexyl group, phenyltetradecyl group, naphthylmethyl group, naphthylethyl group, pyridylmethyl group, pyridylethyl group, pyridylbutyl group, pyridyldodecyl group, pyrimidylmethyl group, pyrazinylmethyl group, imidazolylmethyl group, imidazolylethyl group, imidazolylbutyl group, oxazolylmethyl group, isoxazolylmethyl group, thiazolylmethyl group, thiazolylbutyl group, isothiazolylmethyl group, furylmethyl group, furylethyl group, thenyl group, thienylethyl group, pyrrolylmethyl group, pyrrolylethyl group, benzofuranylmethyl group, benzofuranylethyl group, benzo[b]thienylmethyl group, benzo[b]thienylethyl group, benzimidazolylmethyl group, benzimidazolylethyl group, indolylmethyl group, indolylethyl group, quinolylmethyl group, quinolylethyl group, isoquinolylmethyl group, isoquinolylethyl group, 1,2,3,4-tetrahydronaphthylmethyl group, 1,2,3,4-tetrahydronaphthylethyl group, cyclohexenylethyl group and the like.

In the aforementioned general formula (I) according to the present invention, when R¹ and R², together with the nitrogen atom to which each of them binds, form the saturated or unsaturated heterocyclic group which may further contain one or more heteroatoms selected from the group consisting of oxygen atom, sulfur atom, and nitrogen atom and which may be substituted, said heterocyclic group may be monocyclic or polycyclic group. Examples include aziridinyl group, azetidinyl group, pyrrolidinyl group, piperidinyl group, hexahydro-1H-azepinyl group, piperazinyl group, morpholinyl group, thiomorpholinyl group, pyrazolidinyl group, imidazolidinyl group, imidazolyl group, pyrrolyl group, benzimidazolyl group, indolyl group, isoindolyl group, pyrrolinyl group, indolinyl group, isoindolinyl group, pyrazolyl group, tetrahydroquinolyl group, tetrahydroisoquinolyl group, decahydroquinolyl group, decahydroisoquinolyl group and the like.

In the aforementioned general formula (I) according to the present invention, the cycloalkyl group represented by R⁶ and R⁷ means a cycloalkyl group having 3 to 6 carbon atoms. Examples include cyclopropyl group, cyclobutyl group, cyclopentyl group, cyclohexyl group and the like. The cycloalkyl group may be substituted. When R⁶ and R⁷, together with the nitrogen atom to which each of them binds, form the saturated or unsaturated heterocyclic group which may further contain one or more heteroatoms selected from the group consisting of oxygen atom, sulfur atom, and nitrogen atom and which may be substituted, examples of the heterocyclic group include aziridinyl group, azetidinyl group, pyrrolidinyl group, piperidinyl group, hexahydro-1H-azepinyl group, piperazinyl group, morpholinyl group, thiomorpholinyl group, pyrazolidinyl group, imidazolidinyl group,

In the aforementioned general formula (I) according to the present invention, when the defined group is referred to as "which may be substituted", any substitutable groups may optionally present on said group. The number, kind, and substituting position of the substituents are not particularly limited. When two or more substituents exist, they may be the same or different. Examples of the substituents include oxo group, hydroxyl group which may be protected, an alkoxyl group which may be substituted, alkylthio group, an amino group which may be substituted, a carbamoyl group which may be substituted, an aryloxy group which may be substituted, an arylthio group, an aralkyloxy group, an aralkylthio group, a halogen atom, an alkyl group, trifluoromethyl group, an acyl group, a cycloalkyl group, a cycloalkenyl group, a saturated heterocyclic group which may be substituted, an aryl group which may be substituted, an aralkyl group which may be substituted, cyano group, nitro group, guanidino group, amidino group, sulfamoyl group, carboxyl group, an alkoxycarbonyl group, an aryloxycarbonyl group, an aralkyloxycarbonyl group, an alkylsulfinyl group, an arylsulfinyl group, an aralkylsulfinyl group, an alkylsulfonyl group, an arylsulfonyl group, an aralkylsulfonyl group and the like.

As the protective group of the hydroxyl group, any group may be used so far that the group is substantially inert in a reaction system where a hydroxyl group should not participate in a reaction and the group is readily cleavable under a condition of a specific deprotective reaction. Examples include an alkanoyl group, a trialkylsilyl group, benzyl group, benzyloxycarbonyl group and the like. Examples of the alkanoyl group as the hydroxyl protective group include formyl group, acetyl group, propionyl group, butyryl group, pivaloyl group and the like. Examples of the trialkylsilyl group as the hydroxyl protective group include trimethylsilyl group, triethylsilyl group and the like. The above alkoxyl group which may be substituted means a linear or branched alkoxyl group which may be substituted. Examples include methoxy group, ethoxy group, n-propoxy group, isopropoxy group, n-butoxy group, isobutoxy group, sec-butoxy group, tert-butoxy group, n-pentyloxy group, isopentyloxy group, neopentyloxy group, tert-pentyloxy group, n-hexyloxy group, n-heptyloxy group, n-octyloxy group, n-nonyloxy group, n-decyloxy group, n-undecyloxy group, n-dodecyloxy group, n-tridecyloxy group, n-tetradecyloxy group, methoxyethoxy group and the like. The above alkylthio group means a linear or branched alkylthio group. Examples include methylthio group, ethylthio group, n-propylthio group, isopropylthio group, n-butylthio group, isobutylthio group, sec-butylthio group, tert-butylthio group, n-pentylthio group, isopentylthio group, neopentylthio group, tert-pentylthio group, n-hexylthio group, n-heptylthio group, n-octylthio group, n-nonylthio group, n-decylthio group, n-undecylthio group, n-dodecylthio group, n-tridecylthio group, n-tetradecylthio group and the like.

Examples of the above optionally substituted amino group include amino group, methylamino group, ethylamino group, n-propylamino group, isopropylamino group, n-butylamino group, isobutylamino group, sec-butylamino group, tert-butylamino group, n-pentylamino group, isopentylamino group, neopentylamino group, tert-pentylamino group, n-hexylamino group, n-dodecyl amino group, n-tetradecylamino group, anilino group, benzylamino group, phenethylamino group, phenylpropylamino group, phenylhexylamino group, phenyldodecylamino group, phenyltetradecylamino group, pyridylmethylamino group, dimethylamino group, diethylamino group, dibenzylamino group, N-ethyl-N-methylamino group, N-methylanilino group, N-benzyl-N-methylamino group, acetylamino group, propionylamino group, tert-butoxycarbonylamino group, benzyloxycarbonylamino group and the like. Examples of the optionally substituted carbamoyl group include carbamoyl group, N-methylcarbamoyl group, N-ethylcarbamoyl group, N-n-propylcarbamoyl group, N-isopropylcarbamoyl group, N-n-butylcarbamoyl group, N-isobutylcarbamoyl group, N-sec-butylcarbamoyl group, N-tert-butylcarbamoyl group, N-n-pentyl carbamoyl group, N-isopentylcarbamoyl group, N-neopentylcarbamoyl group, N-tert-pentylcarbamoyl group, N-n-hexylcarbamoyl group, N-n-tetradecylcarbamoyl group, N-carboxylmethylcarbamoyl group, N-carbamoylmethylcarbamoyl group, N-aminoethylcarbamoyl group, N-dimethylaminomethylcarbamoyl group, N-phenylcarbamoyl group, N-pyridylcarbamoyl group, N-benzylcarbamoyl group, N-pyridylmethylcarbamoyl group, N,N-dimethylcarbamoyl group, N,N-diethylcarbamoyl group, N,N-dibenzylcarbamoyl group, N-ethyl-N-methylcarbamoyl group and the like.

The aryloxy group which may be substituted means an aryloxy group whose aryl moiety may be substituted at any position. Examples include phenoxy group, methylphenoxy group, nitrophenoxy group, chlorophenoxy group, naphthyloxy group, pyridyloxy group, pyrimidyloxy group, pyrazinyloxy group, imidazolyloxy group, oxazolyloxy group, isoxazolyloxy group, thiazolyloxy group, isothiazolyloxy group, furyloxy group, thienyloxy group, pyrrolyloxy group, benzofuranyloxy group, benzo[b]thienyloxy group, benzimidazolyloxy group, indolyloxy group, quinolyloxy group, isoquinolyloxy group, (1,2,3,4-tetrahydronaphthalen-5-yl)oxy group, (1,2,3,4-tetrahydronaphthalen-6-yl)oxy group and the like. Examples of the arylthio group include phenylthio group, naphthylthio group, pyridylthio group, pyrimidylthio group, pyrazinylthio group, imidazolylthio group, oxazolylthio group, isoxazolylthio group, thiazolylthio group, isothiazolylthio group, furylthio group, thienylthio group, pyrrolylthio group, benzofuranylthio group, benzo[b]thienylthio group, benzimidazolylthio group, indolylthio group, quinolylthio group, isoquinolylthio group,
(1,2,3,4-tetrahydronaphthalen-5-yl)thio group,
(1,2,3,4-tetrahydronaphthalen-6-yl)thio group and the like. Examples of the aralkyloxy group include benzyloxy group, phenethyloxy group, phenylpropyloxy group, phenylhexyloxy group, phenyldodecyloxy group, phenyltetradecyloxy group, pyridylmethyloxy group and the like. Examples of the aralkylthio group include benzylthio group, phenethylthio group, phenylpropylthio group, phenylhexylthio group, phenyldodecylthio group, phenyltetradecylthio group, pyridylmethylthio group and the like. Examples of the halogen atom include fluorine atom, chlorine atom, bromine atom, and iodine atom.

Examples of the acyl group include formyl group, acetyl group, propionyl group, butyryl group, pivaloyl group, benzoyl group, nicotinoyl group, isonicotinoyl group, pyrimidylcarbonyl group, pyrazinylcarbonyl group, oxazolylcarbonyl group, isoxazolylcarbonyl group, thiazolylcarbonyl group, isothiazolylcarbonyl group, naphthoyl group, furoyl group, benzofuranylcarbonyl group, benzo[b]thienylcarbonyl group, benzimidazolylcarbonyl group, indolylcarbonyl group, thenoyl group, pyrrolylcarbonyl group, quinolylcarbonyl group, isoquinolylcarbonyl group, cyclohexylcarbonyl group, phenylacetyl group, naphthylacetyl group, pyridylacetyl group, pyrimidylacetyl group, pyrazinylacetyl group, imidazolylacetyl group, oxazolylacetyl group, isoxazolylacetyl group, thiazolylacetyl group, isothiazolylacetyl group, furylacetyl group, benzofuranylacetyl group, benzo[b]thienylacetyl group, benzimidazolylacetyl group, indolylacetyl group, thienylacetyl group, pyrrolylacetyl group, quinolylacetyl group, isoquinolylacetyl group, cyclohexylacetyl group, phenylpropionyl group, phenylhexylcarbonyl group, phenyldodecylcarbonyl group, phenyltetradecylcarbonyl group and the like. Examples of the cycloalkenyl group include cyclopentenyl group, cyclohexenyl group and the like.

Examples of the saturated heterocyclic group which may be substituted include aziridinyl group, azetidinyl group, pyrrolidinyl group, piperidinyl group, hexahydro-1H-azepinyl group, piperazinyl group, morpholinyl group, thiomorpholinyl group, tetrahydropyranyl group, tetrahydrothiopyranyl group, oxopyrrolidinyl group, methylpiperazinyl group, pyridylpiperazinyl group and the like. The aryl group which may be substituted means a group which may be substituted at any position of the aryl ring. Examples include optionally substituted monocyclic or polycyclic aromatic rings such as phenyl group, hydroxyphenyl group, methoxyphenyl group, aminophenyl group, acetamidephenyl group, carbamoylphenyl group, fluorophenyl group, chlorophenyl group, bromophenyl group, dichlorophenyl group, toluyl group, n-heptylphenyl group, n-tetradecylphenyl group, trifluoromethylphenyl group, biphenyl group, cyanophenyl group, nitrophenyl group, amidinophenyl group, guanidinophenyl group, sulfamoylphenyl group, naphthyl group, pyridyl group, pyrimidyl group, pyrazinyl group, imidazolyl group, furyl group, thienyl group, pyrrolyl group, benzofuranyl group, benzo[b]thienyl group, benzimidazolyl group, indolyl group, quinolyl group, isoquinolyl group, phenylimidazolyl group, phenylthiazolyl group, pyridylimidazolyl group, pyridylthiazolyl group and the like. The aralkyl group which may be substituted means an aralkyl group whose aryl group may be substituted at any position. Examples include benzyl group, phenethyl group, phenylpropyl group, phenylbutyl group, phenylpentyl group, phenylhexyl group, phenylheptyl group, phenyloctyl group, phenylnonyl group, phenyldecyl group, phenylundecyl group, phenyldodecyl group, phenyltridecyl group, phenyltetradecyl group, naphthylmethyl group, naphthylethyl group, naphthylpropyl group, naphthyl butyl group, pyridylmethyl group, pyridylethyl group, pyridylpropyl group, pyrimidylethyl group, pyrimidylpropyl group, pyrazinylethyl group, pyrazinylpropyl group, imidazolylethyl group, imidazolylpropyl group, imidazolylbutyl group, 4-pyridylimidazolylbutyl group, oxazolylethyl group, oxazolylpropyl group, isoxazolylethyl group, isoxazolylpropyl group, thiazolylethyl group, thiazolylpropyl group, isothiazolylethyl group, isothiazolylpropyl group, furylethyl group, furylpropyl group, thienylethyl group, thienylpropyl group, pyrrolylethyl group, pyrrolylpropyl group, benzofuranylethyl group, benzofuranylpropyl group, benzo[b]thienylethyl group, benzo[b]thienylpropyl group, benzimidazolylethyl group, benzimidazolylpropyl group, indolylethyl group, indolylpropyl group, quinolylethyl group, quinolylpropyl group, quinolylbutyl group, isoquinolylethyl group, isoquinolylpropyl group, isoquinolylbutyl group and the like.

Examples of the alkoxycarbonyl group include methoxycarbonyl group, ethoxycarbonyl group, n-propoxycarbonyl group, isopropoxycarbonyl group, n-butoxycarbonyl group, isobutoxycarbonyl group, sec-butoxycarbonyl group, tert-butoxycarbonyl group, n-pentyloxycarbonyl group, isopentyloxycarbonyl group, neopentyloxycarbonyl group, tert-pentyloxycarbonyl group, n-hexyloxycarbonyl group, n-heptyloxycarbonyl group, n-octyloxycarbonyl group, n-nonyloxycarbonyl group, n-decyloxycarbonyl group, n-undecyloxycarbonyl group, n-dodecyloxycarbonyl group, n-tridecyloxycarbonyl group, n-tetradecyloxycarbonyl group and the like. Examples of the aryloxycarbonyl group include phenoxycarbonyl group, naphthyloxycarbonyl group, pyridyloxycarbonyl group, pyrimidyloxycarbonyl group, pyrazinyloxycarbonyl group, imidazolyloxycarbonyl group, oxazolyloxycarbonyl group, isoxazolyloxycarbonyl group, thiazolyloxycarbonyl group, isothiazolyloxycarbonyl group, furyloxycarbonyl group, thienyloxycarbonyl group, pyrrolyloxycarbonyl group, benzofuranyloxycarbonyl group, benzo[b]thienyloxycarbonyl group, benzimidazolyloxycarbonyl group, indolyloxycarbonyl group, quinolyloxycarbonyl group, isoquinolyloxycarbonyl group and the like. Examples of the aralkyloxycarbonyl group include benzyloxycarbonyl group, phenethyloxycarbonyl group, phenylhexyloxycarbonyl group, phenyldodecyloxycarbonyl group, phenyltetradecyloxycarbonyl group, naphthylmethyloxycarbonyl group, pyridylmethyloxycarbonyl group, pyrimidylmethyloxycarbonyl group, pyrazinylmethyloxycarbonyl group, imidazolylmethyloxycarbonyl group, oxazolylmethyloxycarbonyl group, isoxazolylmethyloxycarbonyl group, thiazolylmethyloxycarbonyl group, isothiazolylmethyloxycarbonyl group, furylmethyloxycarbonyl group, thienylmethyloxycarbonyl group, pyrrolylmethyloxycarbonyl group, benzofuranylmethyloxycarbonyl group, benzo[b]thienylmethyloxycarbonyl group, benzimidazolylmethyloxycarbonyl group, indolylmethyloxycarbonyl group, quinolylmethyloxycarbonyl group, isoquinolylmethyloxycarbonyl group and the like.

Examples of the alkylsulfinyl group include methylsulfinyl group, ethylsulfinyl group, n-propylsulfinyl group, isopropylsulfinyl group, n-butylsulfinyl group, isobutylsulfinyl group, sec-butylsulfinyl group, tert-butylsulfinyl group, n-pentylsulfinyl group, isopentylsulfinyl group, neopentylsulfinyl group, tert-pentylsulfinyl group, n-hexylsulfinyl group, n-heptylsulfinyl group, n-octylsulfinyl group, n-nonylsulfinyl group, n-decylsulfinyl group, n-undecylsulfinyl group, n-dodecylsulfinyl group, n-tridecylsulfinyl group, n-tetradecylsulfinyl group and the like. Examples of the arylsulfinyl group include phenylsulfinyl group, naphthylsulfinyl group, pyridylsulfinyl group, pyrimidylsulfinyl group, pyrazinylsulfinyl group, imidazolylsulfinyl group, oxazolylsulfinyl group, isoxazolylsulfinyl group, thiazolylsulfinyl group, isothiazolylsulfinyl group, furylsulfinyl group, thienylsulfinyl group, pyrrolylsulfinyl group, benzofuranylsulfinyl group, benzo[b]thienylsulfinyl group, benzimidazolylsulfinyl group, indolylsulfinyl group, quinolylsulfinyl group, isoquinolylsulfinyl group and the like. Examples of the aralkylsulfinyl group include benzylsulfinyl group, phenethylsulfinyl group, phenylhexylsulfinyl group, phenyldodecylsulfinyl group, phenyltetradecylsulfinyl group, naphthylmethylsulfinyl group, pyridylmethylsulfinyl group, pyrimidylmethylsulfinyl group, pyrazinylmethylsulfinyl group, imidazolylmethylsulfinyl group, oxazolylmethylsulfinyl group, isoxazolylmethylsulfinyl group, thiazolylmethylsulfinyl group, isothiazolylmethylsulfinyl group, furylmethylsulfinyl group, thienylmethylsulfinyl group, pyrrolylmethylsulfinyl group, benzofuranylmethylsulfinyl group, benzo[b]thienylmethylsulfinyl group, benzimidazolylmethylsulfinyl group, indolylmethylsulfinyl group, quinolylmethylsulfinyl group, isoquinolylmethylsulfinyl group and the like.

Examples of the alkylsulfonyl group include mesyl group, ethylsulfonyl group, n-propylsulfonyl group, isopropylsulfonyl group, n-butylsulfonyl group, isobutylsulfonyl group, sec-butylsulfonyl group, tert-butylsulfonyl group, n-pentylsulfonyl group, isopentylsulfonyl group, neopentylsulfonyl group, tert-pentylsulfonyl group, n-hexylsulfonyl group, n-heptylsulfonyl group, n-octylsulfonyl group, n-nonylsulfonyl group, n-decylsulfonyl group, n-undecylsulfonyl group, n-dodecylsulfonyl group, n-tridecylsulfonyl group, n-tetradecylsulfonyl group and the like. Examples of the arylsulfonyl group include phenylsulfonyl group, naphthylsulfonyl group, pyridylsulfonyl group, pyrimidylsulfonyl group, pyrazinylsulfonyl group, imidazolylsulfonyl group, oxazolylsulfonyl group, isoxazolylsulfonyl group, thiazolylsulfonyl group, isothiazolylsulfonyl group, furylsulfonyl group, thienylsulfonyl group, pyrrolylsulfonyl group, benzofuranylsulfonyl group, benzo[b]thienylsulfonyl group, benzimidazolylsulfonyl group, indolylsulfonyl group, quinolylsulfonyl group, isoquinolylsulfonyl group and the like. Examples of the aralkylsulfonyl group include benzylsulfonyl group, phenethylsulfonyl group, phenylhexylsulfonyl group, phenyldodecylsulfonyl group, phenyltetradecylsulfonyl group, naphthylmethylsulfonyl group, pyridylmethylsulfonyl group, pyrimidylmethylsulfonyl group, pyrazinylmethylsulfonyl group, imidazolylmethylsulfonyl group, oxazolylmethylsulfonyl group, isoxazolylmethylsulfonyl group, thiazolylmethylsulfonyl group, isothiazolylmethylsulfonyl group, furylmethylsulfonyl group, thienylmethylsulfonyl group, pyrrolylmethylsulfonyl group, benzofuranylmethylsulfonyl group, benzo[b]thienylmethylsulfonyl group, benzimidazolylmethylsulfonyl group, indolylmethylsulfonyl group, quinolylmethylsulfonyl group, isoquinolylmethylsulfonyl group and the like.

Examples of the alkyl group or cycloalkyl group which may be a substituent include those explained above.

In the compounds of the present invention represented by the aforementioned general formula (I), when the above defined group or functional group, or a part of the above defined group or functional group is a group or functional group corresponding to a "saturated homocyclic group", "saturated heterocyclic group", "unsaturated homocyclic group", or "unsaturated heterocyclic group", said cyclic groups substitute/bind at any position on a substitutable/bindable atom on a ring, unless substituting/binding position is specifically defined.

The compounds of the present invention represented by the aforementioned general formula have asymmetric carbon atoms, and accordingly, stereoisomers such as optical isomers, diastereoisomers, and geometrical isomers may exist. These isomers and mixtures thereof, and salts thereof also fall within the scope of the present invention. Among the compounds of the present invention represented by the aforementioned general formula (I), the compounds wherein R¹ is hydrogen atom may exist as the tautomers represented by the general formula (IV). Said isomers and salt thereof as well as stereoisomers thereof based on asymmetric carbon atoms also fall within the scope of the present invention.

The compounds represented by the aforementioned general formula (I) can be converted into salts, if desired, preferably into pharmacologically acceptable salts. The salt formed can be converted into compounds in free forms. Examples of the salts of the compounds of the present invention represented by the aforementioned general formula (I) include acid addition salts or alkali addition salts. Examples of the acid addition salts include, for example, mineral acid salts such as hydrochloride, hydrobromide, nitrate, sulfate, hydroiodide and phosphate, organic acid salts such as acetate, propionate, butyrate, isobutyrate, formate, valerate, isovalerate, pivalate, trifluoroacetate, acrylate, maleate, tartrate, citrate, oleate, laurate, stearate, enanthate, caprylate, caprate, palmitate, myristate, heptadecanoate, succinate, lactobionate, glutarate, sebacate, gluconate, glycolate, sorbate, benzoate, methanesulfonate, ethanesulfonate, 2-hydroxyethanesulfonate, benzenesulfonate, phthalate, terephthalate, cinnamate, p-toluenesulfonate, laurylsulfate, gluceptate, malate, malonate, aspartate, glutamate, adipate, oxalate, nicotinate, picrate, thiocyanate, undecanoate, mandelate, fumarate, 10-camphorsulfonate, lactate, 5-oxotetrahydrofuran-2-carboxylate and 2-hydroxyglutarate. Examples of alkali addition salts include, for example, mineral alkali salts such as sodium salt, potassium salt, calcium salt, magnesium salt and ammonium salt, and organic base salts such as ethanolamine salt and N,N-dialkylethanolamine salt, and salts of the optically active substance thereof.

The compounds of the present invention represented by the aforementioned general formula (I) or salts thereof can exist in the forms of any crystals depending on manufacturing conditions, or may exist as any hydrates or solvates formed with organic solvents. These crystalline forms, hydrates, and solvates, and mixtures thereof also fall within the scope of the present invention.

Preferred compounds of the present invention include the compounds set out below. However, the present invention is not limited to these compounds. In the compounds shown in the tables below, it should be understood that the compounds wherein R¹ is hydrogen atom may exist as tautomers as explained above, and such tautomers also fall within the scope of preferred compounds. In the table, Me represents methyl group, Et represents ethyl group, n-Pr represents n-propyl group, i-Pr represents isopropyl group, n-Bu represents n-butyl group, i-Bu represents isobutyl group, sec-Bu represents sec-butyl group, tert-Bu represents tert-butyl group, n-Pent represents n-pentyl group, i-Pent represents isopentyl group, neo-Pent represents neopentyl group, tert-Pent represents tert-pentyl group, n-Hex represents n-hexyl group, n-Hept represents n-heptyl group, n-Oct represents n-octyl group, n-Non represents n-nonyl group, n-Dec represents n-decyl group, n-Undec represents n-undecyl group, n-Dodec represents n-dodecyl group, n-Tridec represents n-tridecyl group, n-Tetradec represents n-tetradecyl group, and Ac represents acetyl group.

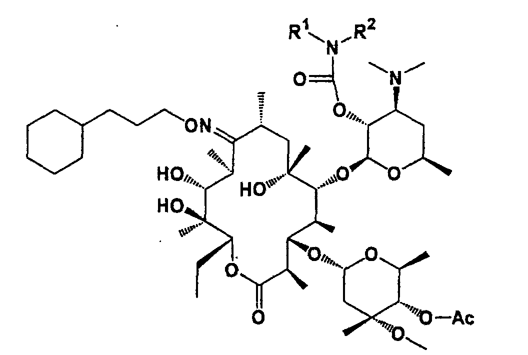

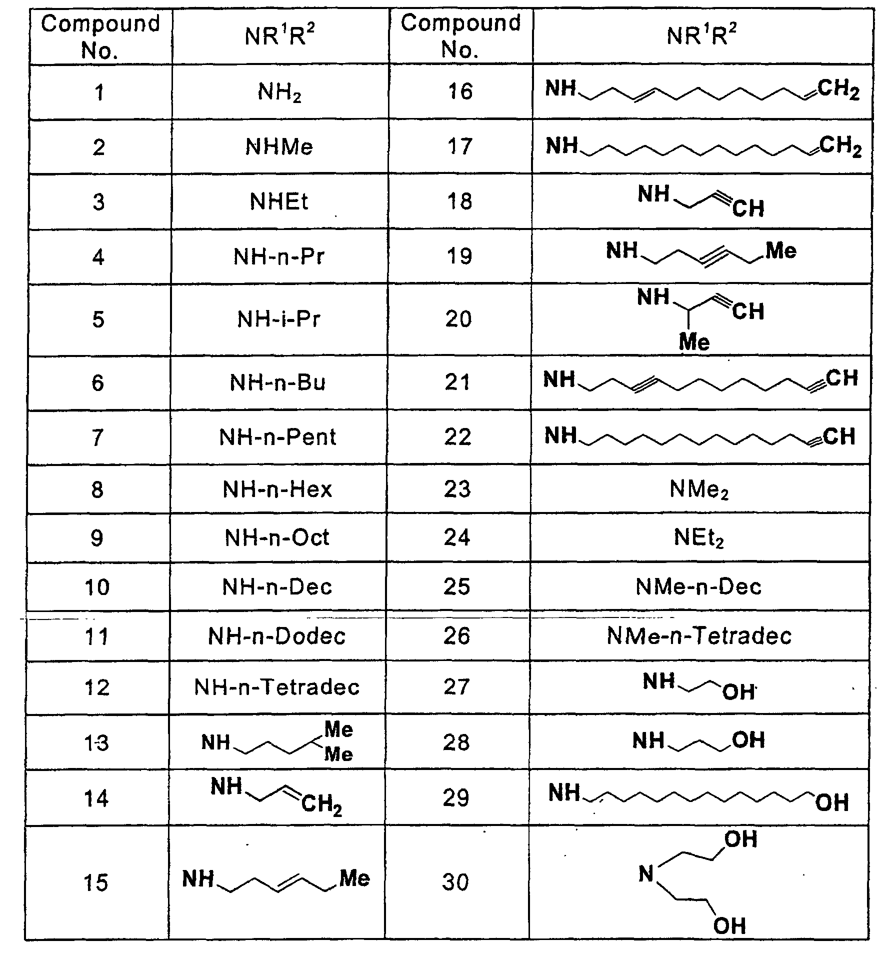

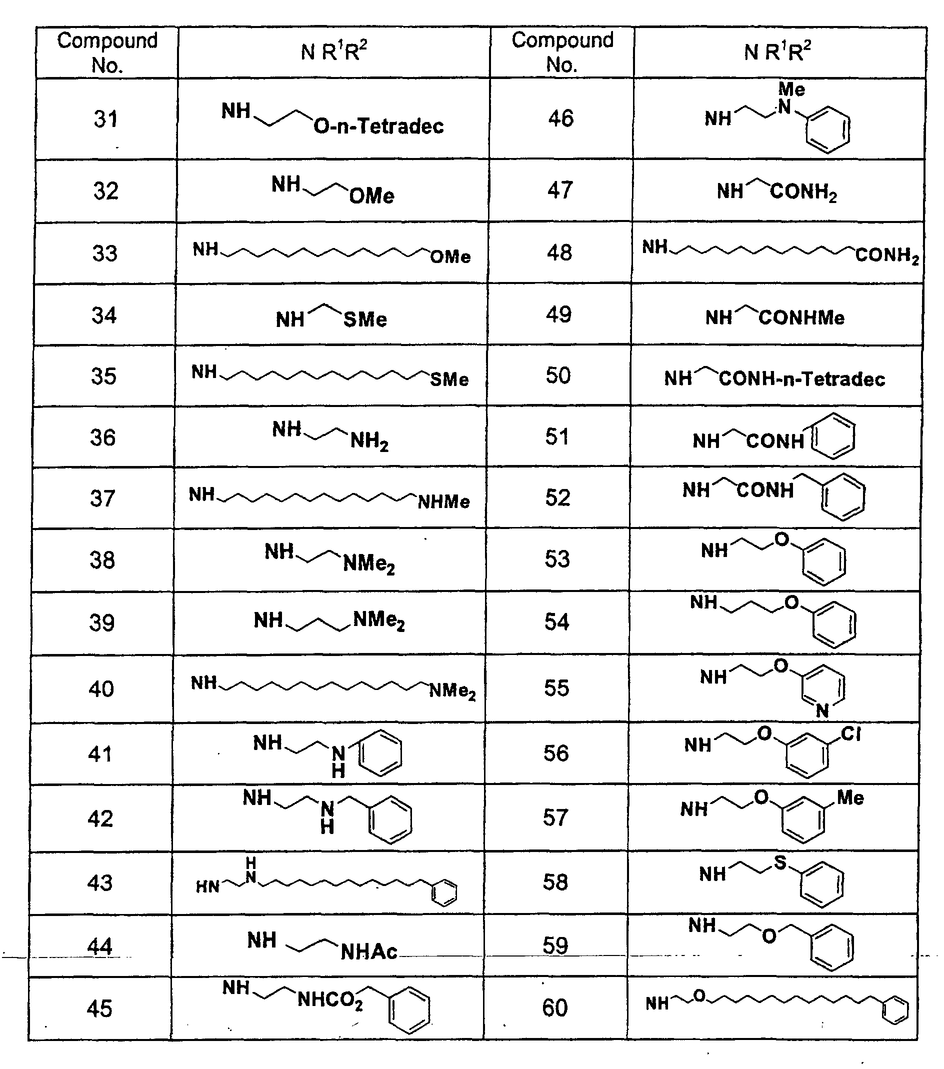

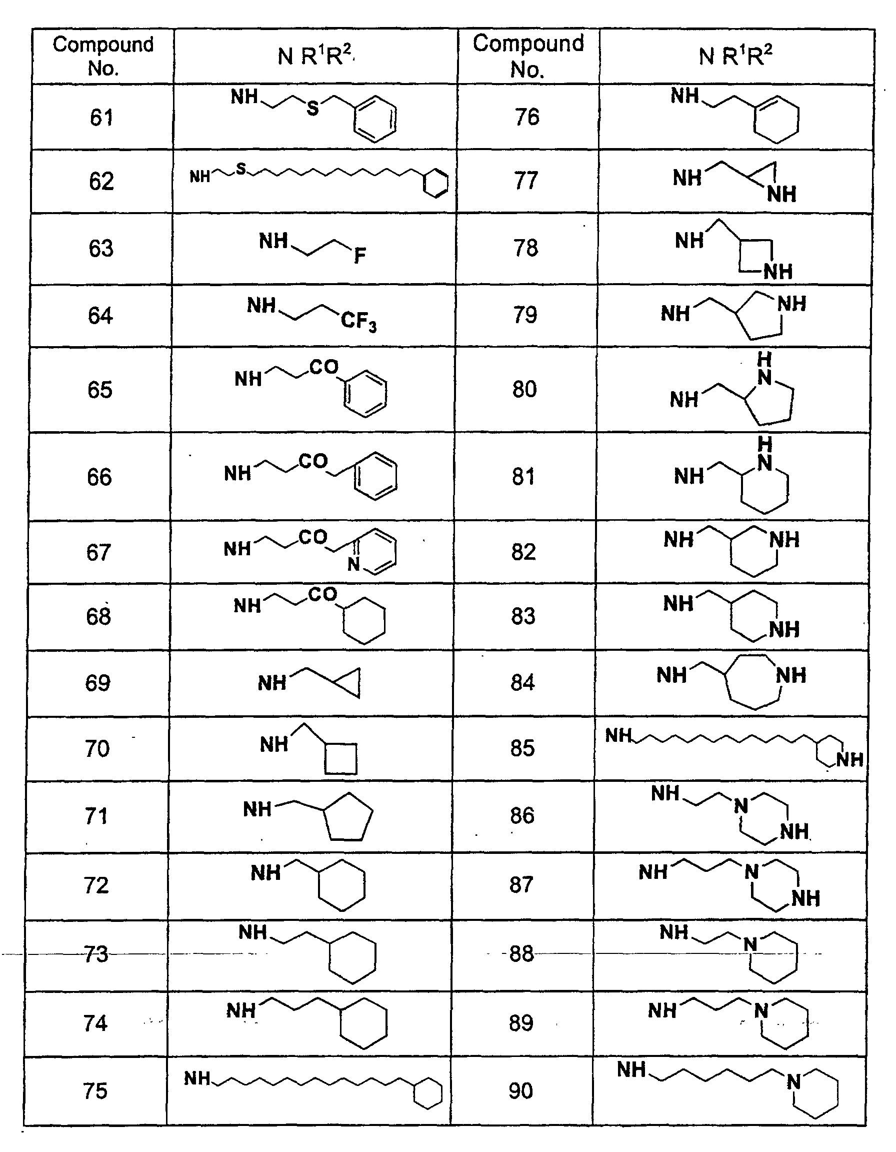

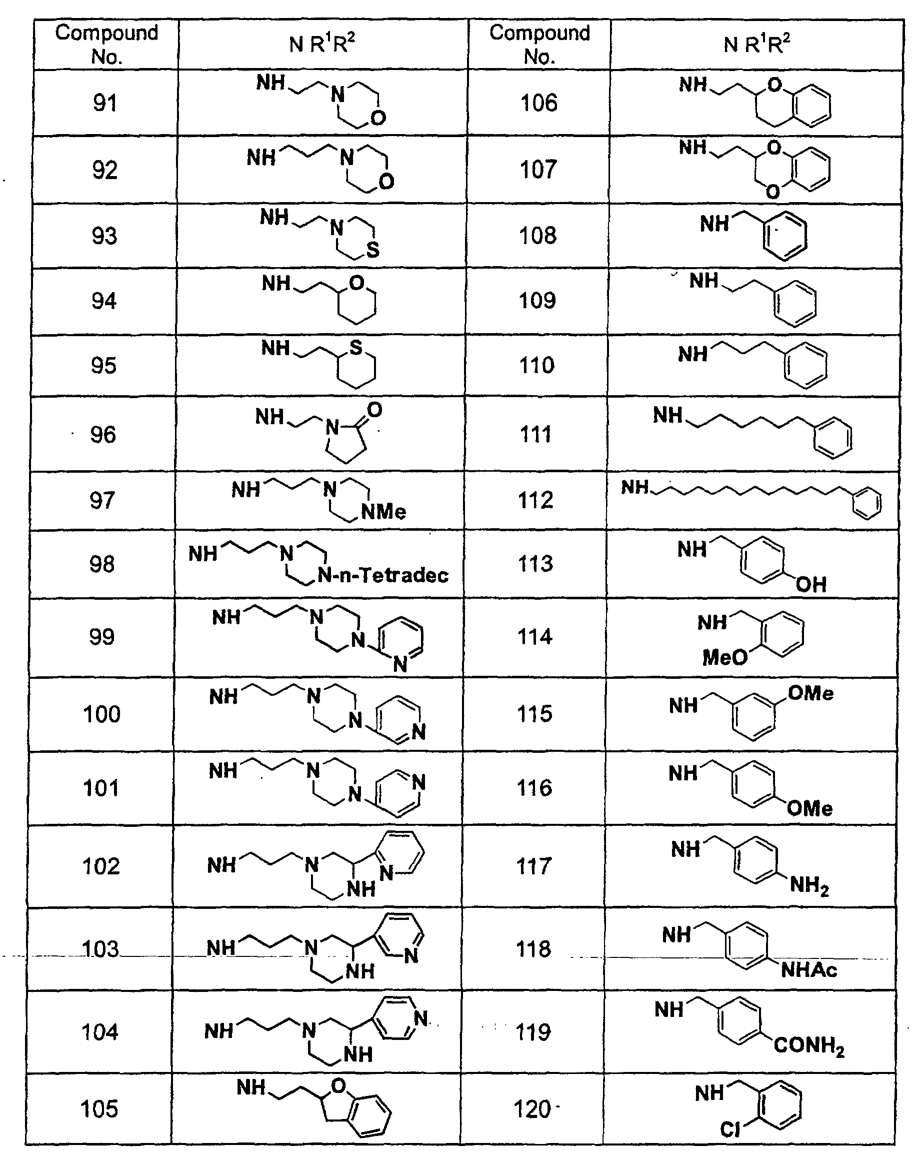

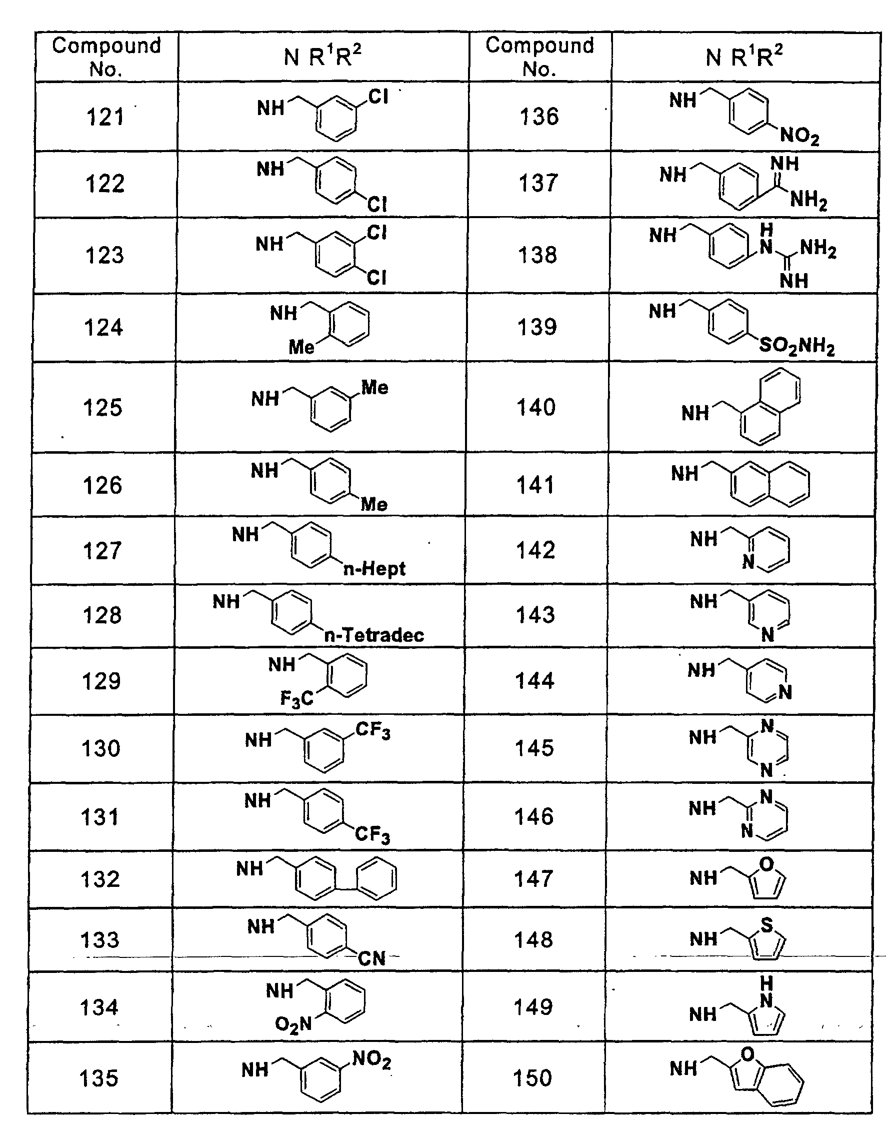

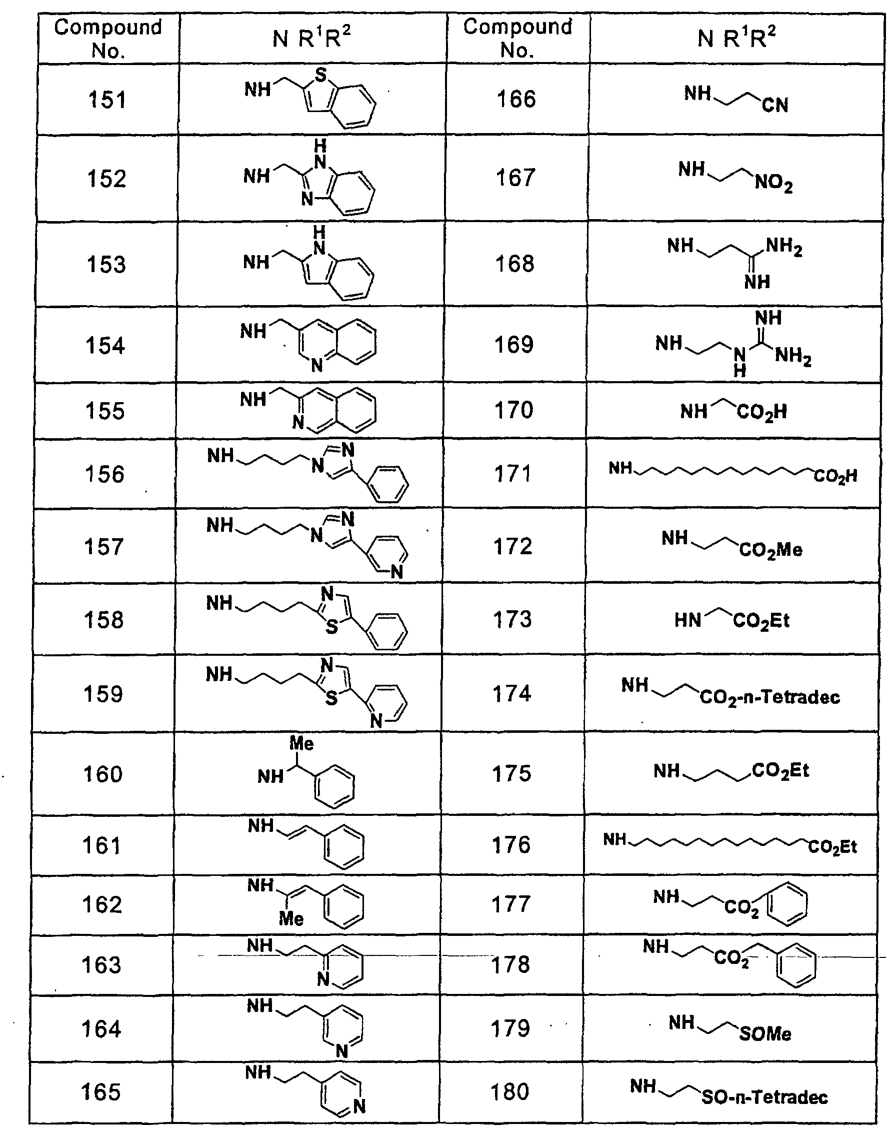

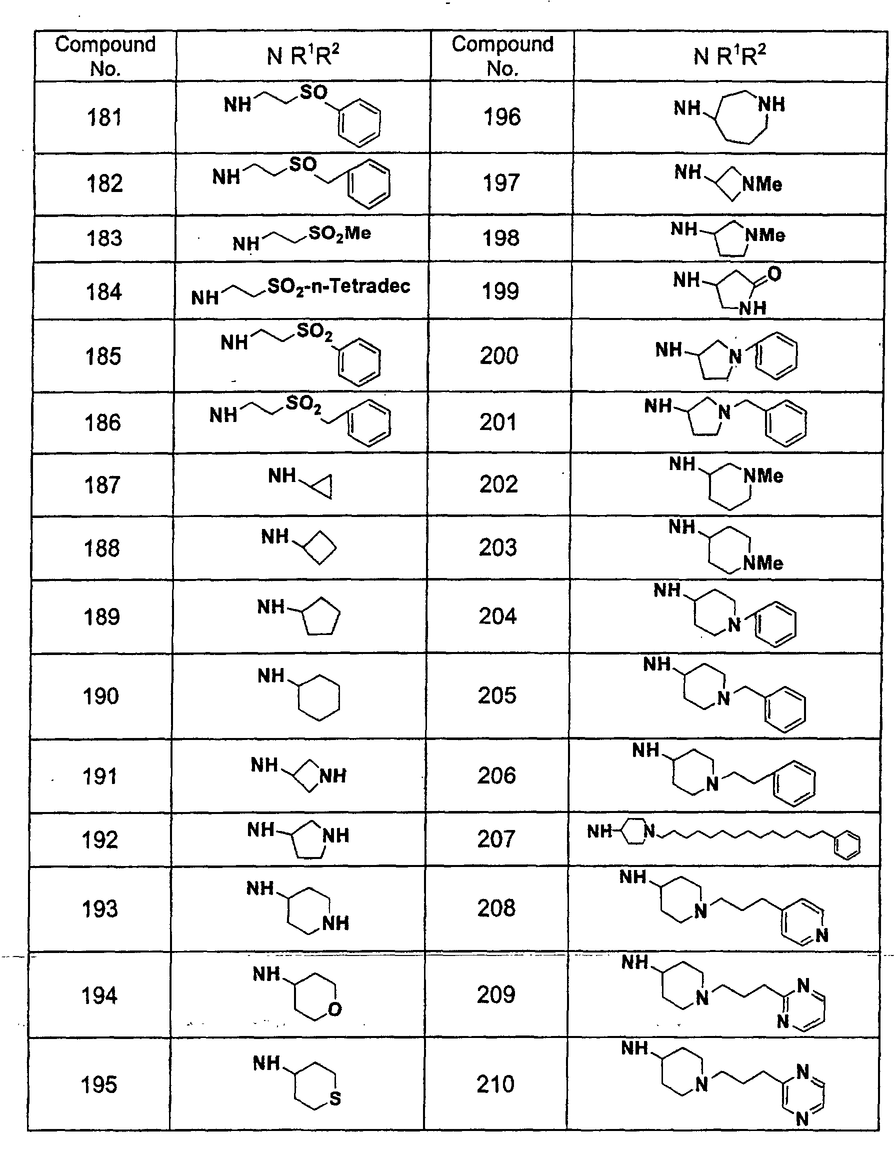

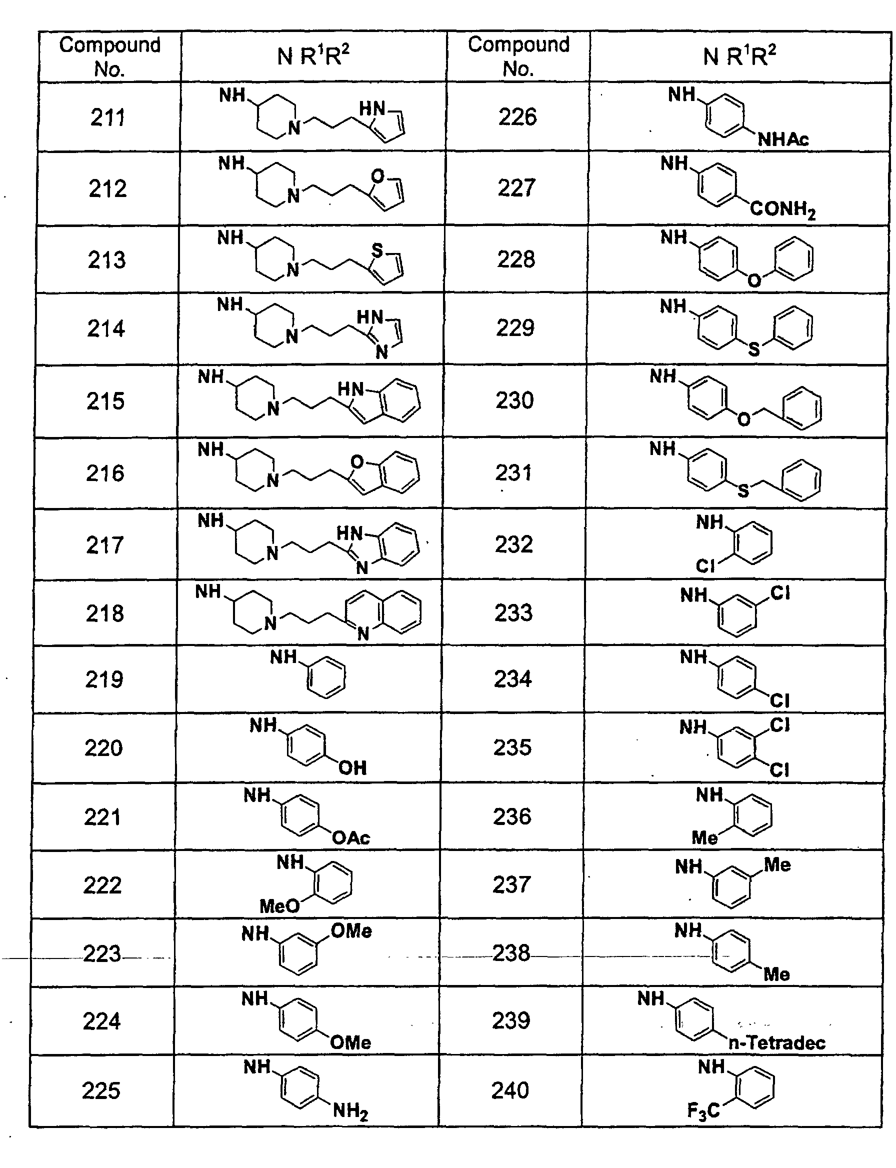

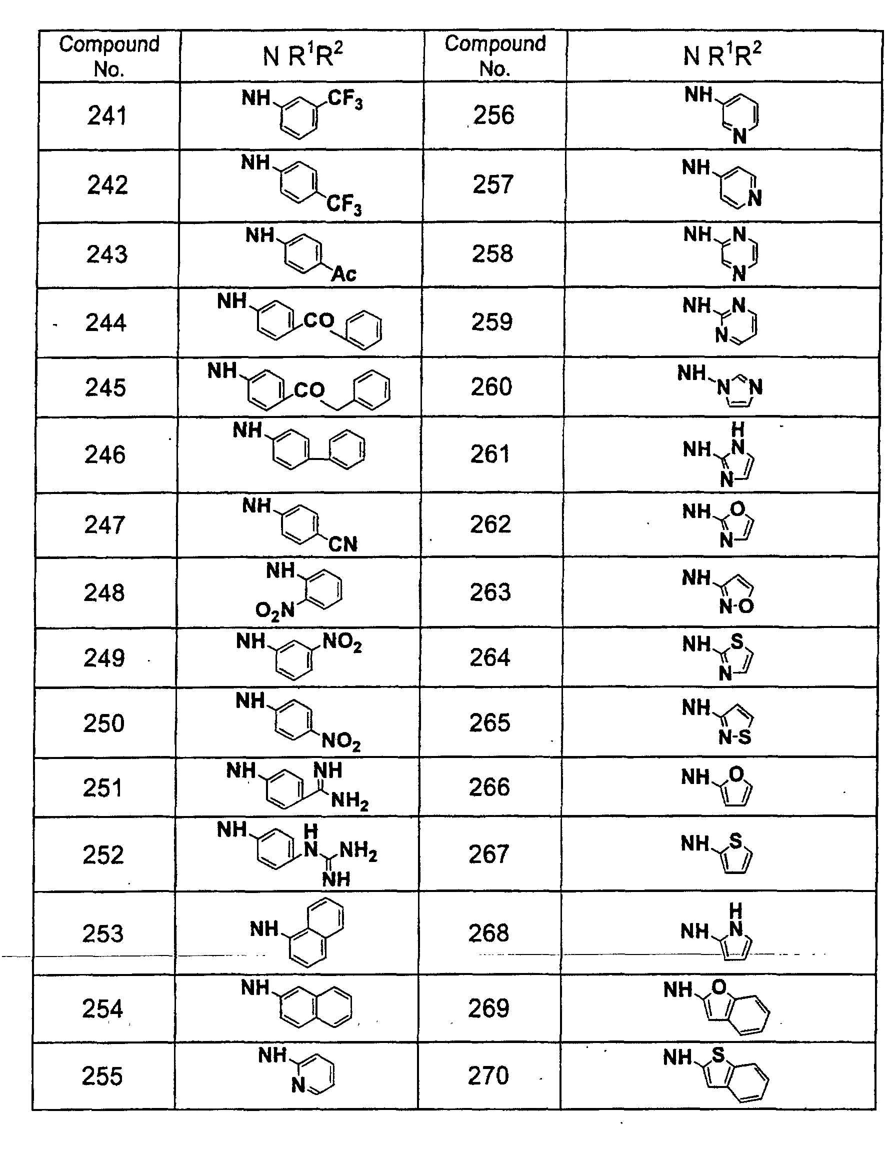

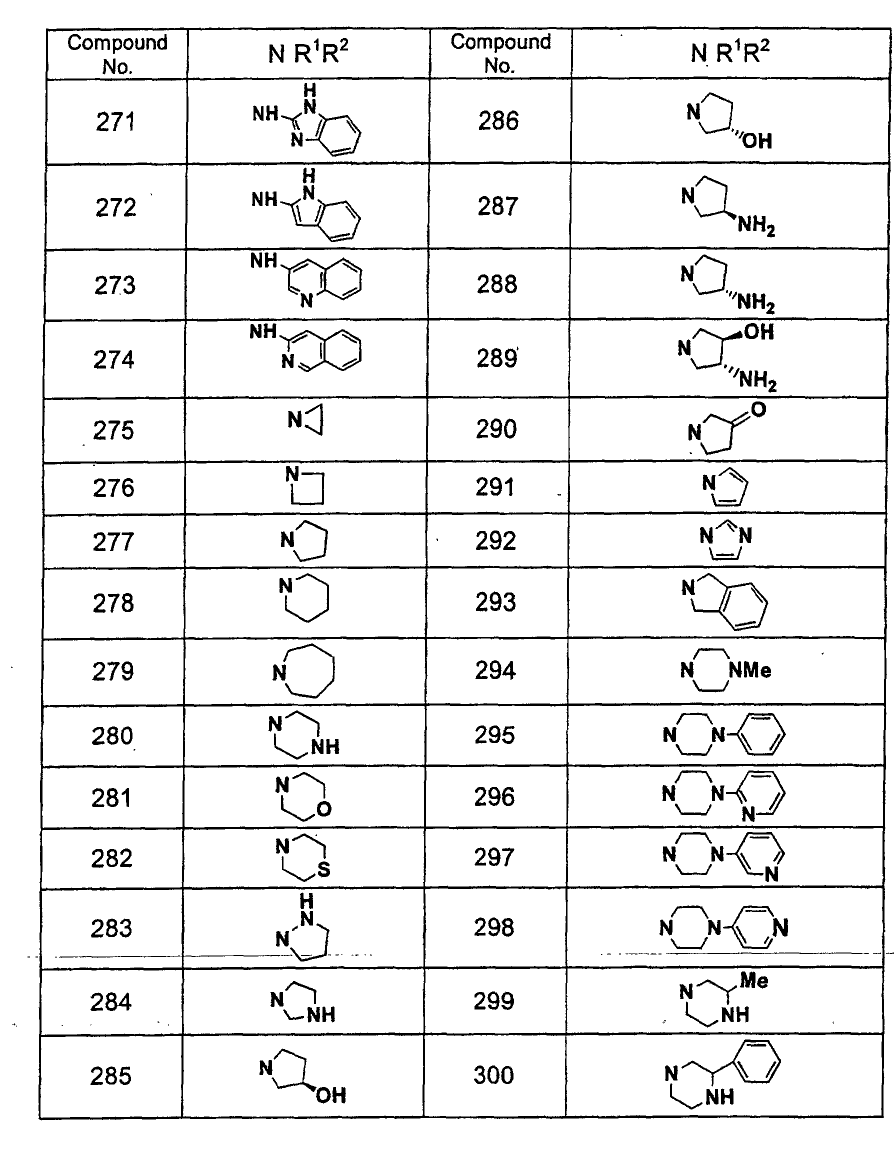

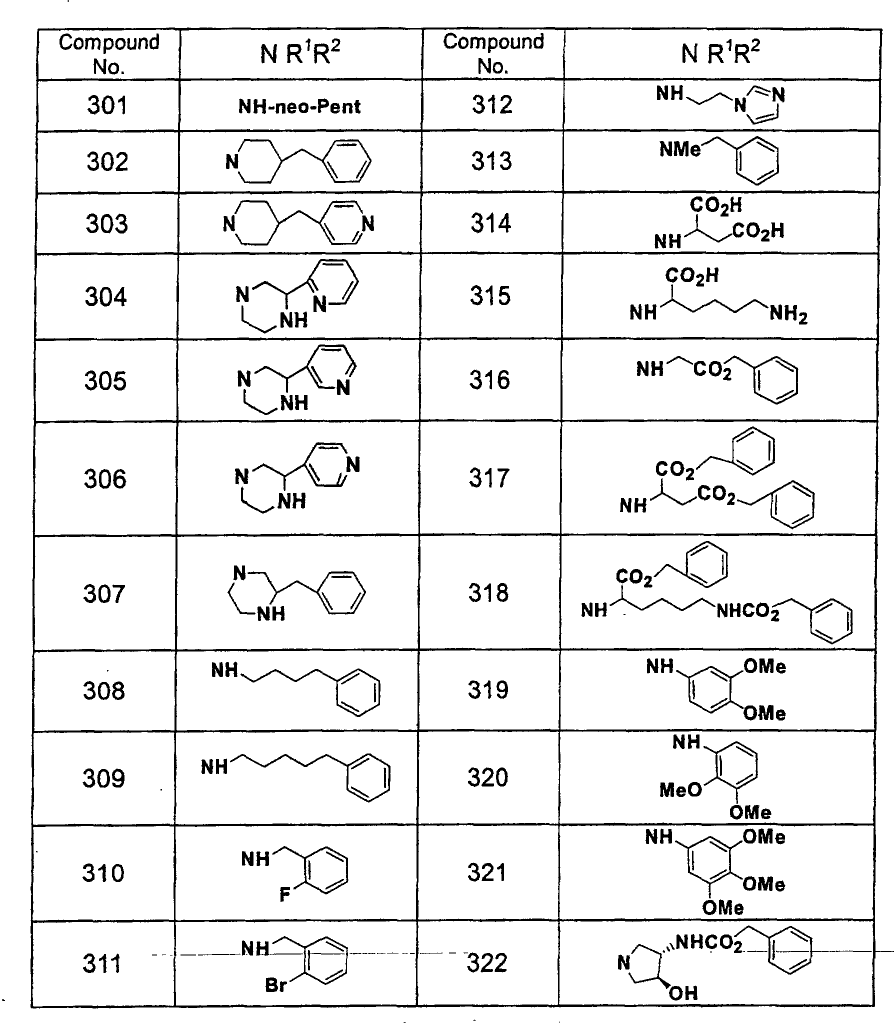

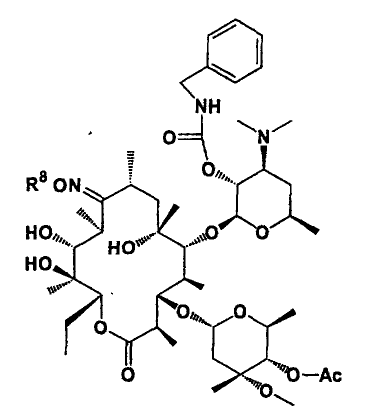

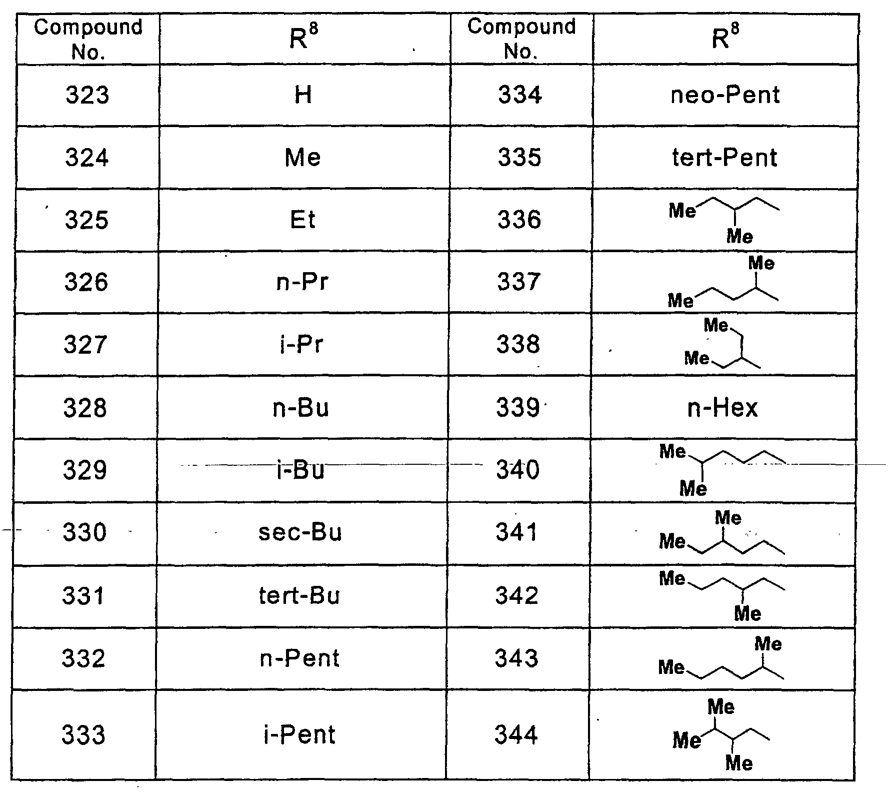

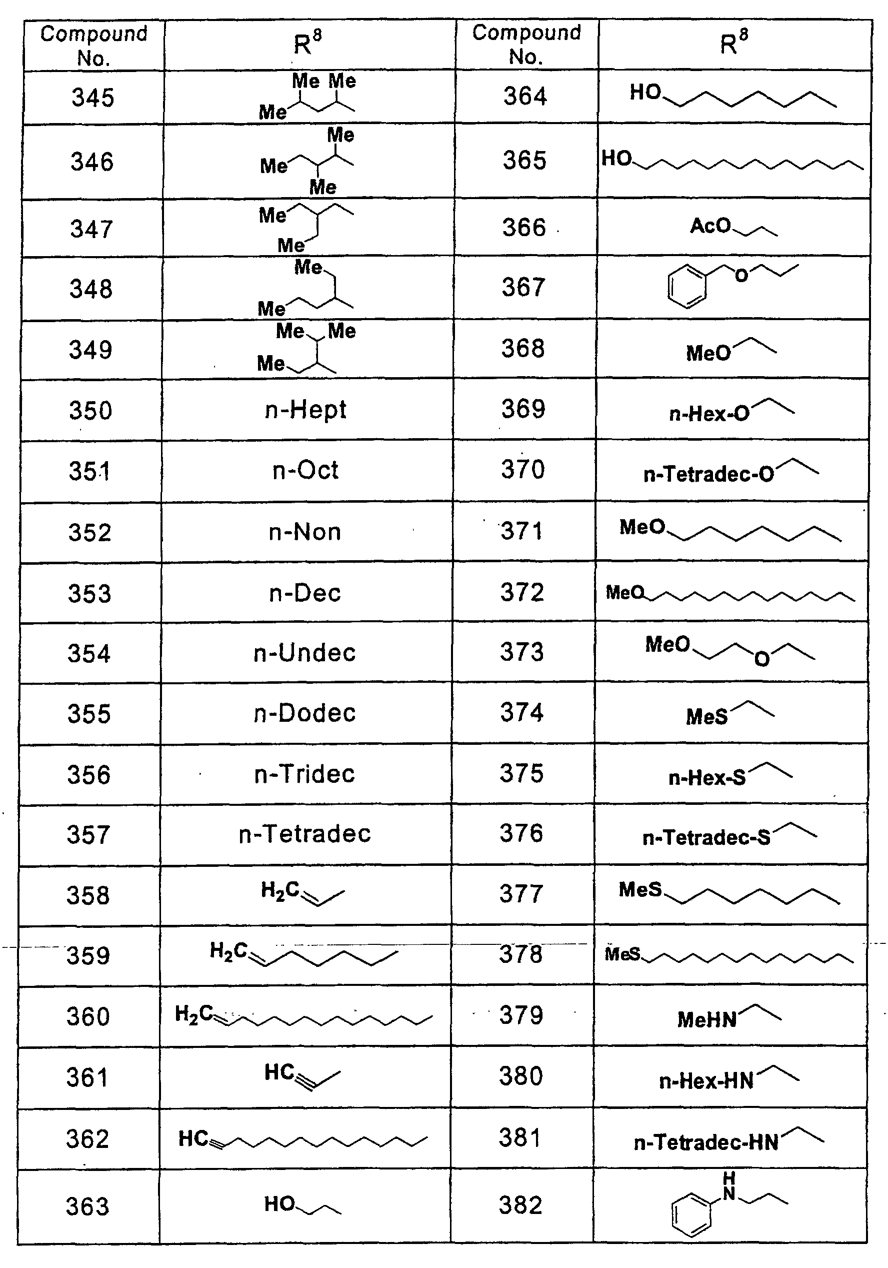

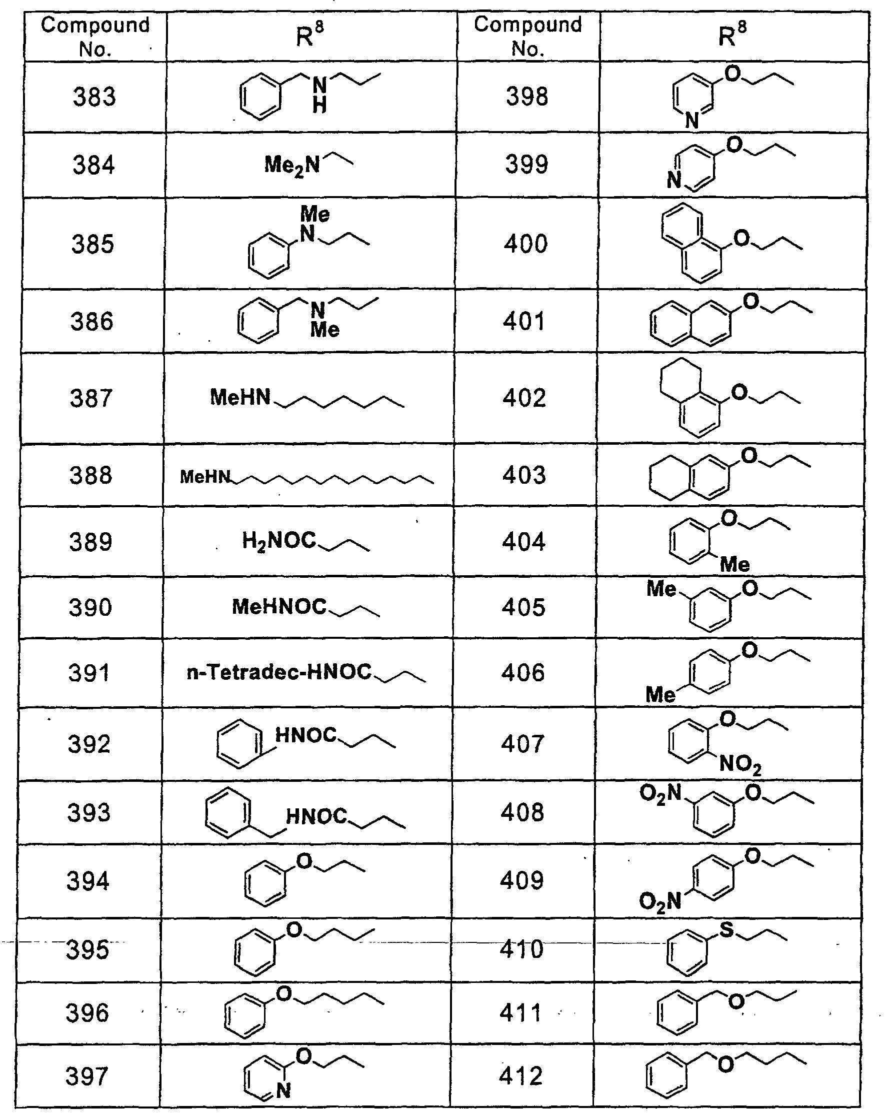

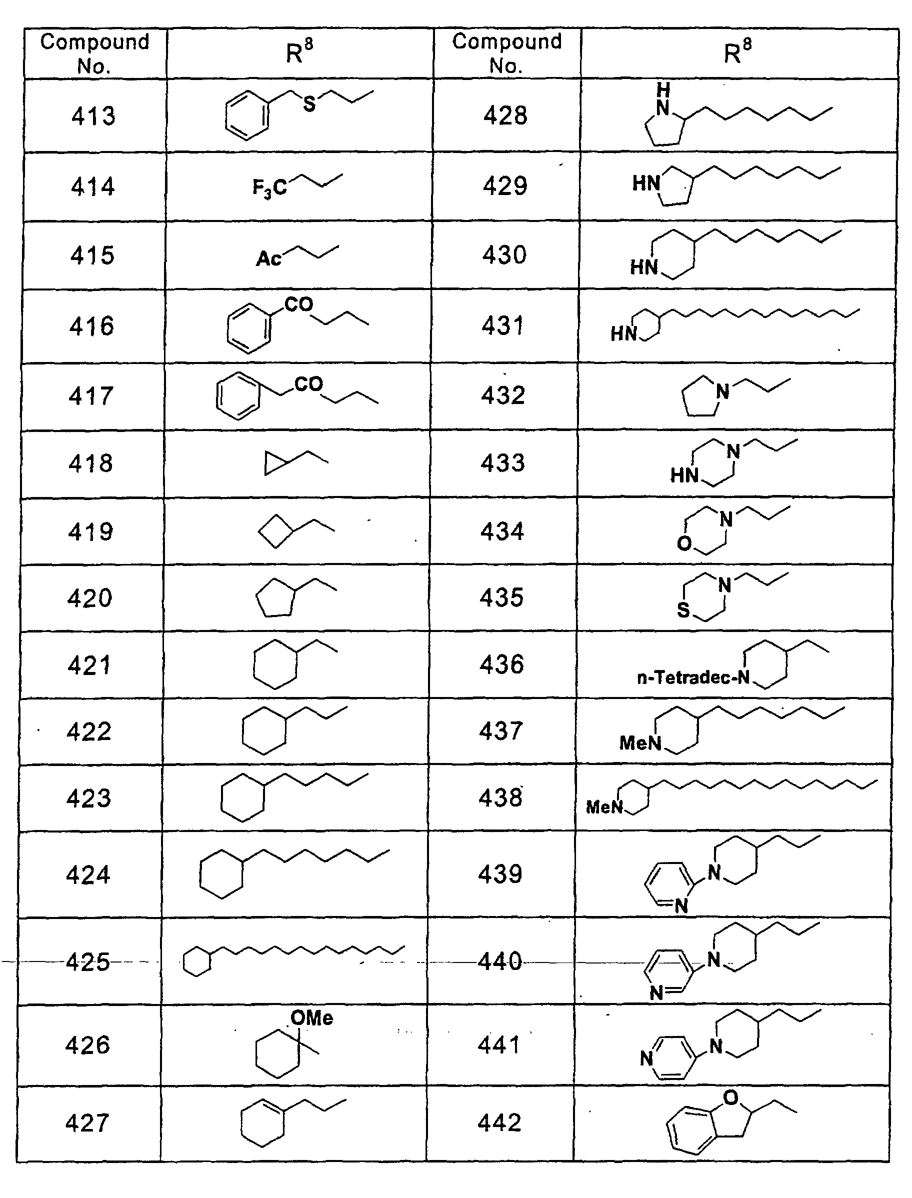

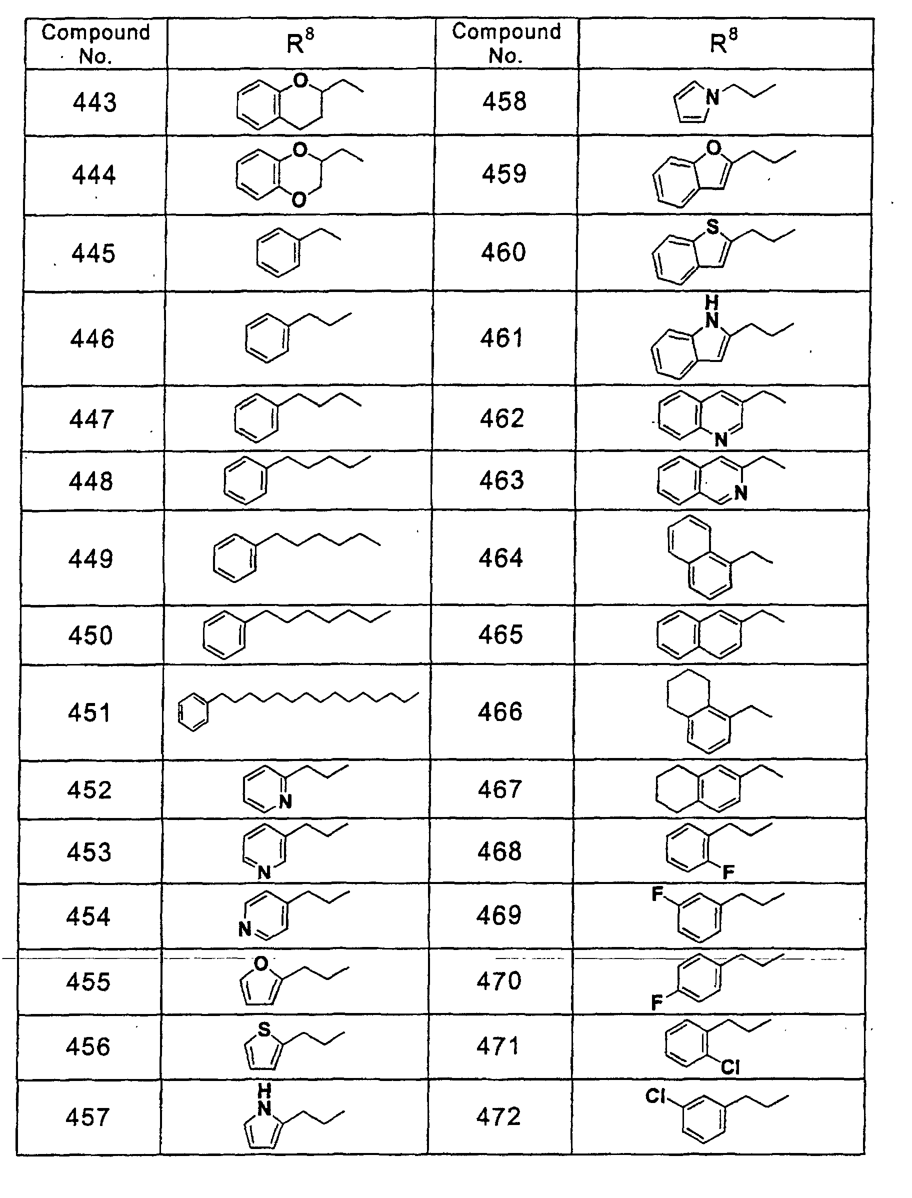

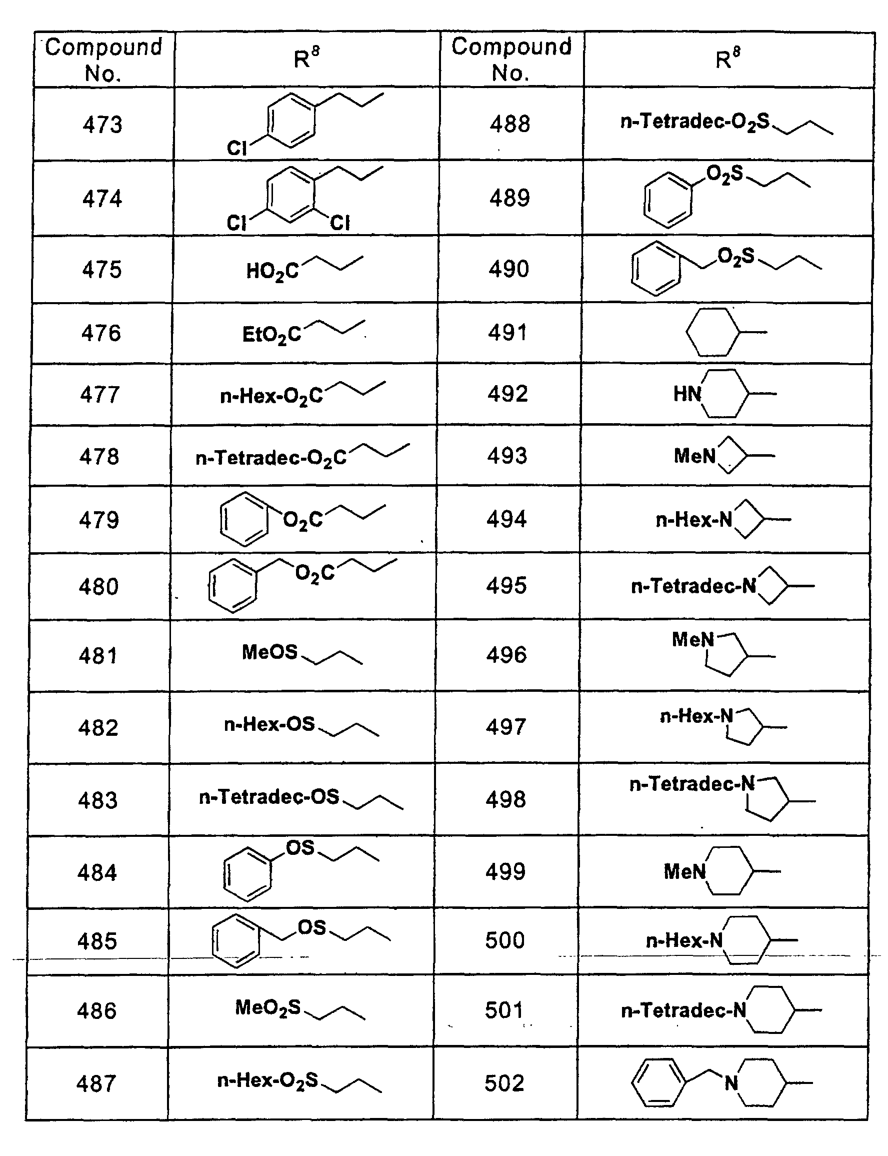

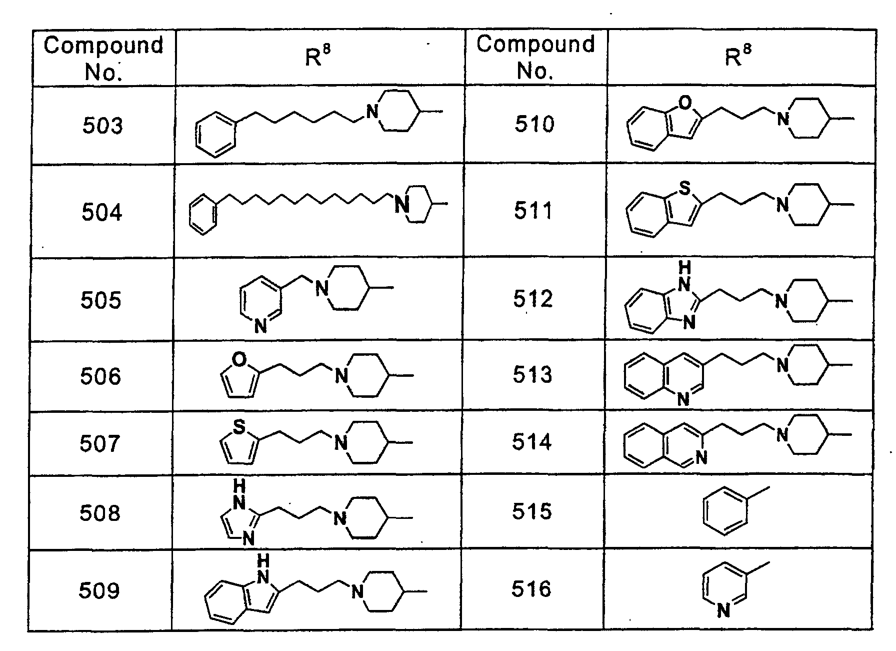

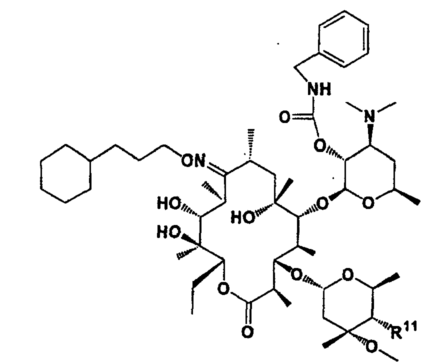

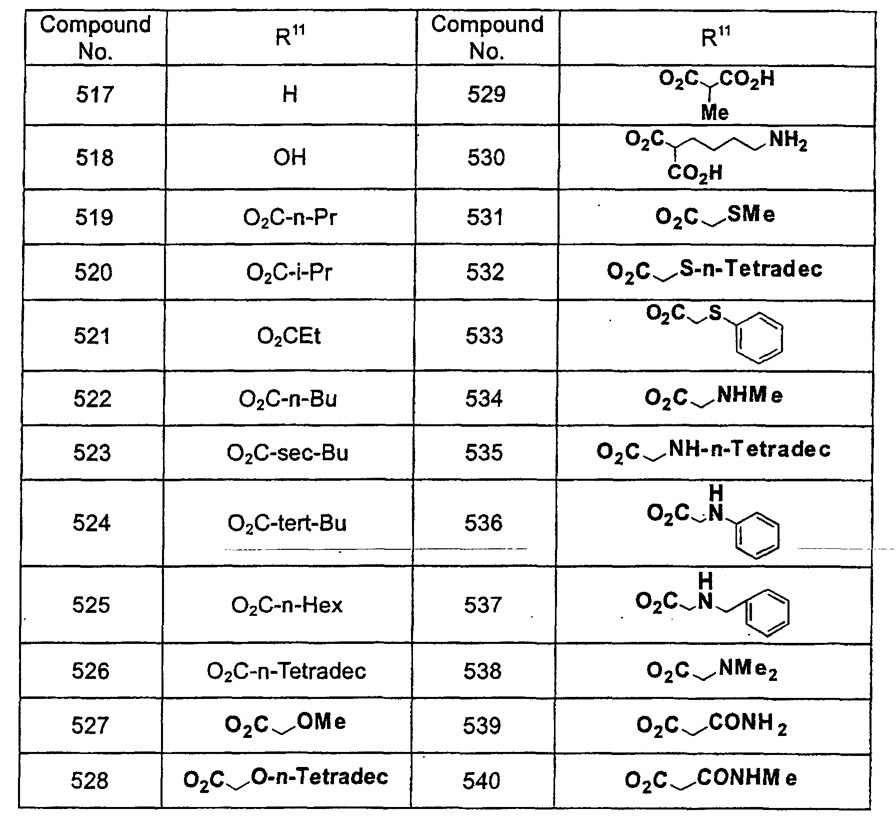

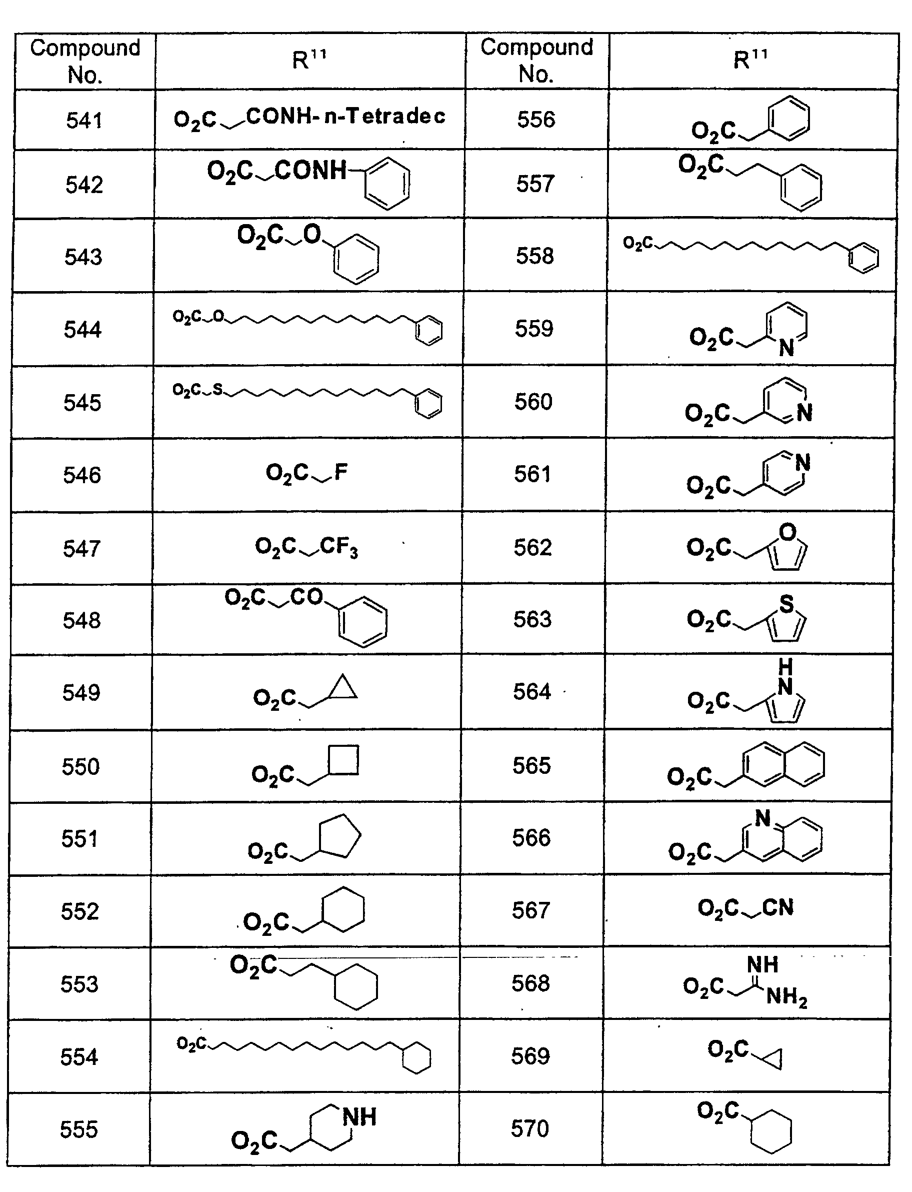

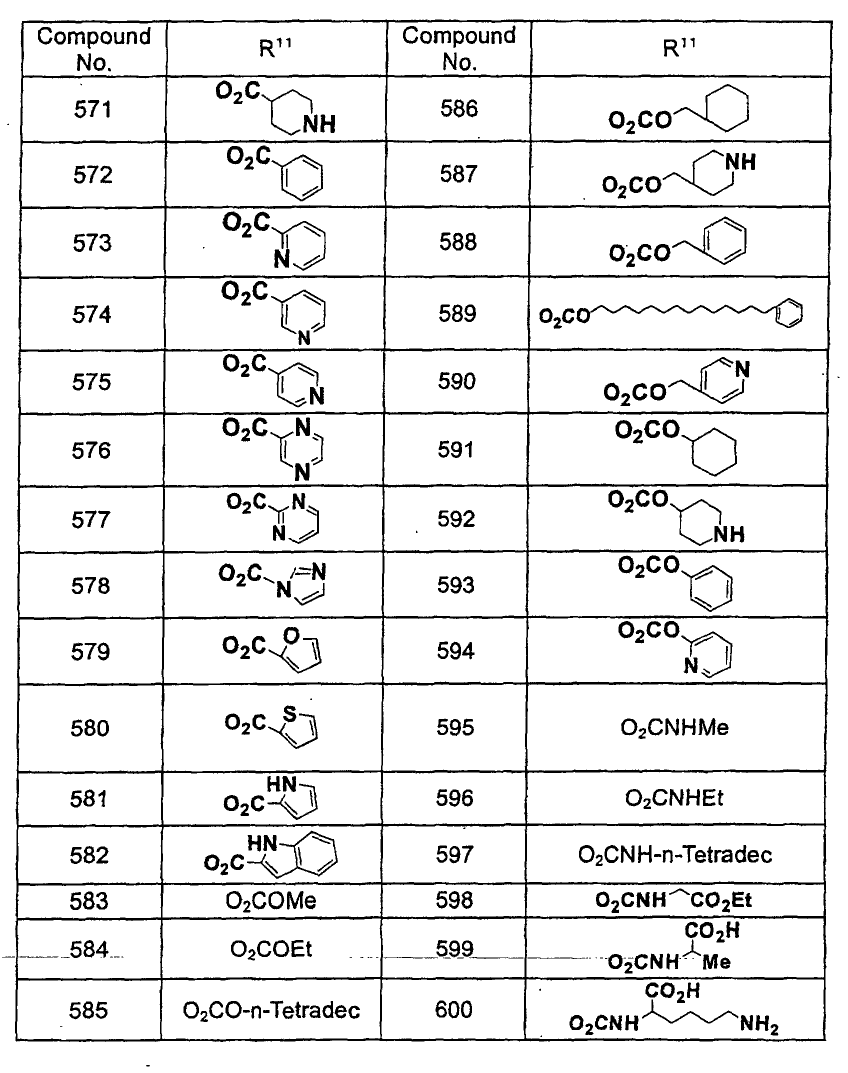

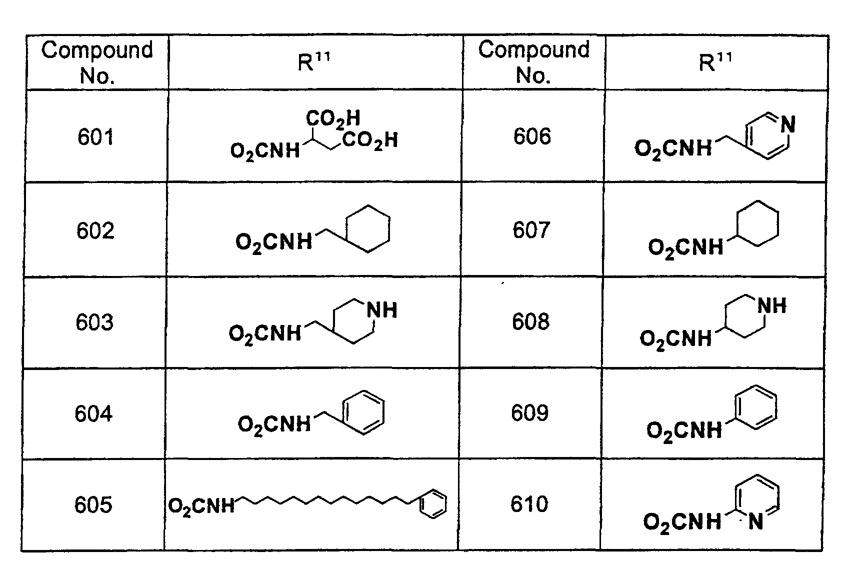

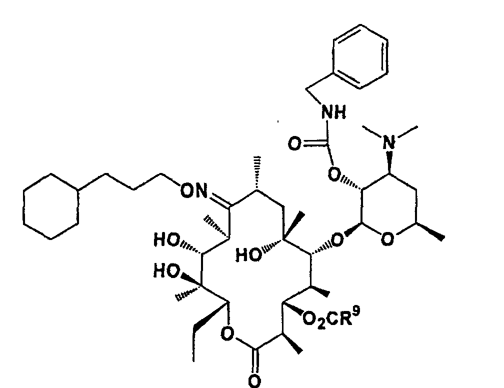

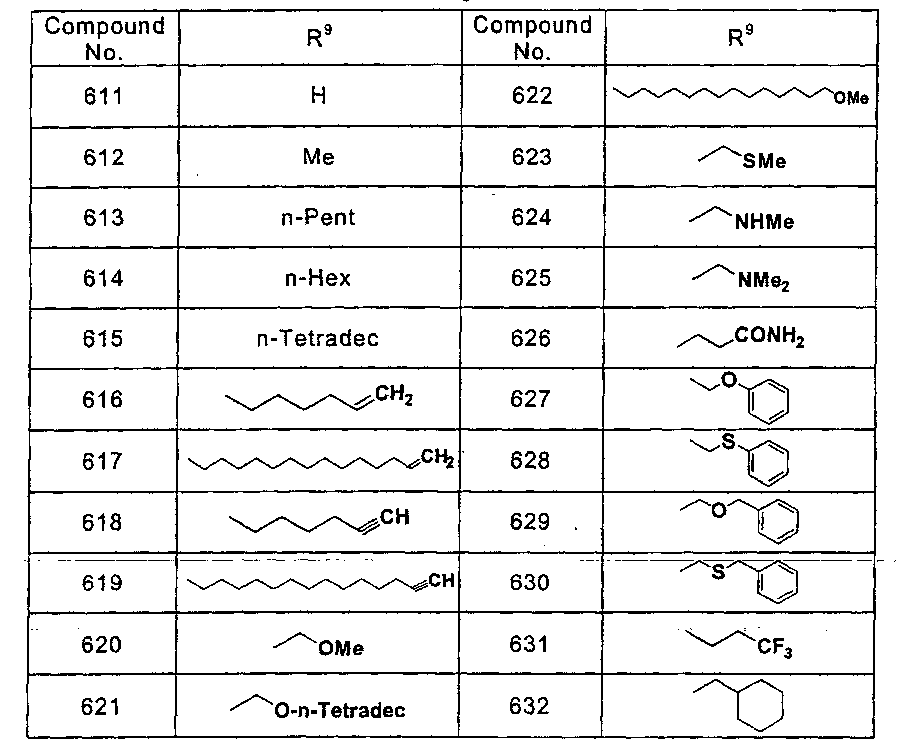

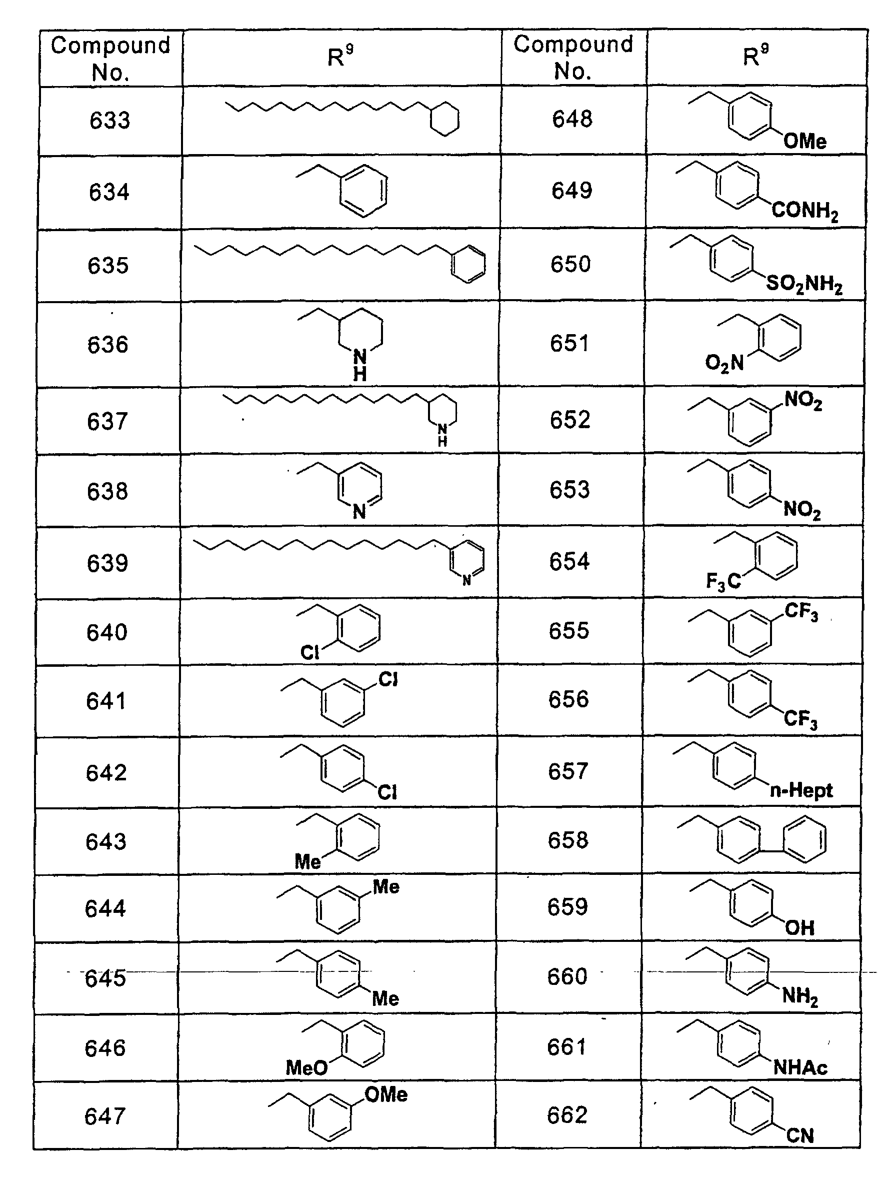

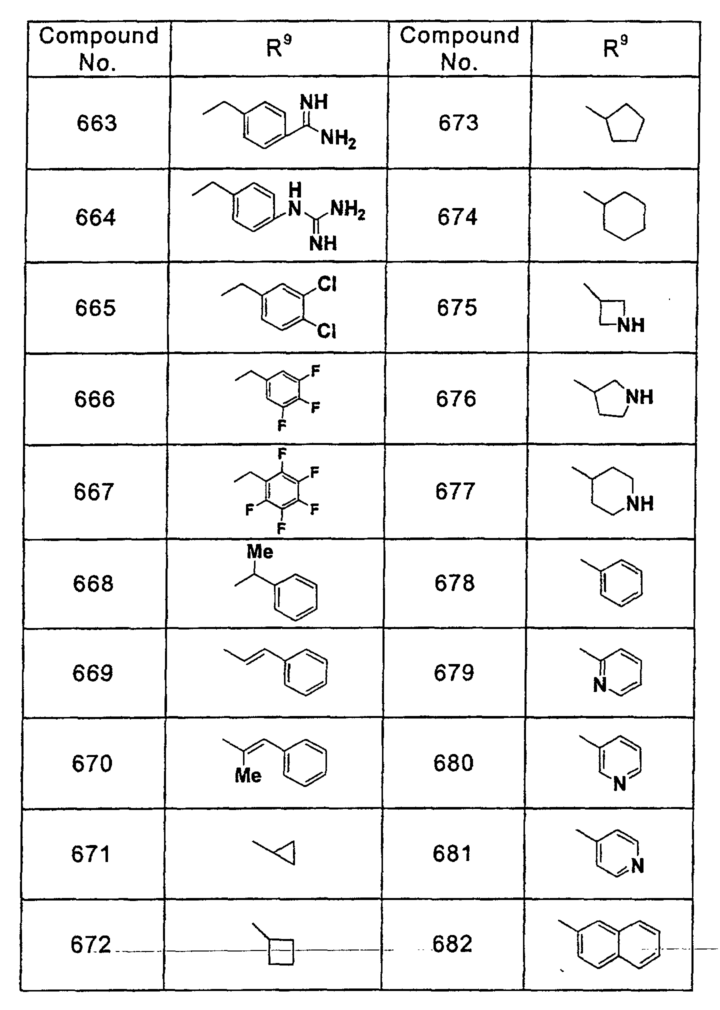

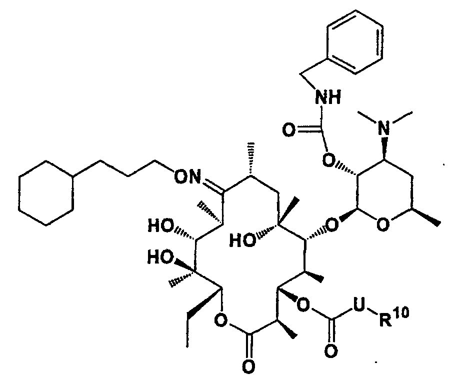

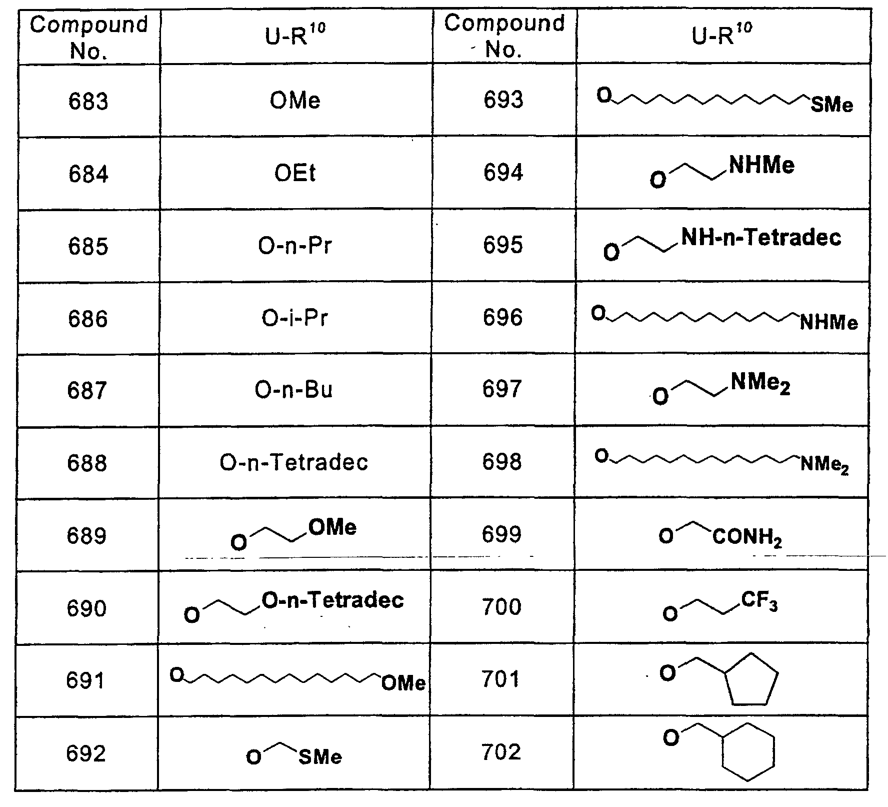

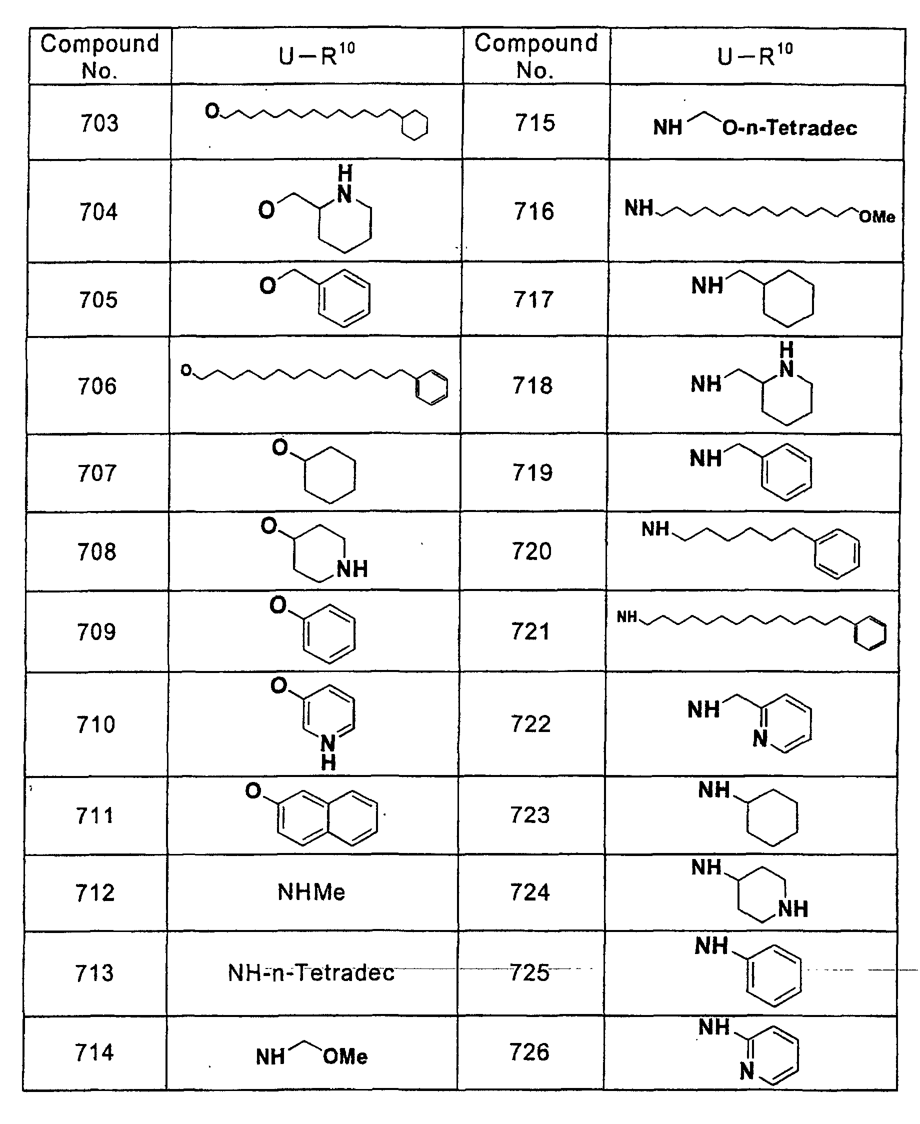

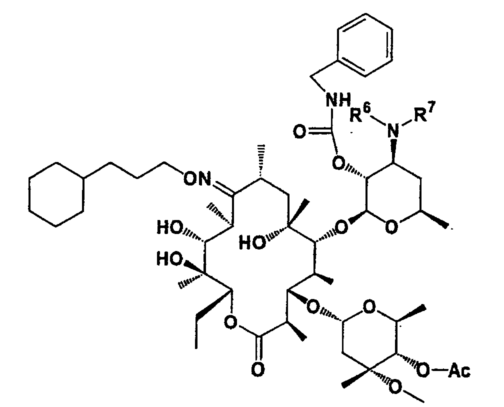

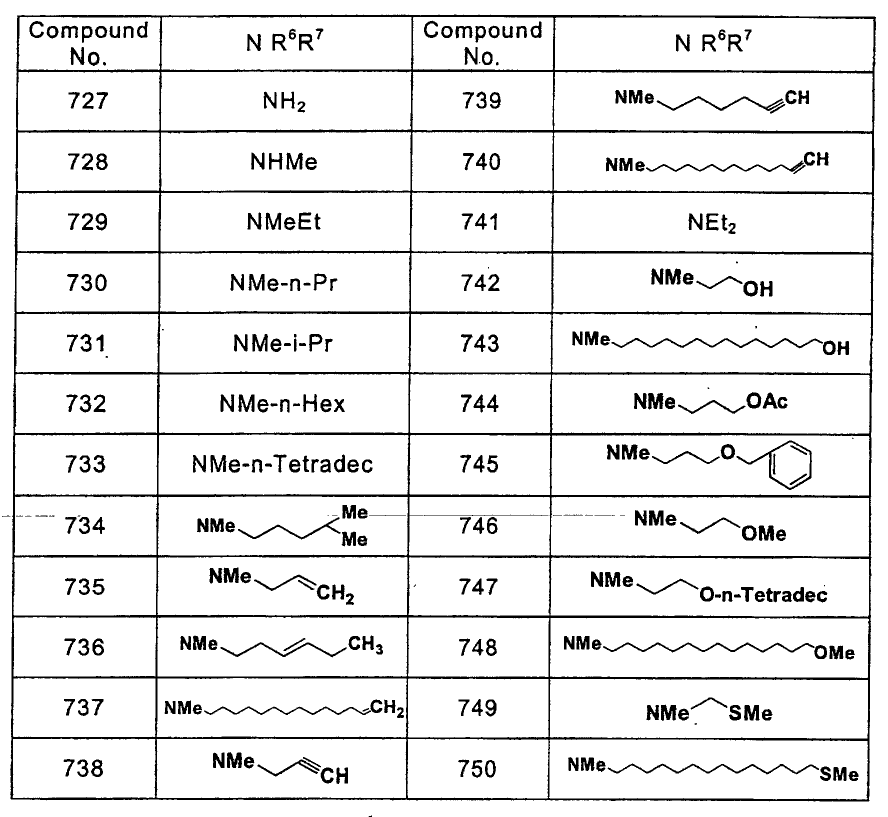

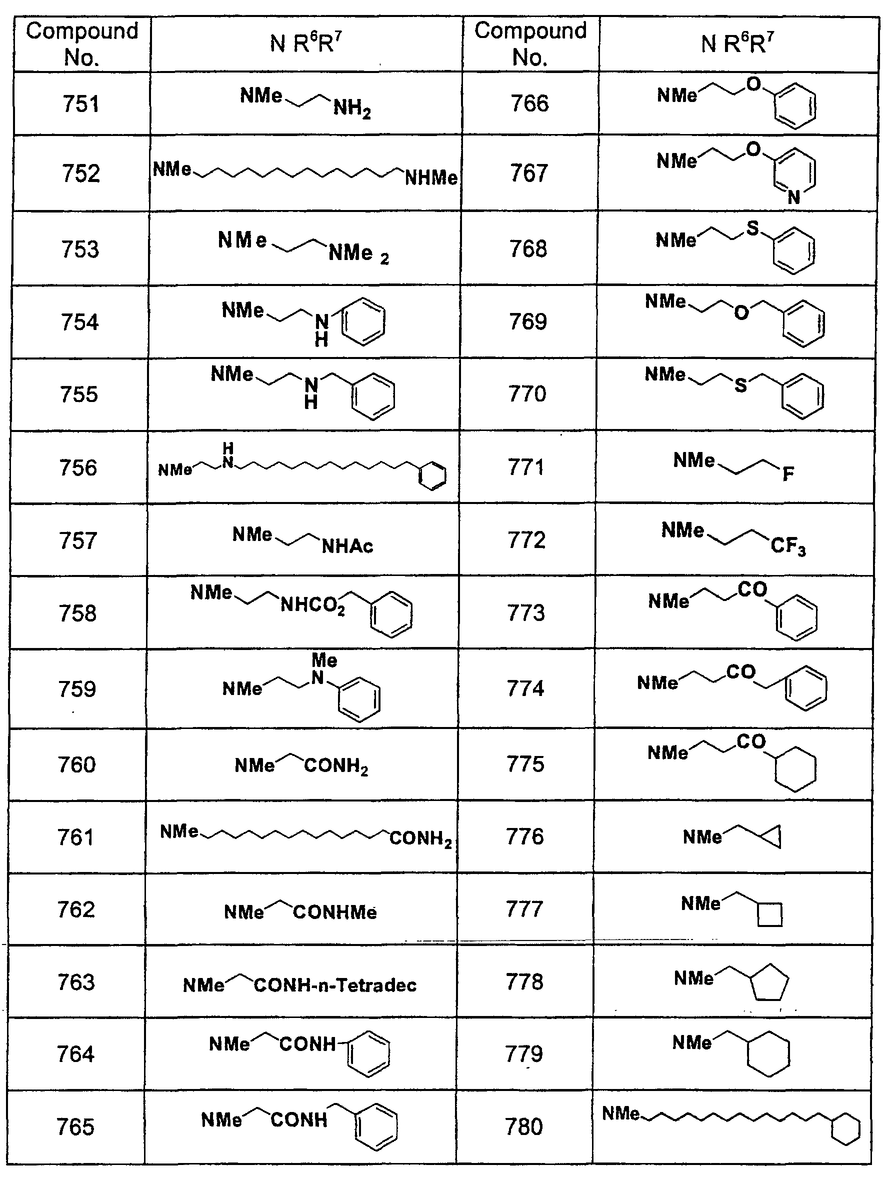

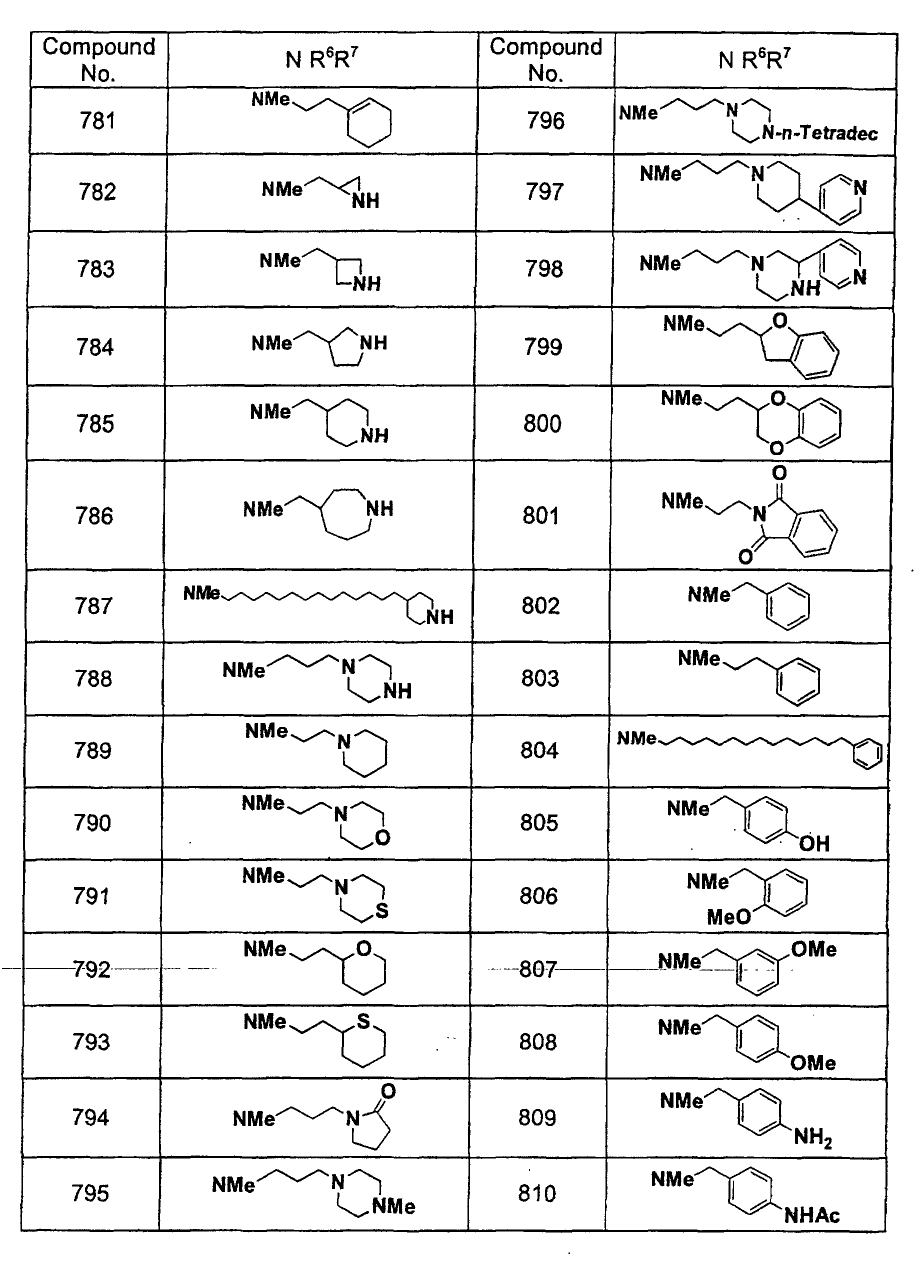

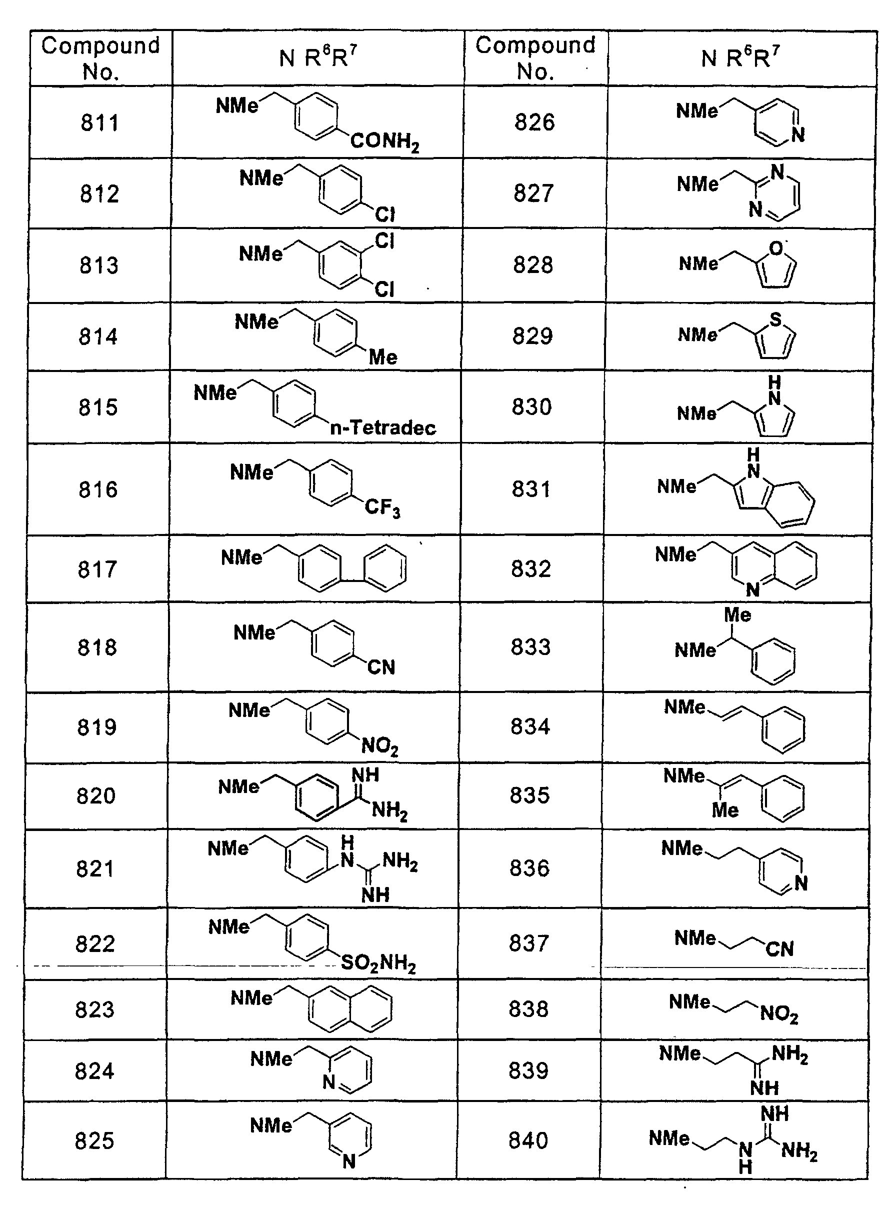

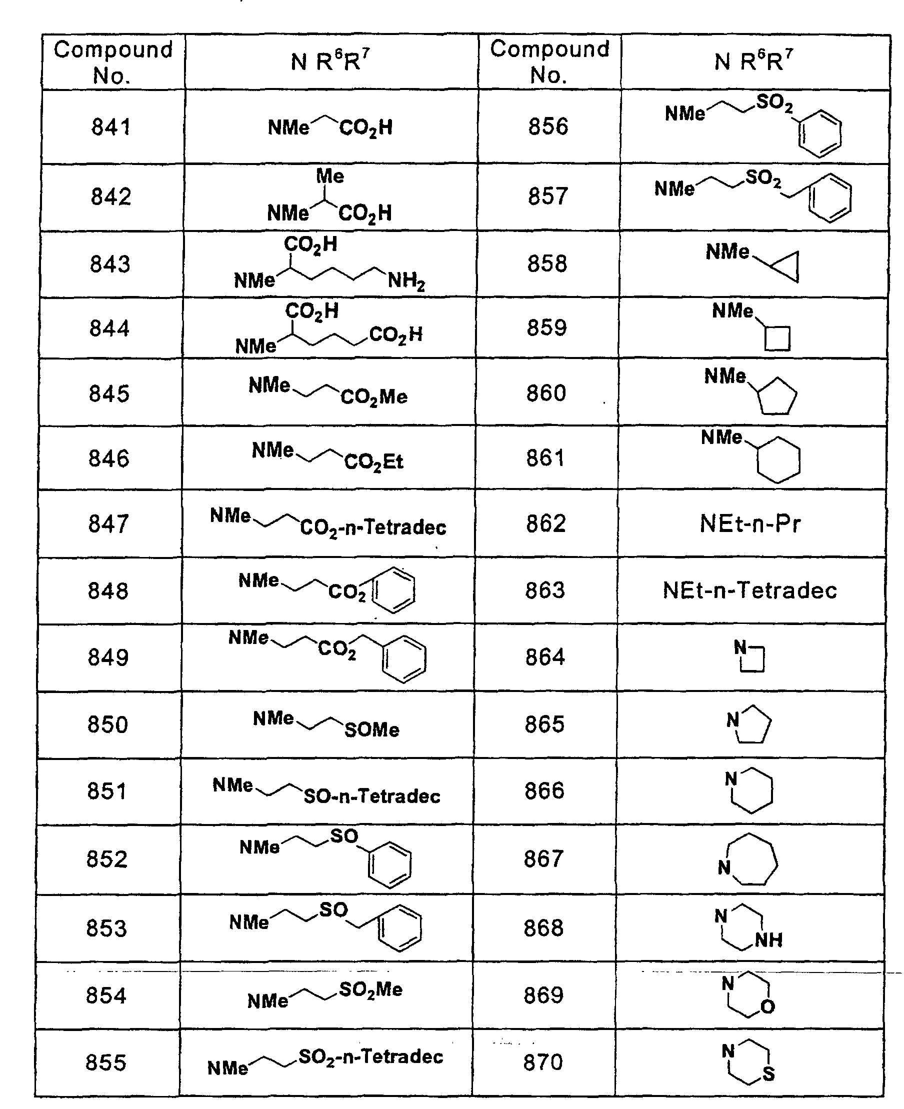

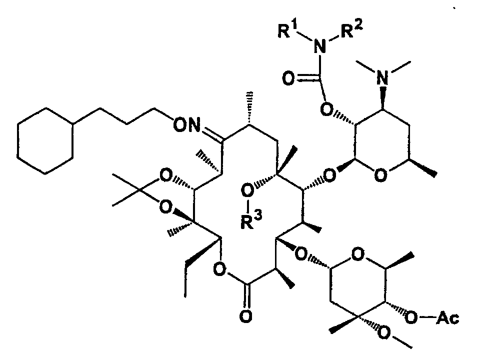

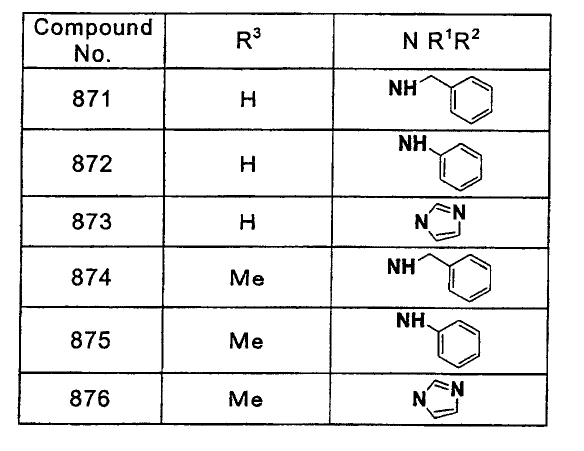

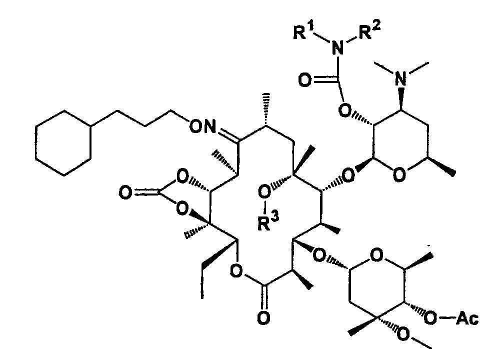

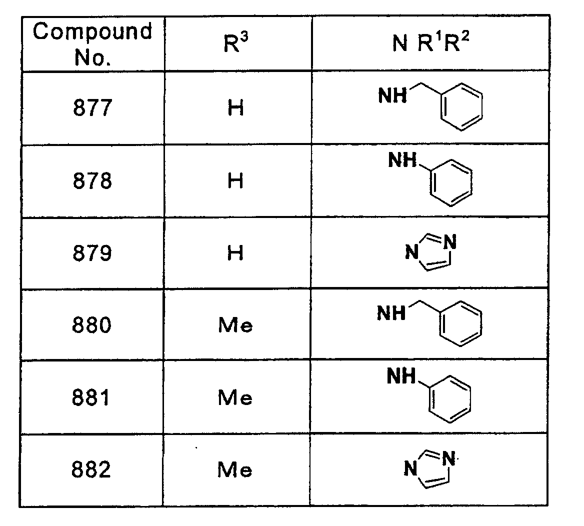

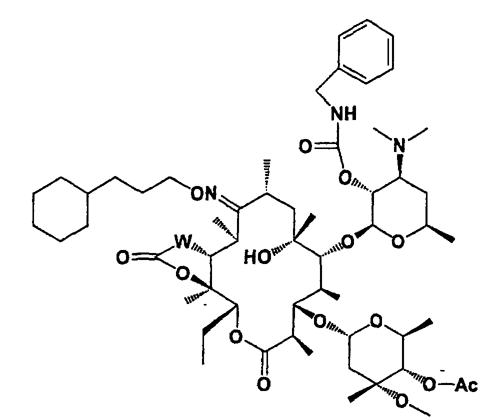

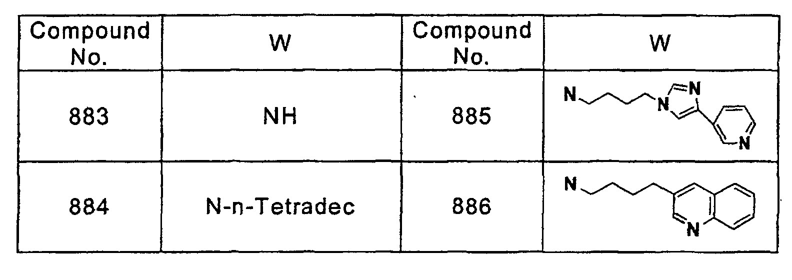

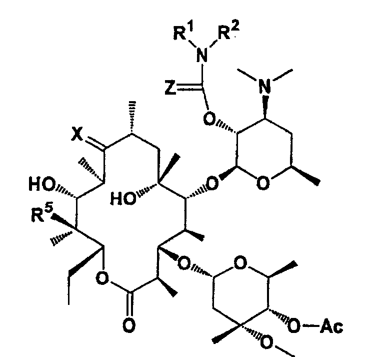

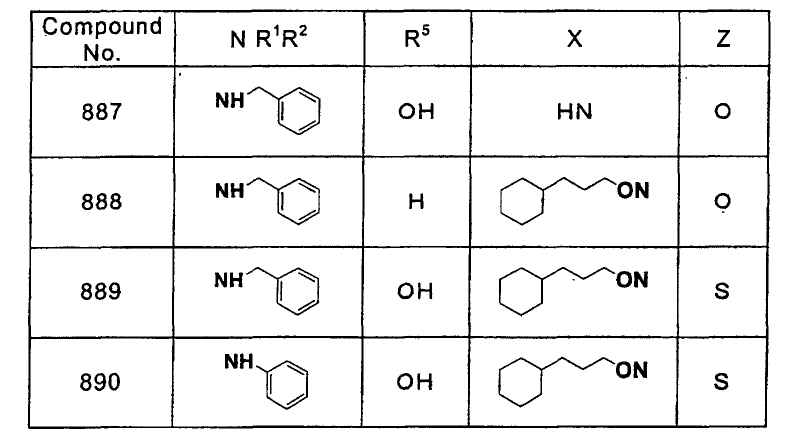

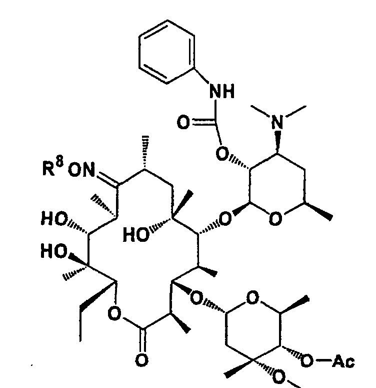

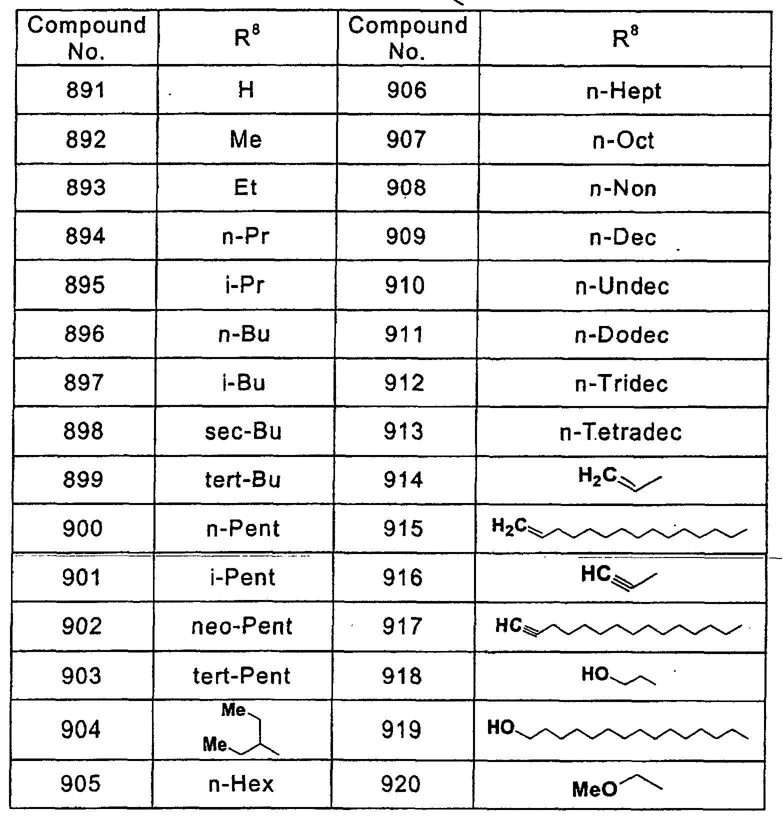

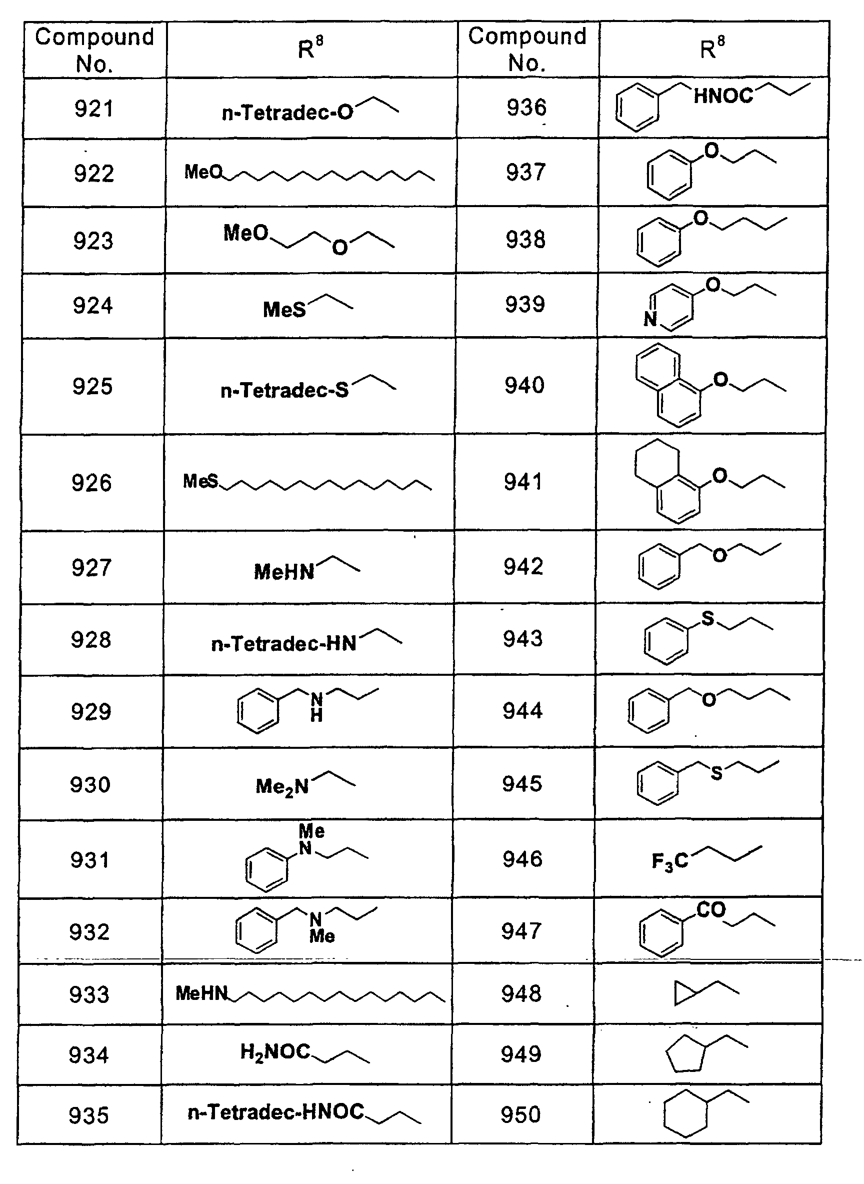

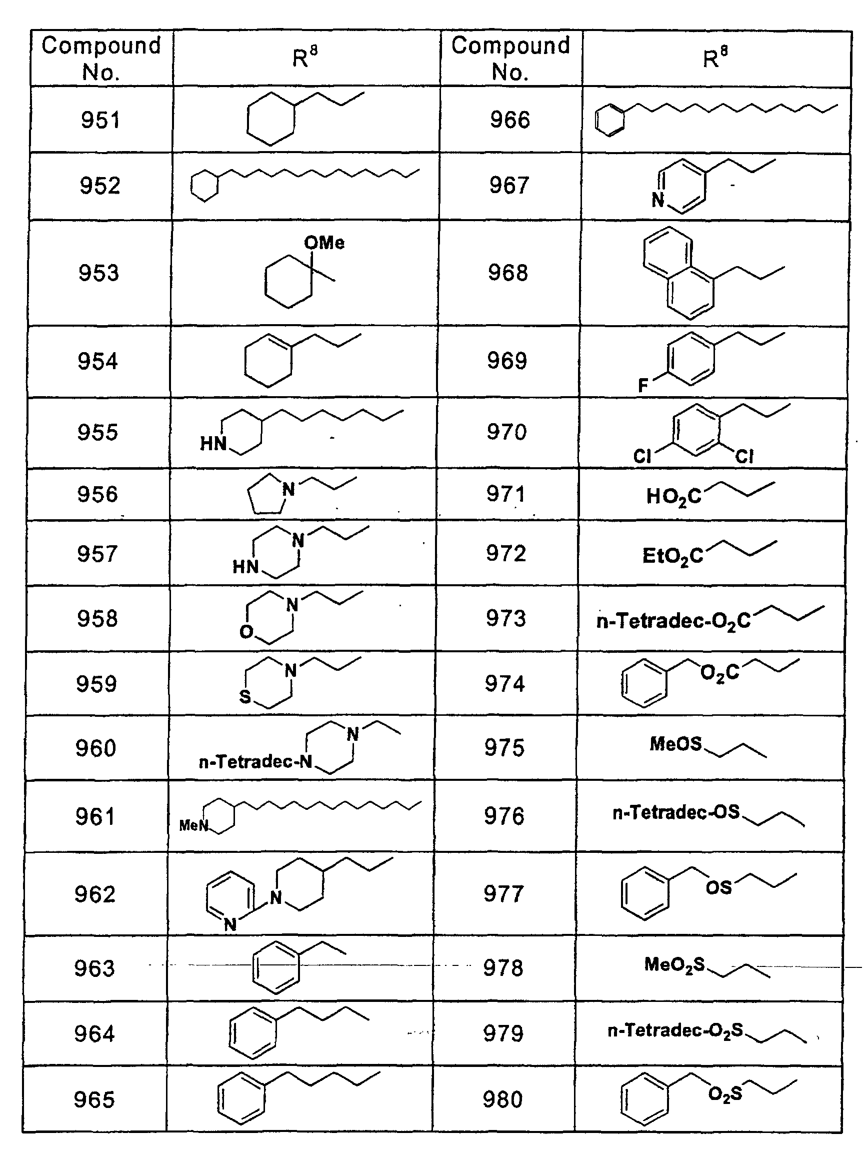

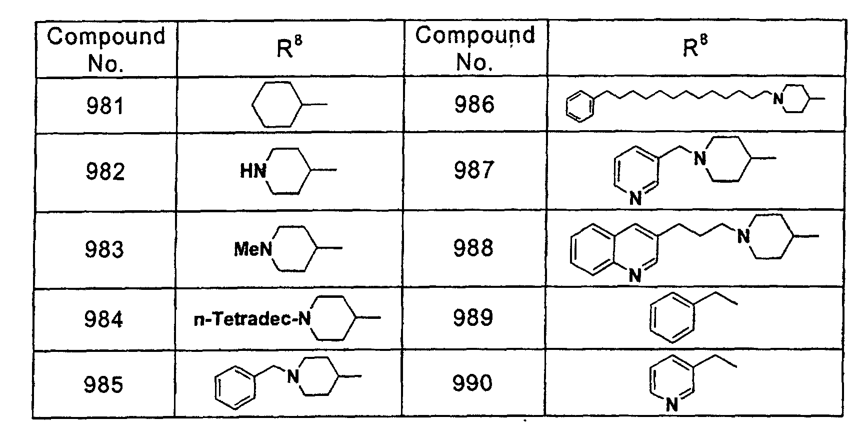

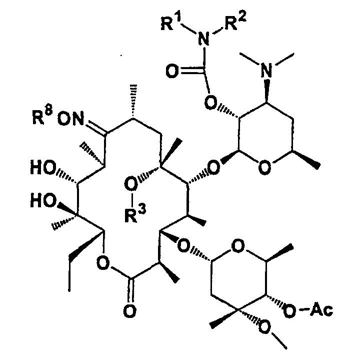

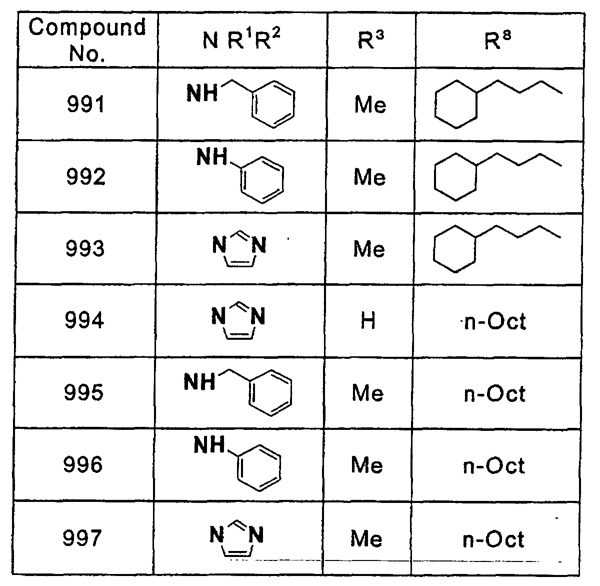

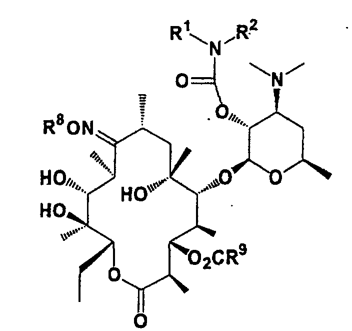

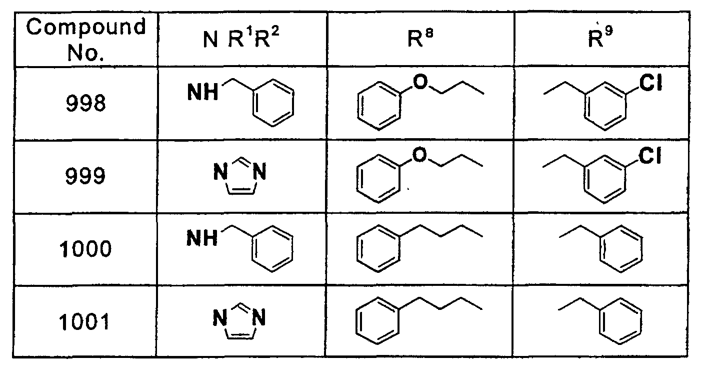

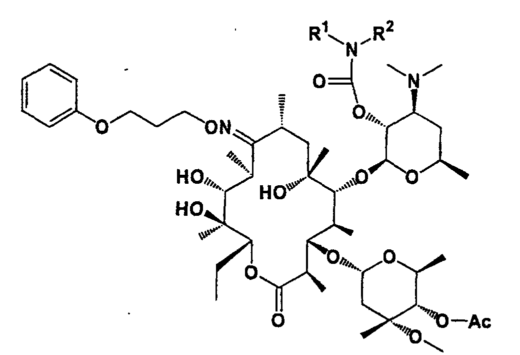

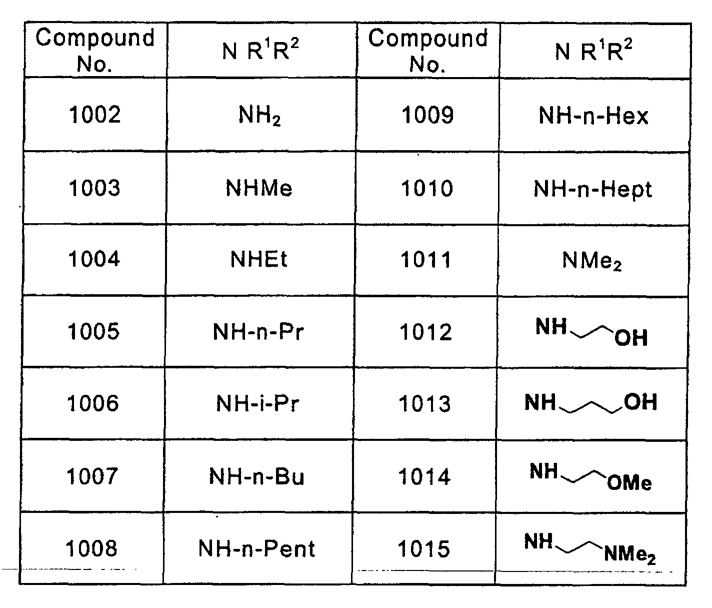

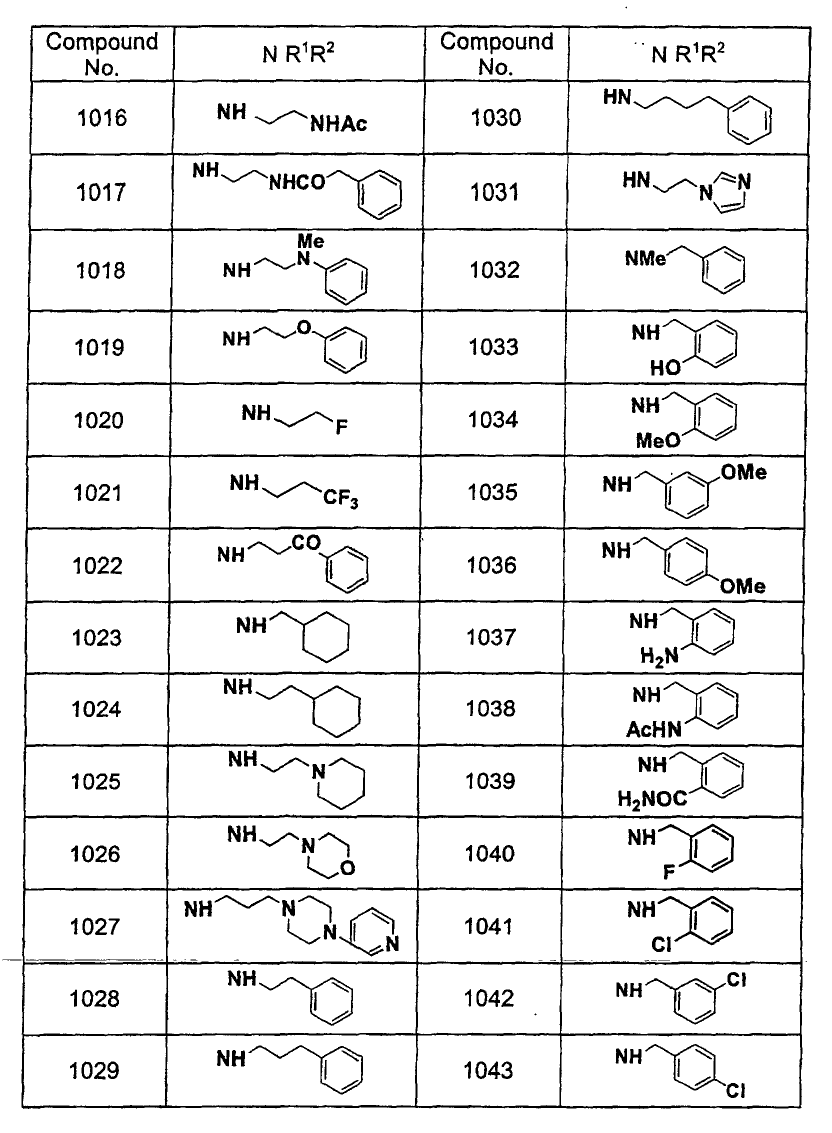

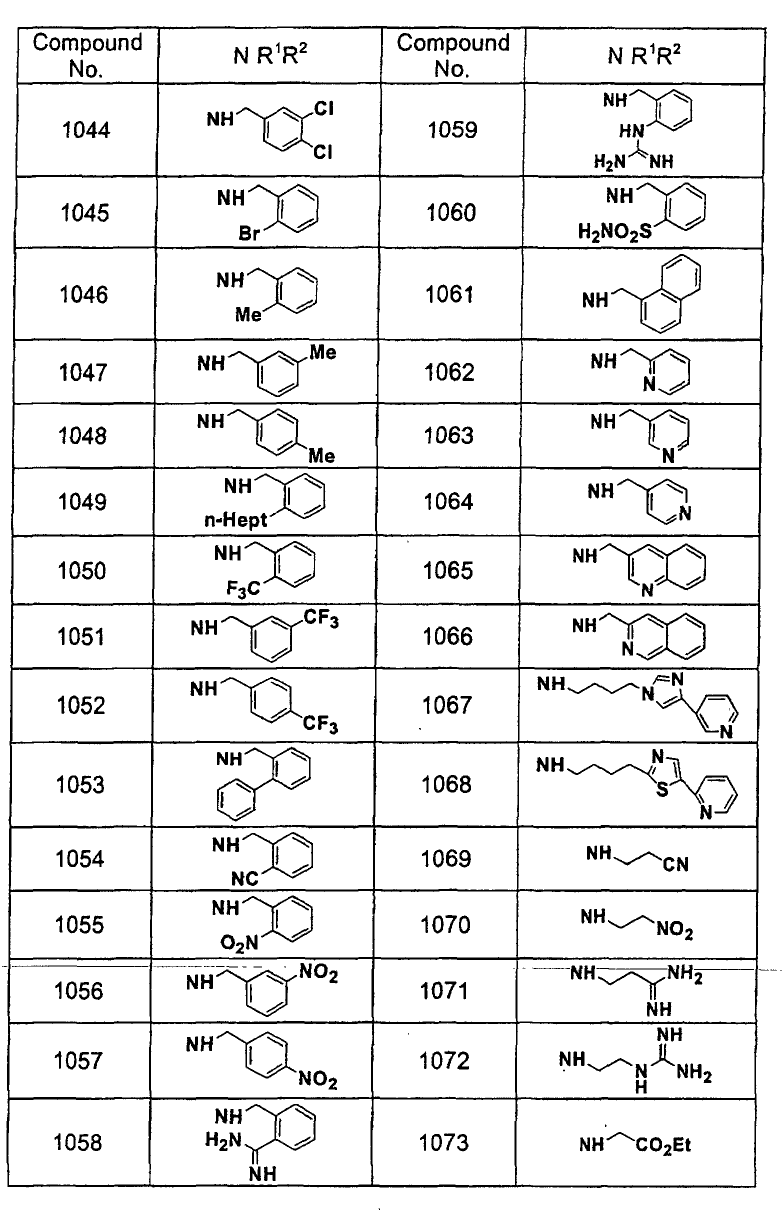

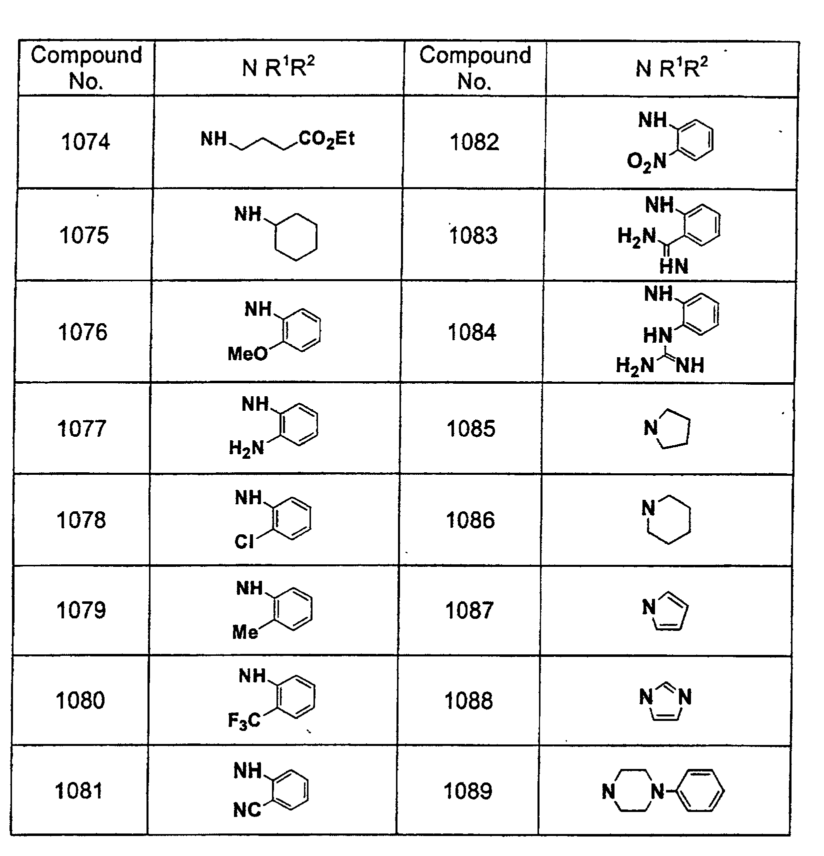

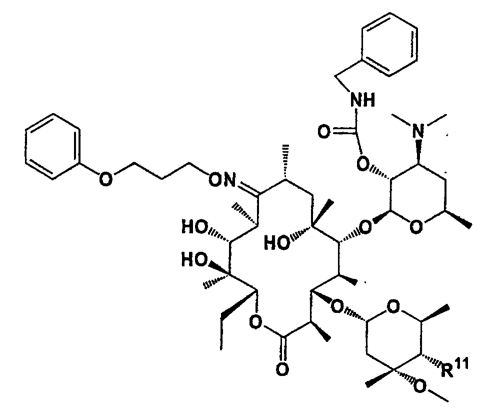

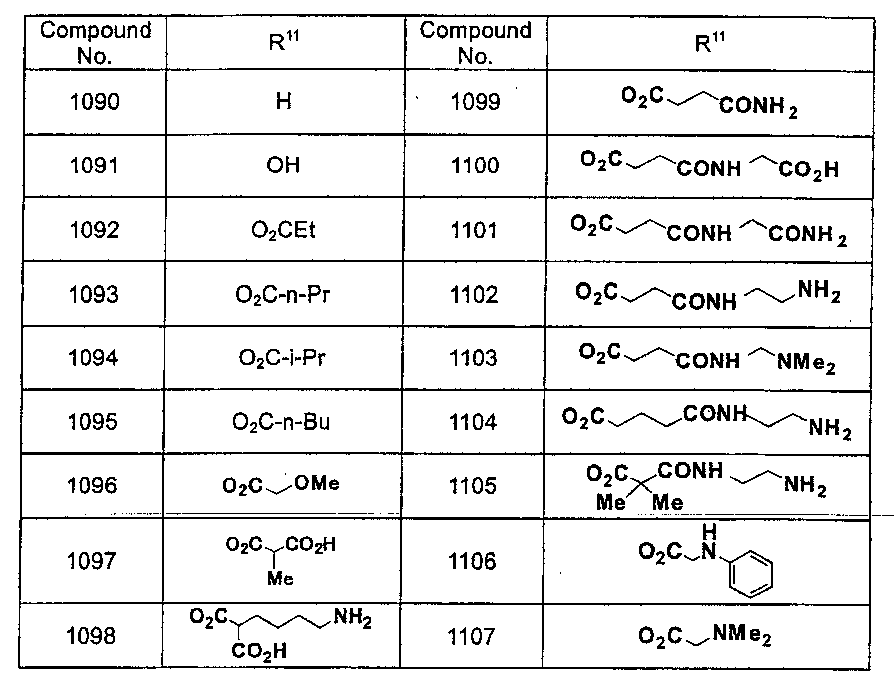

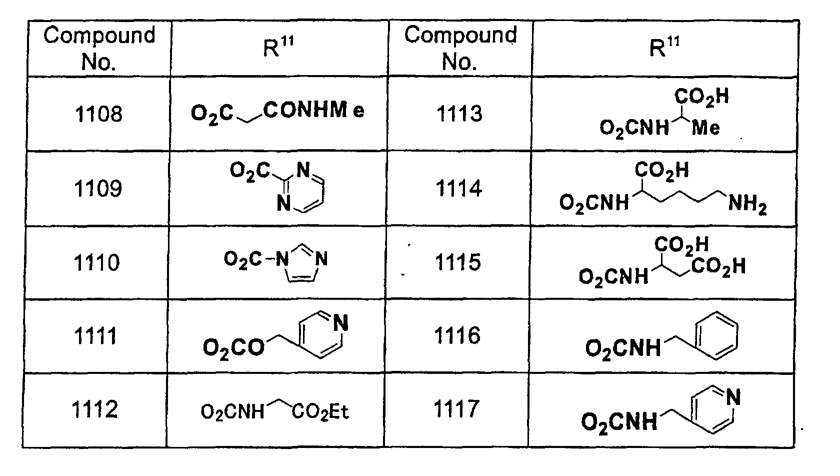

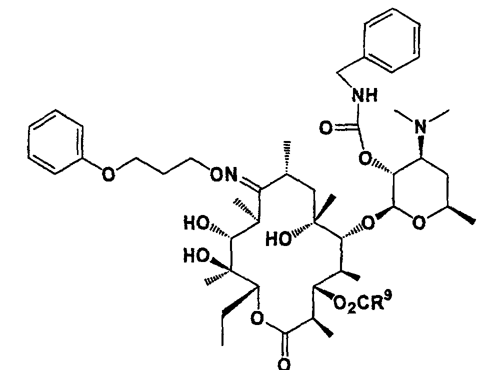

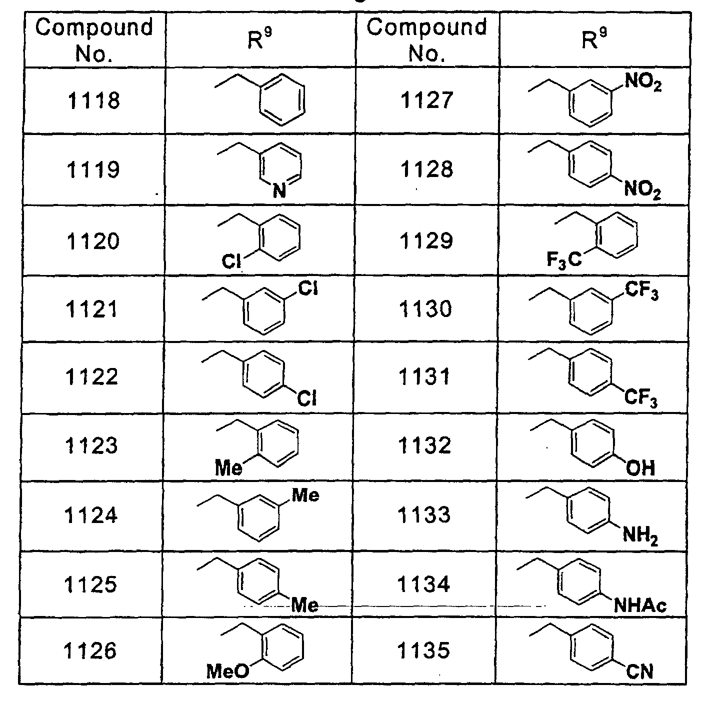

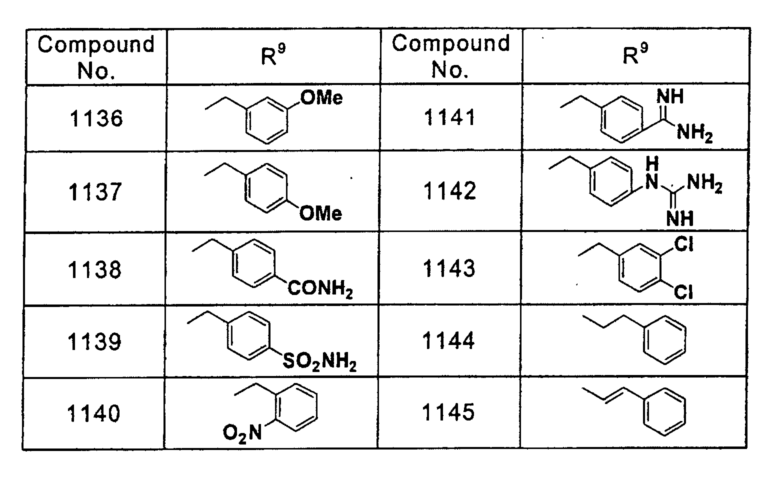

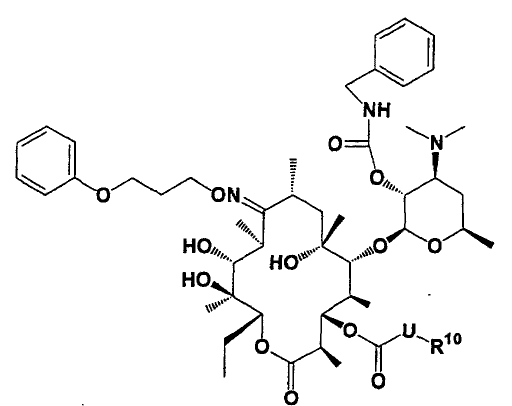

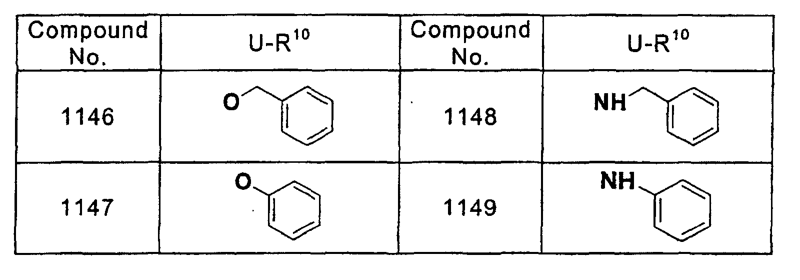

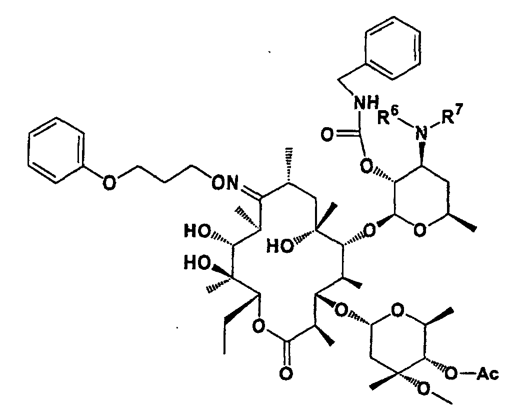

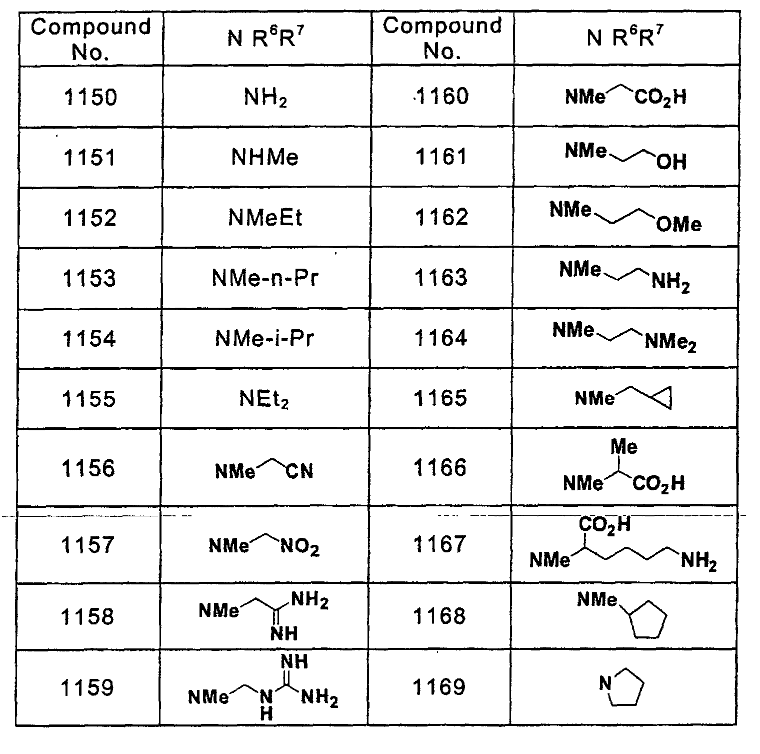

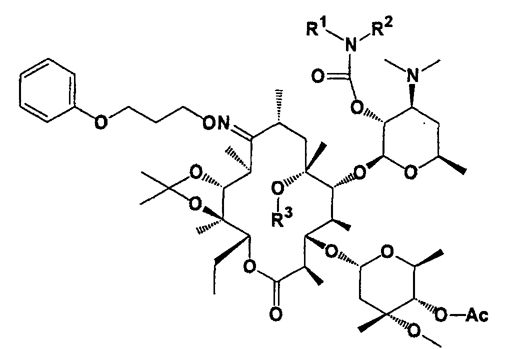

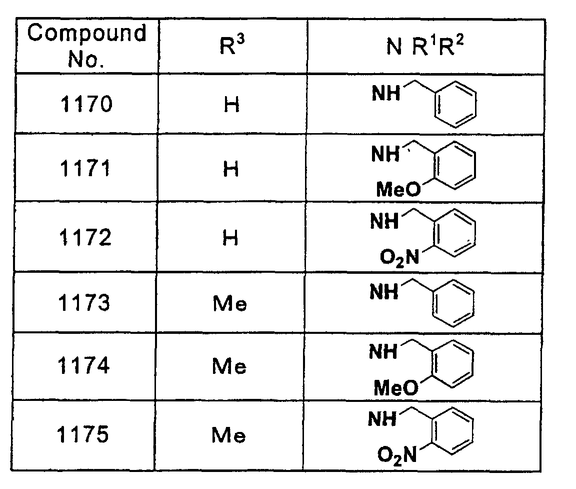

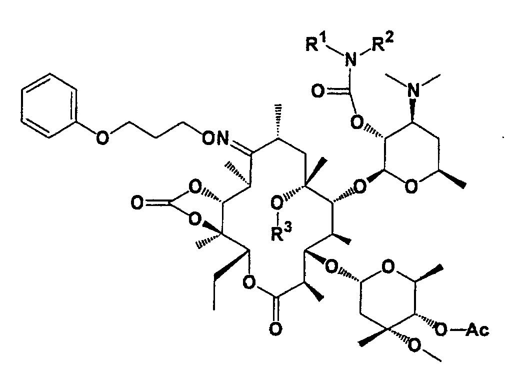

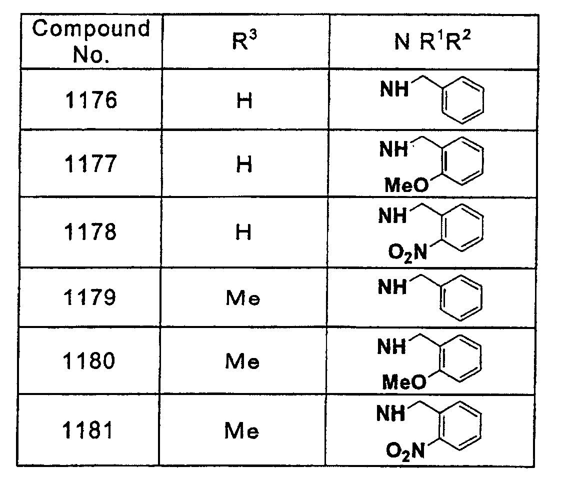

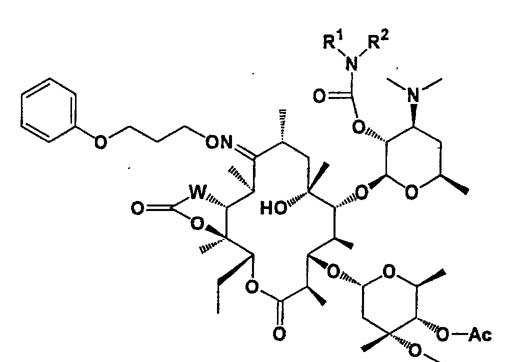

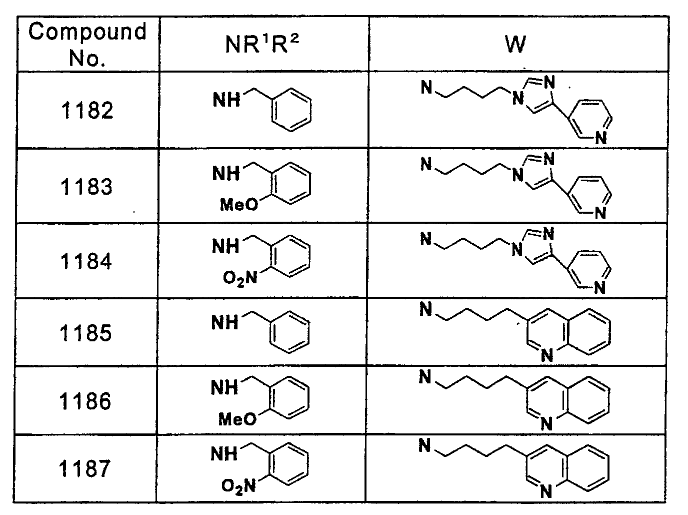

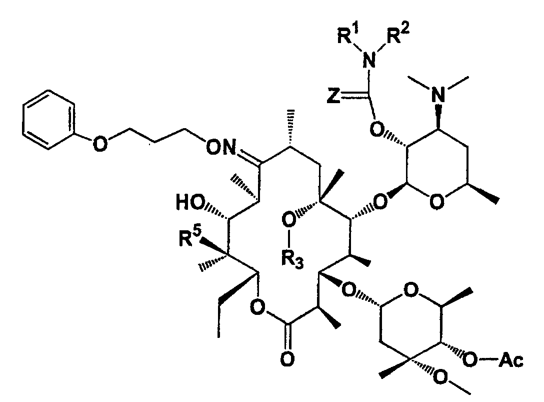

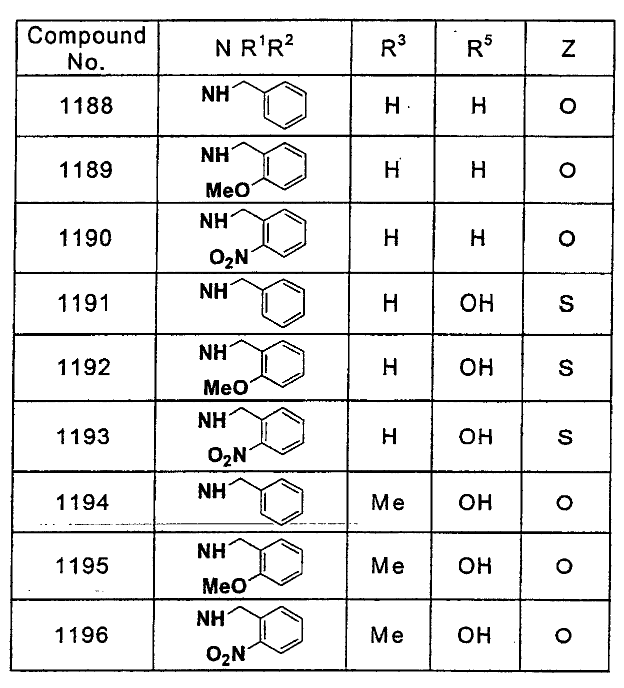

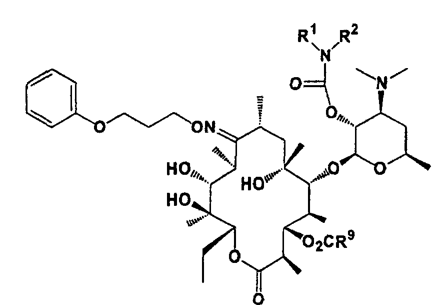

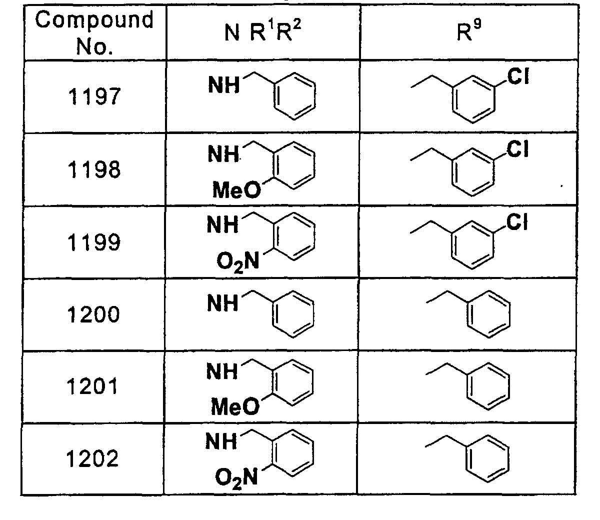

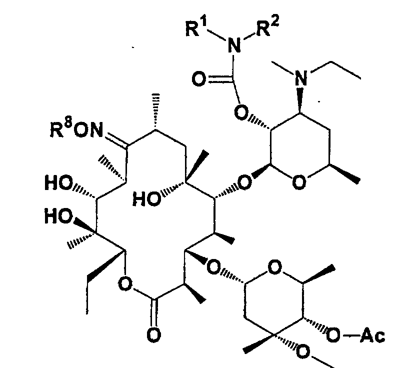

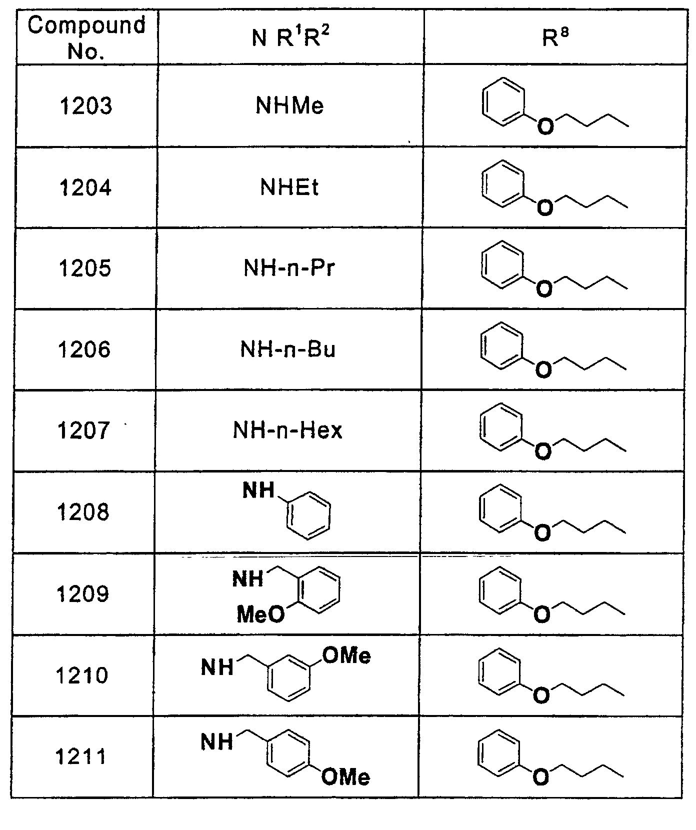

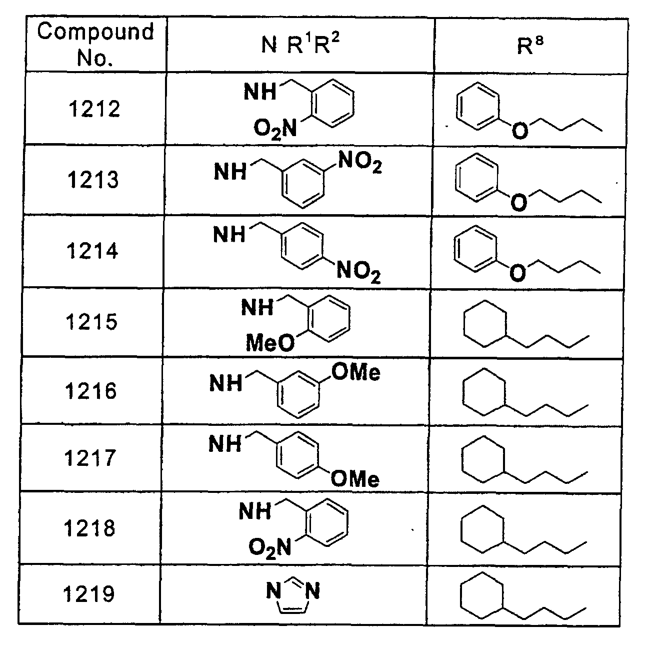

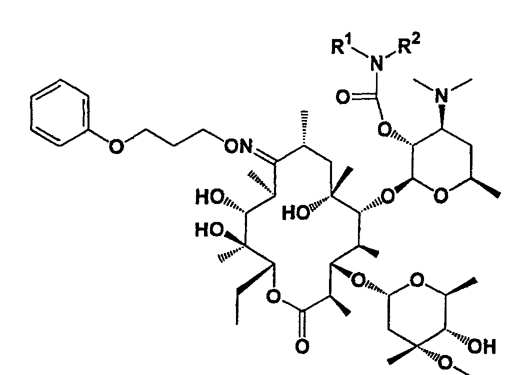

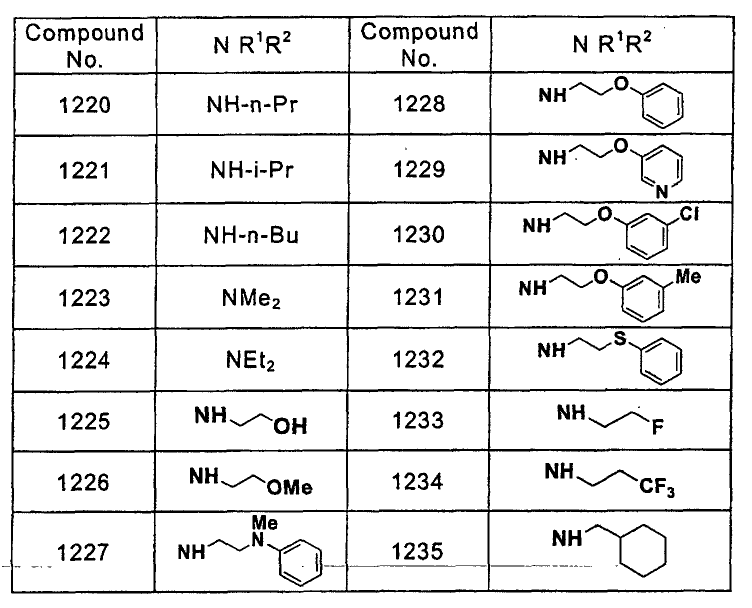

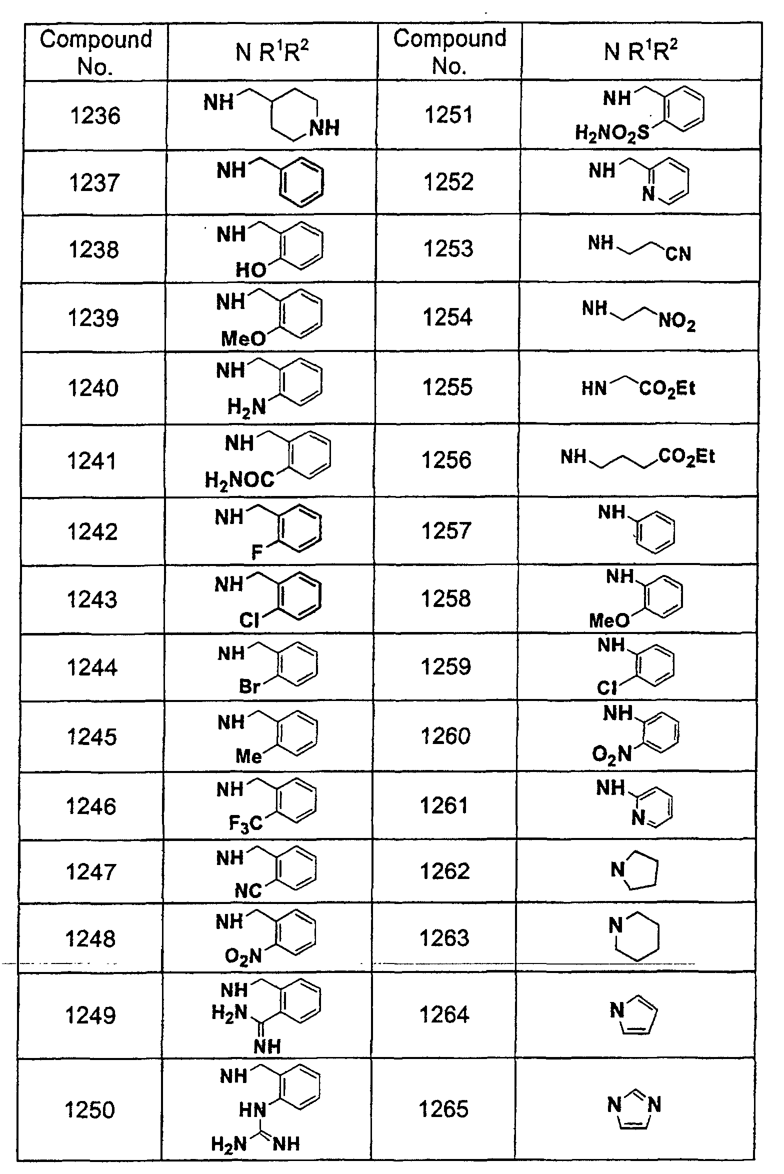

The novel erythromycin derivatives represented by the aforementioned general formula (I) of the present invention can be prepared by, for example, the methods explained below. However, the method for preparing the compounds of the present invention is not limited to these methods.

According to the first embodiment of the method for preparing the compounds of the present invention, the compounds represented by the aforementioned general formula (I) can be prepared by reacting the compound represented by the following general formula (V): 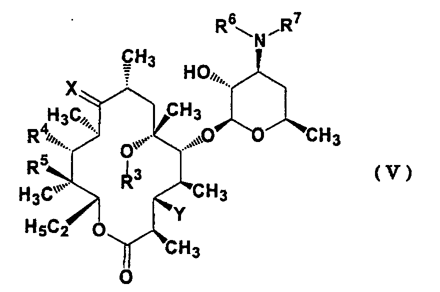 wherein R³, R⁴, R⁵, R⁸, R⁷, X, and Y have the same meaning as those defined above, with the compound represented by the following general formula (VI):

R¹⁴-N=C=Z (VI)

wherein Z has the same meaning as that defined above, and R¹⁴ represents an alkyl group which may be substituted, an alkenyl group which may be substituted, an alkynyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, in the presence or absence of lithium chloride in the absence or presence of a solvent. Alternatively, the target compound can be prepared by reacting the compound represented by the aforementioned general formula (V) with the compound represented by the following general formula (VII) in the presence or absence of a base in the absence or presence of a solvent:

R¹⁵R¹⁶N-C(=Z)-Q (VII)

wherein Z has the same meaning as that defined above, and R¹⁵ and R¹⁶ independently represent an alkyl group which may be substituted, an alkenyl group which may be substituted, an alkynyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, or R¹⁵ and R¹⁶ may combine together with the nitrogen atom to which they bind to form a saturated or unsaturated heterocyclic group which may further contain one or more heteroatoms selected from the group consisting of oxygen atom, sulfur atom, and nitrogen atom and which may be substituted, and Q represents a chlorine atom or 1-imidazolyl group.

Example of the base used in the above method for preparation include, for example, organic bases such as triethylamine, pyridine, diisopropylethylamine, 4-dimethylaminopyridine, 1,8-diazabicyclo[5.4.0]-7-undecene, 1,2,2,6,6-pentamethylpiperidine and the like, or inorganic bases such as sodium carbonate, potassium carbonate, sodium hydrogencarbonate, potassium hydrogencarbonate and the like. The solvent used in the above method for preparation may be any solvent so long as it, per se, is inert in the reaction and does not inhibit the reaction. Examples include halogenated-hydrocarbon-solvents-such as dichloromethane, 1,2-dichloroethane, and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide, and hexamethylenephosphoric triamide, ester solvents such as methyl acetate and ethyl acetate, ether solvents such as tetrahydrofuran, diethyl ether, and 1,4-dioxane, organic base solvents such as pyridine, picoline, lutidine, and collidine, or mixed solvents thereof. The reaction is performed at a temperature ranging from under ice-cooling to 200°C.

According to the second embodiment of the method for preparing the compounds of the present invention, the compounds represented by the following general formula (VIII) which fall within the compounds represented by the aforementioned general formula (I): 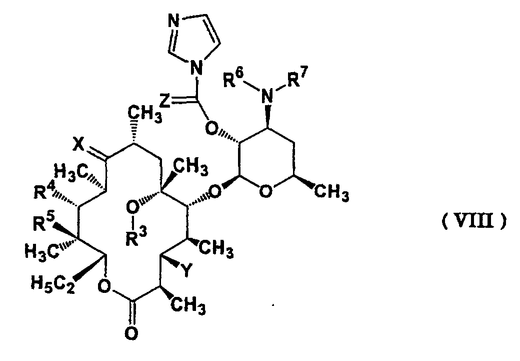 wherein R³, R⁴, R⁵, R⁶, R⁷, X, Y, and Z have the same meaning as those defined above, in which R¹ and R² combine together with the nitrogen atom to which they bind to form 1-imidazolyl group, can be prepared by reacting the compound represented by the aforementioned general formula (V) with the N,N'-carbonyldiimidazole or N,N'-thiocarbonyldiimidazole represented by the following general formula (IX) in the presence or absence of a base in the absence or presence of a solvent: 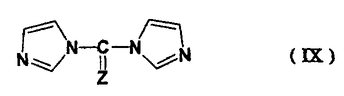 wherein Z has the same meaning as that defined above.

Example of the base used in the above method for preparation include, for example, organic bases such as triethylamine, pyridine, diisopropylethylamine, 4-dimethylaminopyridine, 1,8-diazabicyclo[5.4.0]-7-undecene, 1,2,2,6,6-pentamethylpiperidine and the like, or inorganic bases such as sodium carbonate, potassium carbonate, sodium hydrogencarbonate, potassium hydrogencarbonate and the like. The solvent used in the above method for preparation may be any solvent so long as it, per se, is inert in the reaction and does not inhibit the reaction. Examples include halogenated hydrocarbon solvents such as dichloromethane, 1,2-dichloroethane, and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide, and hexamethylenephosphoric triamide, ester solvents such as methyl acetate and ethyl acetate, ether solvents such as tetrahydrofuran, diethyl ether, and 1,4-dioxane, organic base solvents such as pyridine, picoline, lutidine, and collidine, or mixed solvents thereof. The reaction is performed at a temperature ranging from under ice-cooling to 200°C.

According to the third embodiment of the method for preparation of the compounds of the present invention, the compound represented by the aforementioned general formula (I) can be prepared by reacting the compound represented by the aforementioned general formula (VIII) which is obtained in the above second method, with the compound represented by the following general formula (X):

NHR¹R² (X)

wherein R¹ and R² have the same meaning as those defined above, in the presence or absence of a base in the absence or presence of a solvent.

The compound represented by the aforementioned general formula (VIII) obtained in the second method can be used as a raw material for the third method without isolation and purification to obtain the compound of the present invention.

Example of the base used in the above method for preparation include, for example, organic bases such as triethylamine, pyridine, diisopropylethylamine, 4-dimethylaminopyridine, 1,8-diazabicyclo[5.4.0]-7-undecene, 1,2,2,6,6-pentamethylpiperidine and the like, or inorganic bases such as sodium carbonate, potassium carbonate, sodium hydrogencarbonate, potassium hydrogencarbonate and the like. The solvent used in the above method for preparation may be any solvent so long as it, per se, is inert in the reaction and does not inhibit the reaction. Examples include halogenated hydrocarbon solvents such as dichloromethane, 1,2-dichloroethane, and chloroform, aromatic hydrocarbon solvents such as benzene and toluene, aprotic polar solvents such as acetone, acetonitrile, N,N-dimethylformamide, N-methyl-2-pyrrolidone, dimethyl sulfoxide, tetramethylene sulfolan, tetramethylene sulfoxide, and hexamethylenephosphoric triamide, ester solvents such as methyl acetate and ethyl acetate, ether solvents such as tetrahydrofuran, diethyl ether, and 1,4-dioxane, organic base solvents such as pyridine, picoline, lutidine, and collidine, or mixed solvents thereof. The reaction is performed at a temperature ranging from under ice-cooling to 200°C.

According to the forth embodiment of the method for preparation of the compounds of the present invention, the compounds among those represented by the aforementioned general formula (I) wherein a substituent on the optionally substitutable functional group is carboxyl group can be prepared by hydrolysis of compounds wherein a substituent on the optionally substitutable functional group is a substituted carboxyl group such as alkoxycarbonyl group, aryloxycarbonyl group, or aralkyloxycarbonyl group.

The hydrolysis can be performed by a method known per se under an acidic or alkaline condition in the presence or absence of a cation scavenger such as anisole and thioanisole in a solvent. For acidic hydrolysis, acids such as hydrochloric acid, an ethyl acetate solution of hydrogen chloride, an ethanolic solution of hydrogen chloride, sulfuric acid, hydrobromic acid, trifluoroacetic acid, p-toluenesulfonic acid, formic acid, and acetic acid can be used. For basic hydrolysis, bases such as hydroxides, carbonates, hydrogencarbonates, or alcoholate of an alkali metal such as sodium and potassium or of alkaline-earth metal such as magnesium and calcium can be used. As solvent for the hydrolysis, water, organic solvents such as methanol, ethanol, n-propanol, tetrahydrofuran, ethyl acetate, methylene chloride, 1,2-dichloroethane, 1,4-dioxane, N,N-dimethylformamide, and water-containing solutions of the above organic solvents may be used. The reaction can be carried out at a temperature ranging from 0°C to the reflux temperature of a solvent.

According to the fifth embodiment of the method for preparation of the compounds of the present invention, the compounds among those represented by the aforementioned general formula (I) wherein a substituent on the optionally substitutable functional group is an amino group can be prepared by hydrolysis or hydrogenolysis using a metal catalyst of compounds wherein a substituent on the optionally substitutable functional group is a substituted amino group such as benzylamino group, benzyloxycarbonylamino group, tert-butoxycarbonylamino group.

The hydrolysis can be performed by a method known per se according to the forth preparation method.

The hydrogenolysis can be performed by using a metal catalyst such as platinum, palladium/carbon, Raney nickel, and Pearlman's reagent in a solvent such as water, methanol, ethanol, n-propanol, acetic acid, and a mixed solvent thereof in the presence or absence of an acid such as hydrochloric acid at a temperature ranging from room temperature to the reflux temperature of the solvent under a hydrogen pressure ranging from normal pressure to 200 Pa.

The compound represented by the aforementioned general formula (V), used as the starting material for preparation of the compound of the present invention, can be prepared by referring to, for example, the publications set out below. Examples of the publication as references for preparation methods include Journal of Medicinal Chemistry, 17, 953, 1974; Journal of Medicinal Chemistry, 38, 1793, 1995; Japanese Patent Unexamined Publication No. (Sho)58-159500; Japanese Patent Unexamined Publication No. (Sho)61-225194; Japanese Patent Unexamined Publication No. (Sho)62-81399; Japanese Patent Unexamined Publication No. (Sho)62-292795; Japanese Patent Unexamined Publication No. (Sho)63-107921; Japanese Patent Unexamined Publication No. (Hei)5-255375; Japanese Patent Unexamined Publication No. (Hei)8-53355; Japanese Patent Unexamined Publication No. (Hei)8-104638; Japanese Patent Unexamined Publication No. (Hei)10-67795; Japanese Patent Unexamined Publication No. (Hei)11-116593; Japanese Patent Unexamined PublicationNo. (Hei)11-236395; U.S. Patent No. 6,034,069 and the like. According to the method described therein, each of the compounds in the following scheme can be prepared, wherein R³, R⁴, R⁵, R⁶, R⁷, R⁹, R¹⁰, R¹¹, U, and X have the same meaning as those defined above.

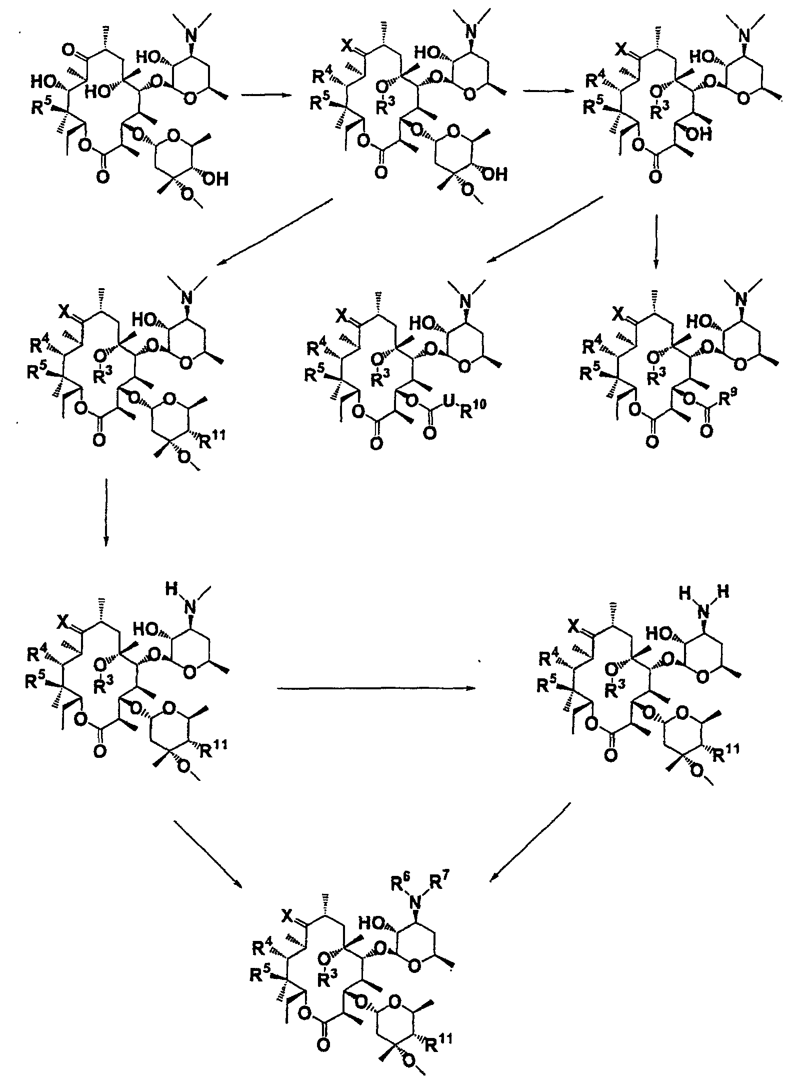

The medicament comprising, as an active ingredient, at least one substance selected from the group consisting of the novel erythromycin derivatives represented by the aforementioned general formula (I) thus prepared and pharmacologically acceptable salt thereof, and hydrate thereof and solvate thereof may be generally administered as oral formulations such as capsules, tablets, subtilized granules, granules, powders, syrups, dry syrups, solutions and the like, or as injections, suppositories, eye drops, eye ointments, ear drops, nasal drops, inhalants, dermal preparations and the like. These formulations can be prepared according to ordinary methods by addition of pharmacologically and pharmaceutically acceptable additives. As additives for oral formulations and suppositories, pharmaceutical ingredients such as, for example, excipients such as lactose, D-mannitol, corn starch and crystalline cellulose; disintegrating agents such as carboxymethylcellulose, calcium carboxymethylcellulose, partly pregelatinized starch, croscarmellose sodium, and crospovidone; binders such as hydroxypropylcellulose, hydroxypropylmethylcellulose, and polyvinylpyrrolidone; lubricants such as magnesium stearate, talc, hardened oil, dimethylpolysiloxane, hydrated silicon dioxide, colloidal silicon dioxide, and carnauba wax; coating agents such as hydroxypropylmethylcellulose, sucrose, and titanium oxide; plasticizers such as polyethylene glycol, triethyl citrate, and glycerin fatty acid esters; base materials such as polyethylene glycol and hard fat and the like may be used. For injections, eye drops, ear drops, and nasal drops, pharmaceutical ingredients such as, for example, dissolving agents and dissolving aids that can constitute aqueous preparations or preparations to be dissolved upon use such as distilled water for injection, physiological saline, and propylene glycol; pH modifiers such as inorganic or organic acids and bases; isotonic agents such as sodium chloride, glucose, and glycerin; stabilizers such as benzoic acid, citric acid, sodium bisulfate and the like may be used. For eye ointments and dermal preparations, pharmaceutical ingredients suitable for ointments, creams, and patches such as white soft paraffin, macrogol, grycerin, liquid paraffin, higher alcohols, fatty acid esters, glycerin fatty acid esters, polyethylene glycol fatty acid esters, carboxyvinyl polymers, acryl-type adhesives, rubber-type adhesives, silicone resins, cotton cloth and the like may be used. For inhalants, propellants such as carbon dioxide, propane, nitrogen gas and the like; dissolving aids such as ethanol, propylene glycol and the like; surfactants such as sorbitan trioleate and the like; and excipients such as lactose and the like may be used.

When the medicament of the present invention is administered to a patient, doses may be appropriately chosen depending on symptoms of the patient or route of administration. For example, generally for an adult, a daily dose (weight of an active ingredient) of about 10 to 2,000 mg for oral administration, or about 1 to 1,000 mg for parenteral administration may be administered once a day or several times as divided portions. It is desirable that the doses are suitably increased or decreased depending on the purpose of therapeutic or preventive treatment, a part of infection and a type of pathogenic bacteria, the age and symptoms of a patient and the like.

### EXAMPLES

The present invention will be explained more concretely by Examples. However, the scope of the present invention is not limited to these examples. The abbreviations in the tables have the following meanings : Me, methyl group; Et, ethyl group; n-Pr, n-propyl group; n-Bu, n-butyl group; n-Hex, n-hexyl group; n-Oct, n-octyl group; n-Dec, n-decyl group; n-Dodec, n-dodecyl group; n-Tetradec, n-tetradecyl group; Ac, acetyl group.

### Example 1: 4"-O-Acetyl-2'-O-phenylaminocarbonylerythromycin A 9-[O-(n-octyl)oxime]

To a solution of 0.50 g of 4"-O-acetylerythromycin A 9-[O-(n-octyl)oxime] in 5.0 ml of tetrahydrofuran, 0.12 ml of phenyl isocyanate was added, and the mixture was stirred at room temperature for 30.5 hours. And then, the reaction mixture was stirred at 50°C of outer temperature for 17 hours. The reaction mixture was concentrated under reduced pressure, and the obtained residue was added with water, and alkalified by saturated aqueous solution of sodium hydrogencarbonate, and extracted with ethyl acetate. The extract was washed successively with water and saturated brine, and dried over sodium sulfate, and the solvent was evaporated under reduced pressure. The residue was purified by column chromatography (silica gel, ethyl acetate : n-heptane = 1 : 1) to obtain 0.50 g of a colorless amorphous solid. NMR spectrum δ (CDCl₃)ppm:0.81(3H,t,J=7.5Hz),0.88(3H,t,J=7Hz),0.97(3H,d,J=7.5 Hz),1.00-1.78(44H,m),1.83-2.00(2H,m);2.12(3H,s),2.28(6H,s),2.40(1H,d,j=15.5Hz),2.58 -2.65(2H,m),2.77-2.85(1H,m),3.08(1H,s),3.10(3H,s),3.52(1H,d,J=8Hz),3.55-3.70(2H,m), 3.67(1H,s),3.94-4.02(2H,m),3.96(1H,d,J=10.5Hz),4.42(1H,s),4.43-4.50(1H,m),4.47(1H,d ,J=7.5Hz),4.74(1H,d,J=10Hz),4.76(1H,dd,J=10.5,7.5Hz),4.90(1H,d,J=4.5Hz),5.08(1H,d d,J=11,2Hz),7.04(1H,t,J=7.5Hz),7.22-7.32(2H,m),7.45(2H,d,J=7.5Hz)

In accordance with the method of Example 1, the compounds of Examples 2 through 34 were obtained.

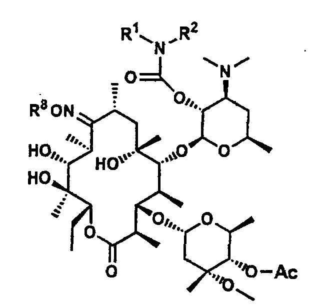

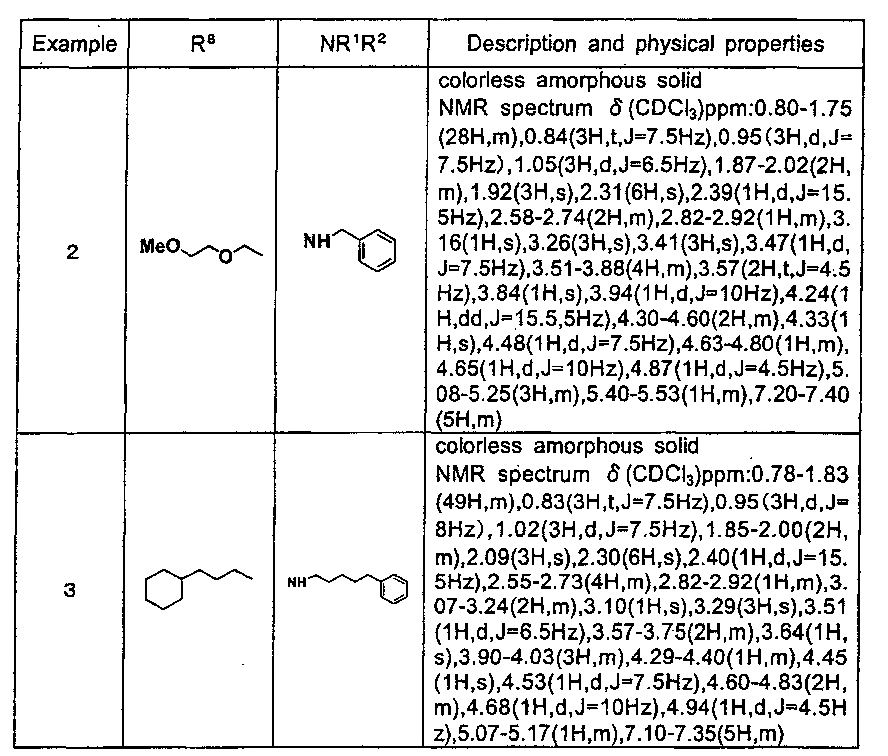

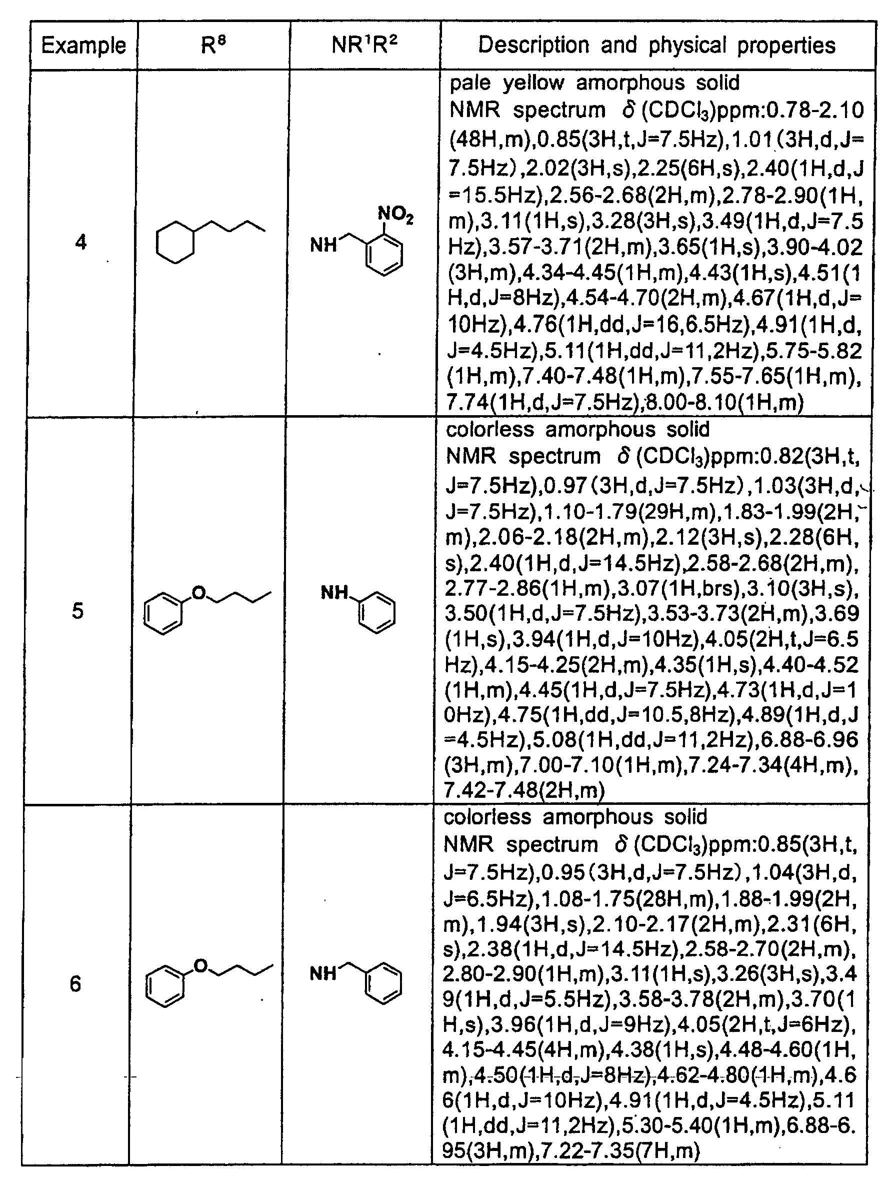

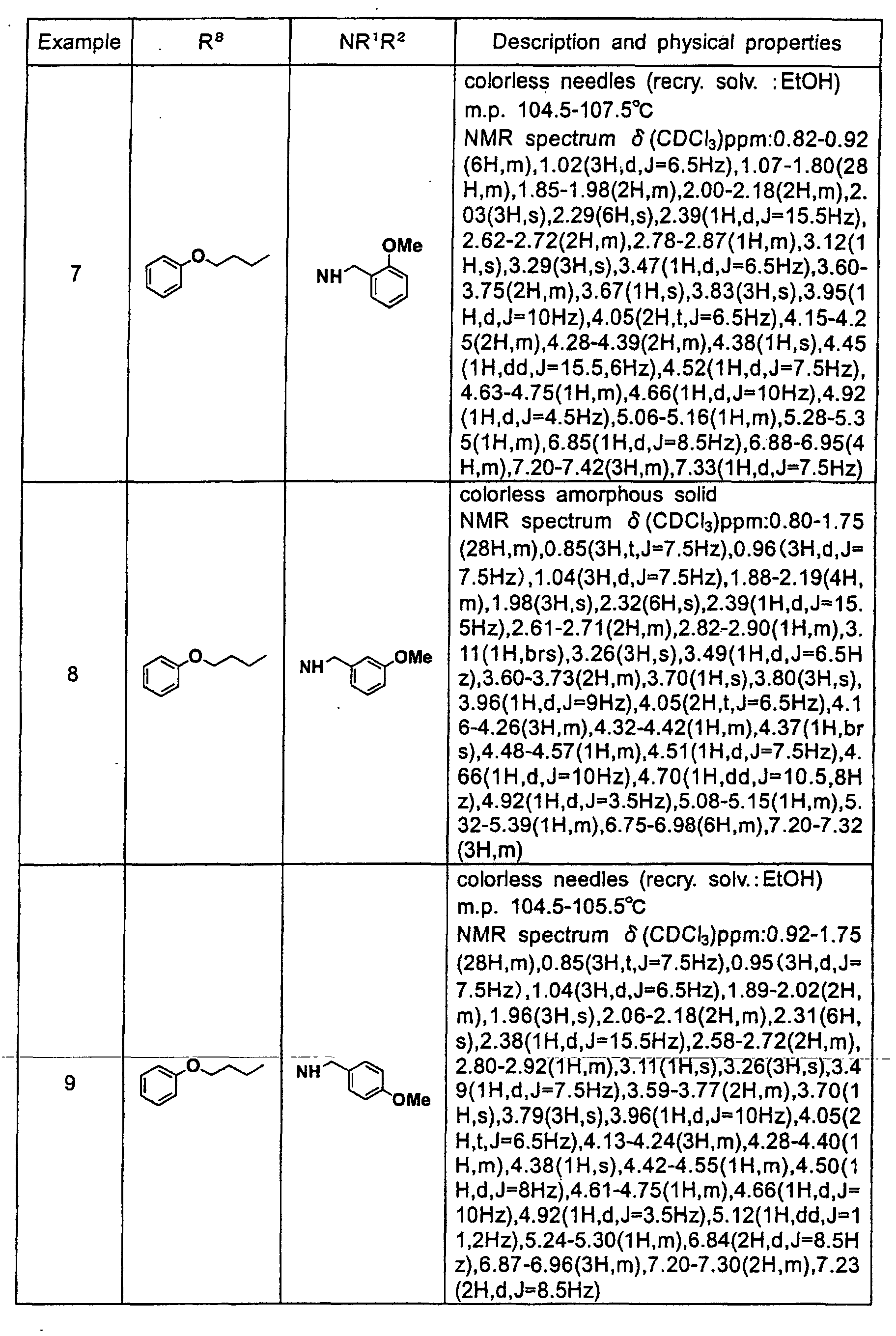

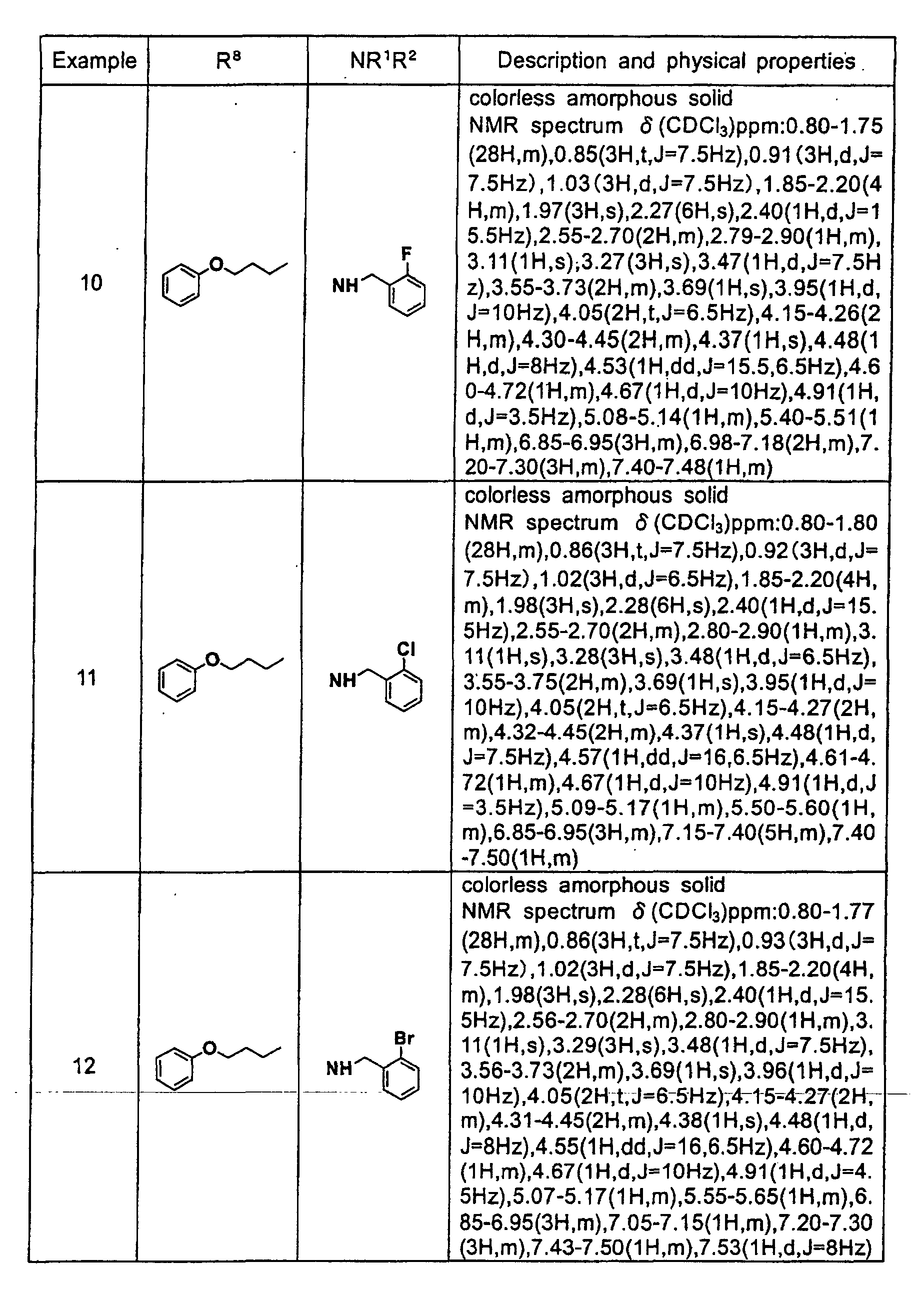

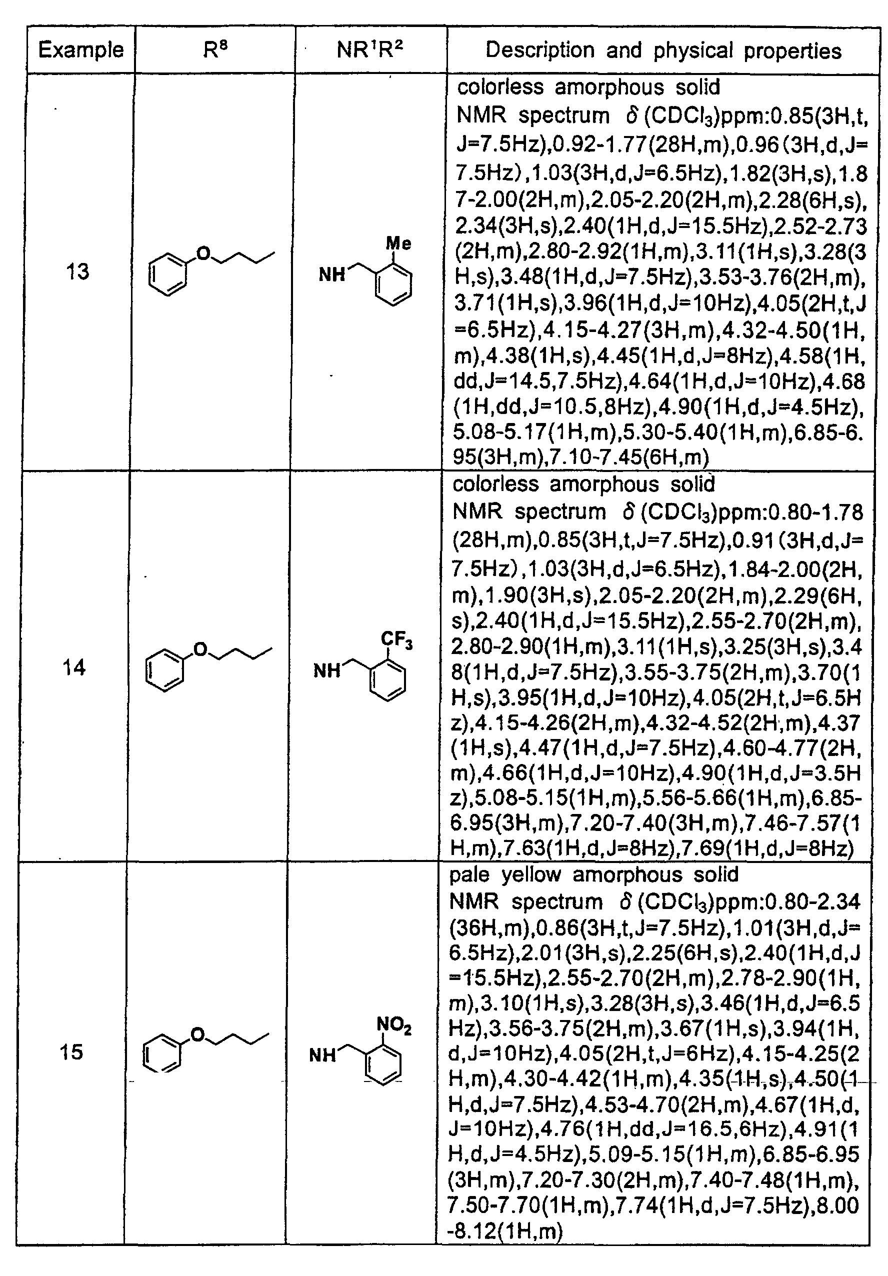

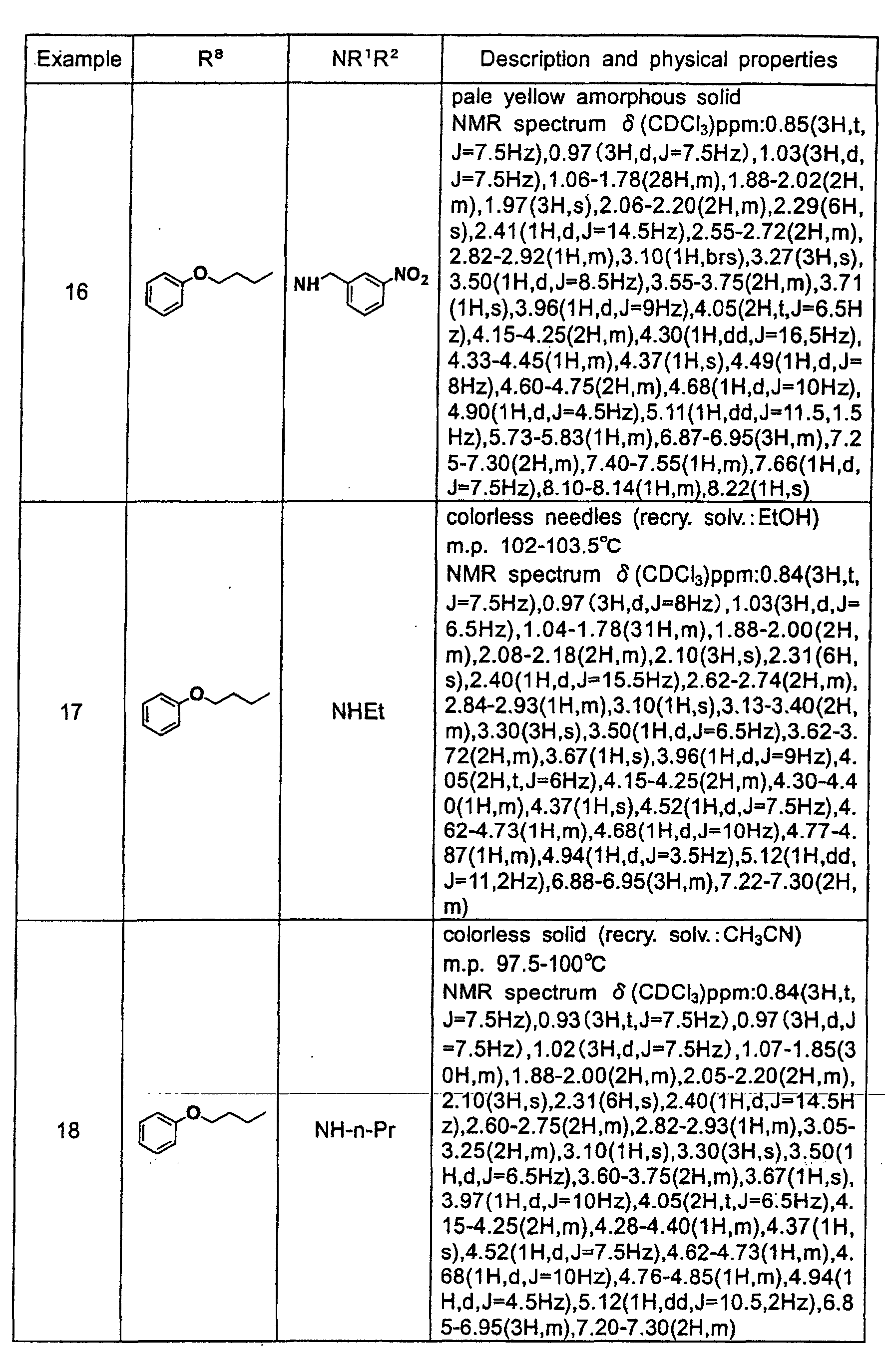

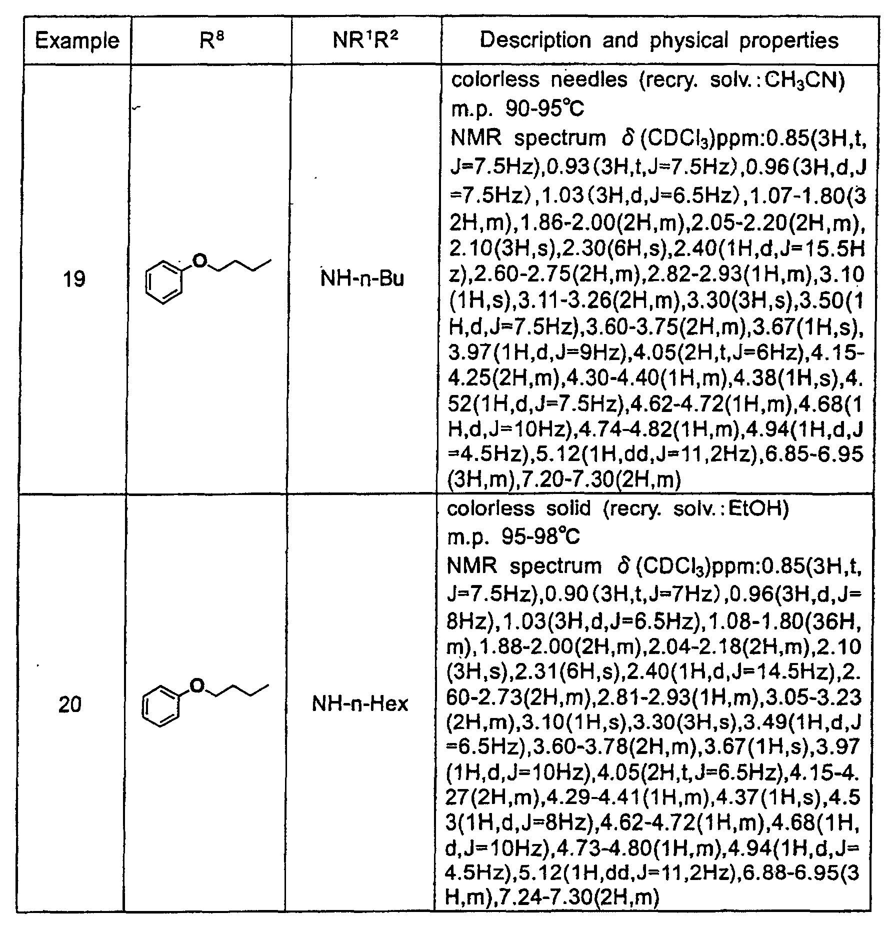

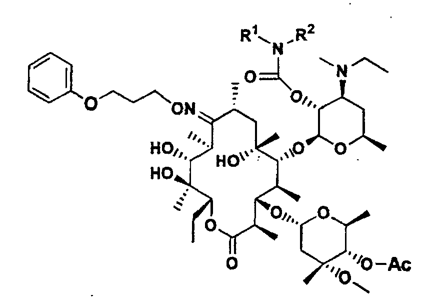

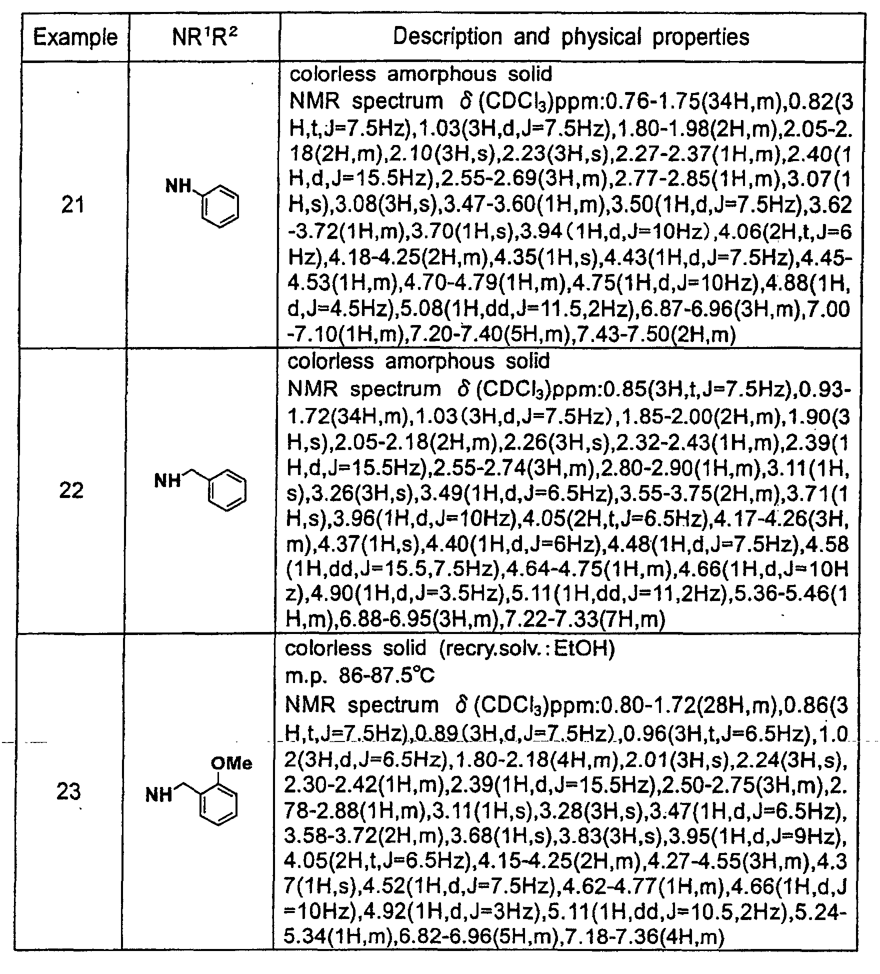

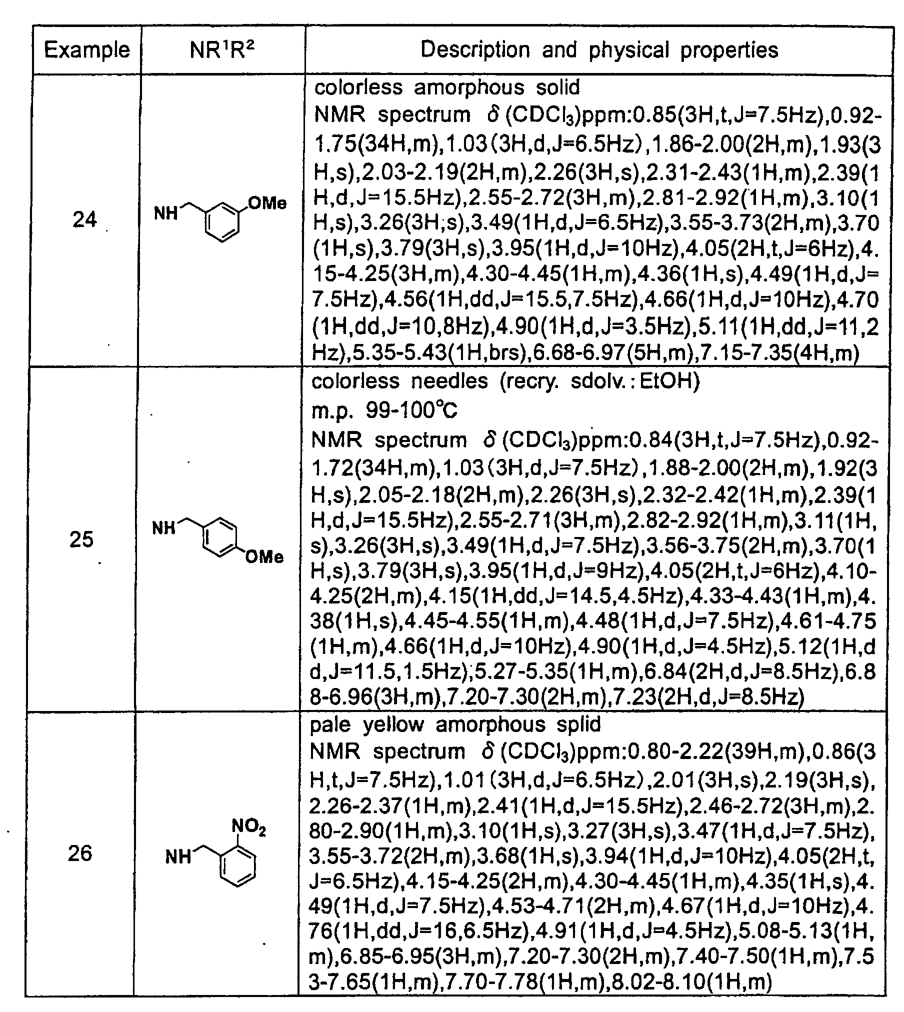

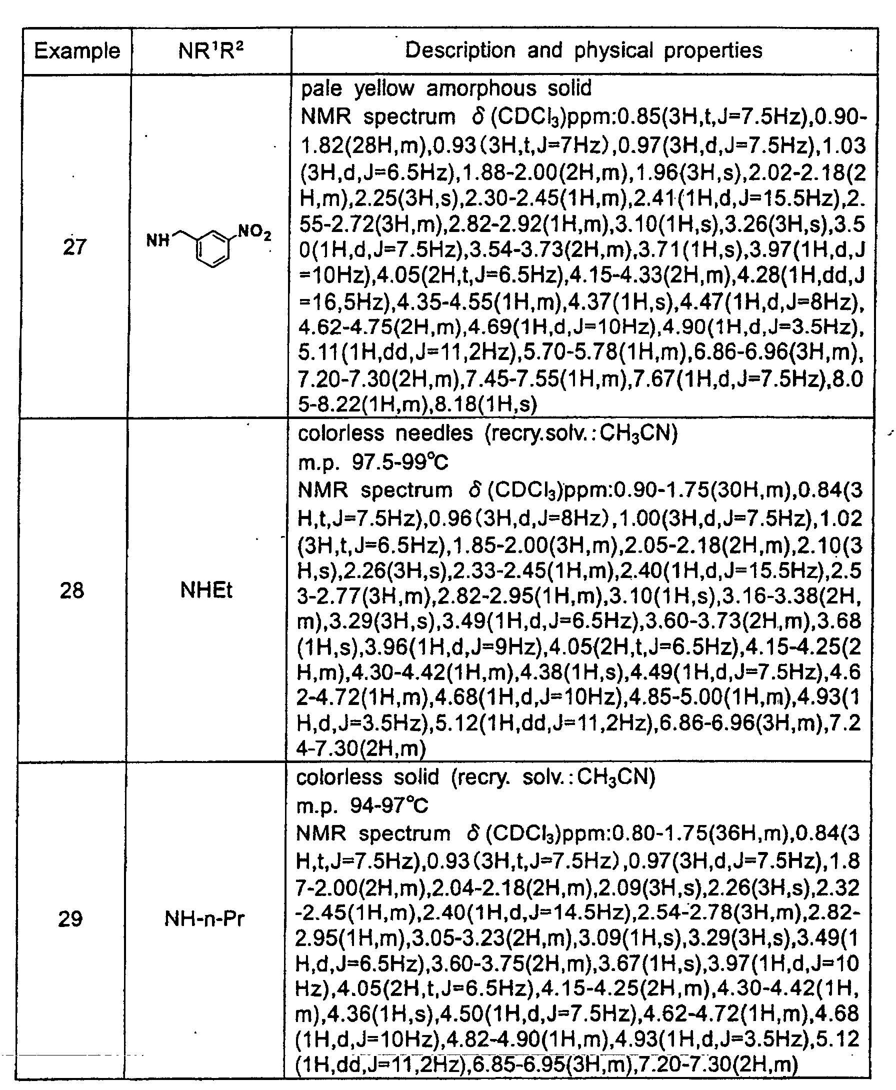

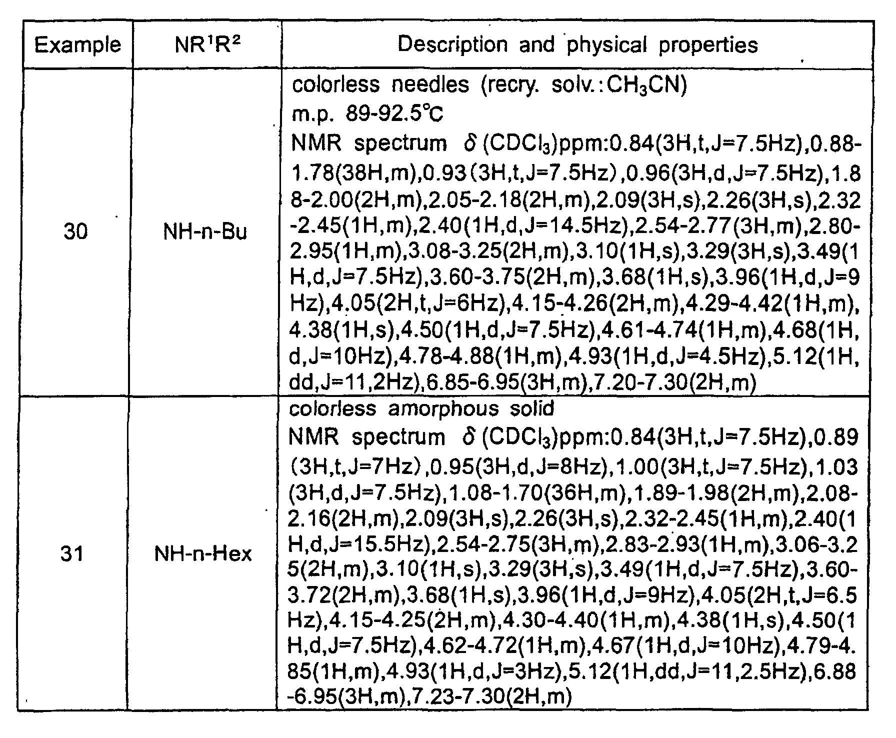

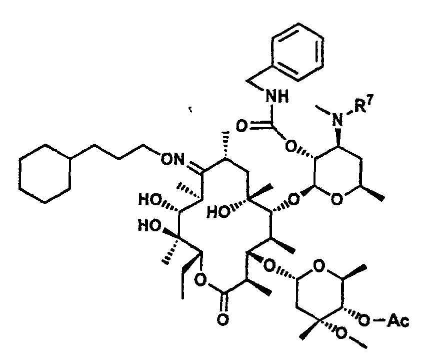

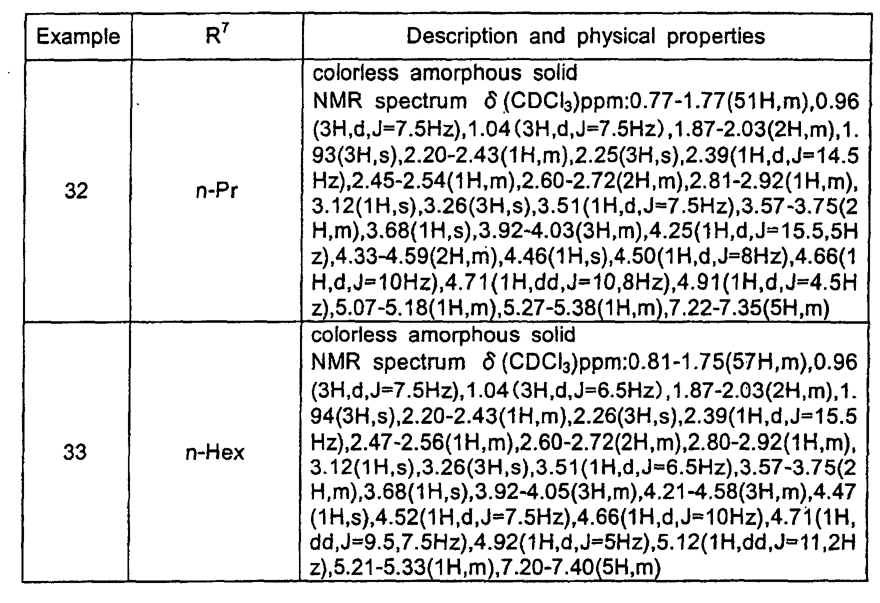

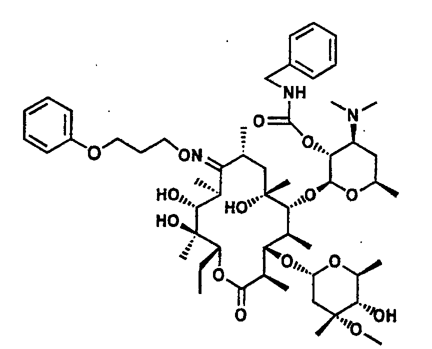

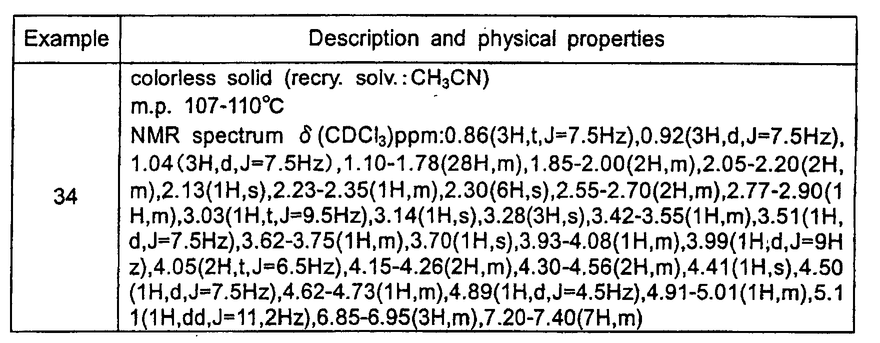

### Example 35: 4"-O-Acetyl-2'-O-(1-imidazolylcarbonyl)erythromycin A 9-[O-(3-cyclohexylpropyl)oxime]

To a solution of 0.50 g of 4"-O-acetylerythromycin A 9-[O-(3-cyclohexylpropyl)oxime] in 5.0 ml of dried toluene, 0.11 g of N; N'-carbonyldiimidazole was added, and the mixture was stirred at 80°C of outer temperature for 21 hours. The reaction mixture was added with water, and alkalified by saturated aqueous solution of sodium hydrogencarbonate, and extracted with ethyl acetate. The extract was washed successively with water and saturated brine, and dried over sodium sulfate, and the solvent was evaporated under reduced pressure. The obtained residue was added with n-heptane and heated, and filtrated under hot condition. The filtrate was cooled and the precipitated crystals were collected by filtration to obtain 0.56 g of a colorless solid. Recrystallization from diisopropyl ether gave a colorless solid having the melting point of from 136.5 to 137.5°C.
NMR spectrum δ (CDCl₃)ppm:0.72-2.00(48H,m),0.77(3H,d,J=7.5Hz),0.81(3H,t,J=7.5 Hz),2.12(3H,s),2.27(6H,s),2.41(1H,d,J=15.5Hz),2.59-2.64(1H,m),2.77-2.86(2H,m),3.08( 1H,s),3.39(3H,s),3.55(1H,d,J=6.5Hz),3.58(1H,s),3.60-3.72(1H,m),3.77-3.87(1H,m),3.94-4.02(3H,m),4.27-4.34(1H,m),4.42(1H,s),4.60-4.80(2H,m),4.70(1H,d,J=10Hz),4.80-4.90( 2H,m),4.97(1H,d,J=5Hz),5.09(1H,dd,J=11,2.5Hz),7.08(1H,s),7.40(1H,s),8.06(1H,s)

In accordance with the method of Example 35, the compounds of Examples 36 through 39 were obtained.

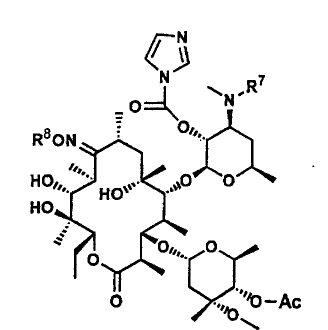

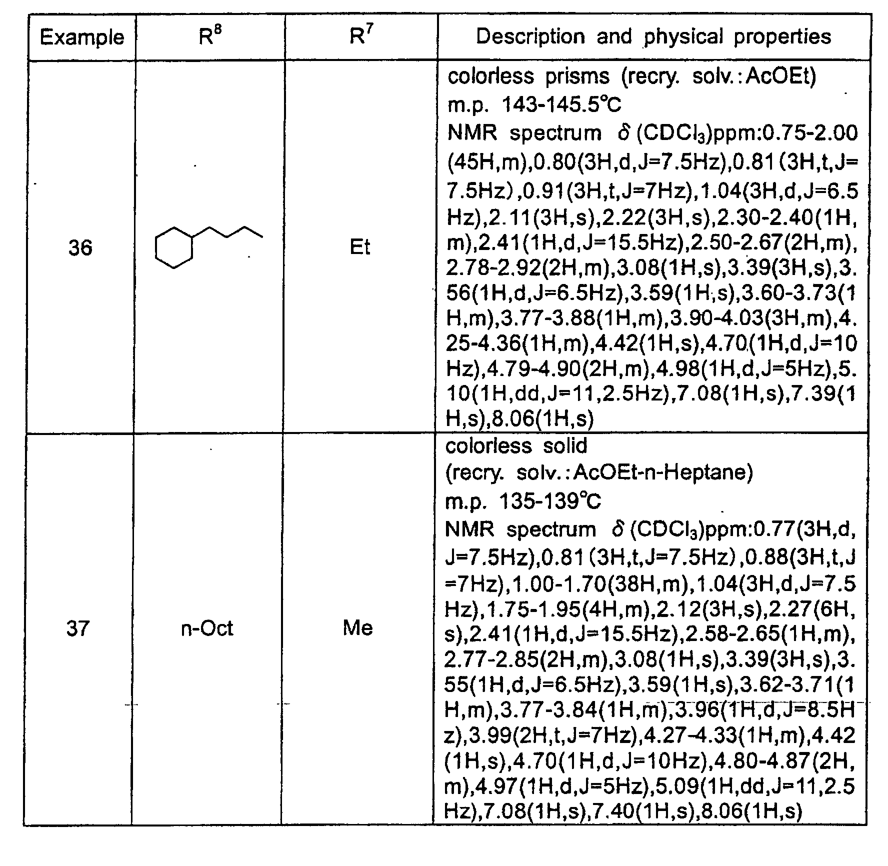

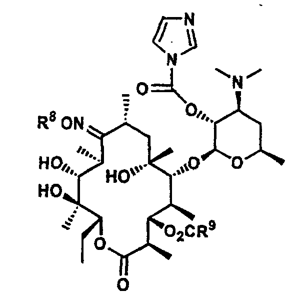

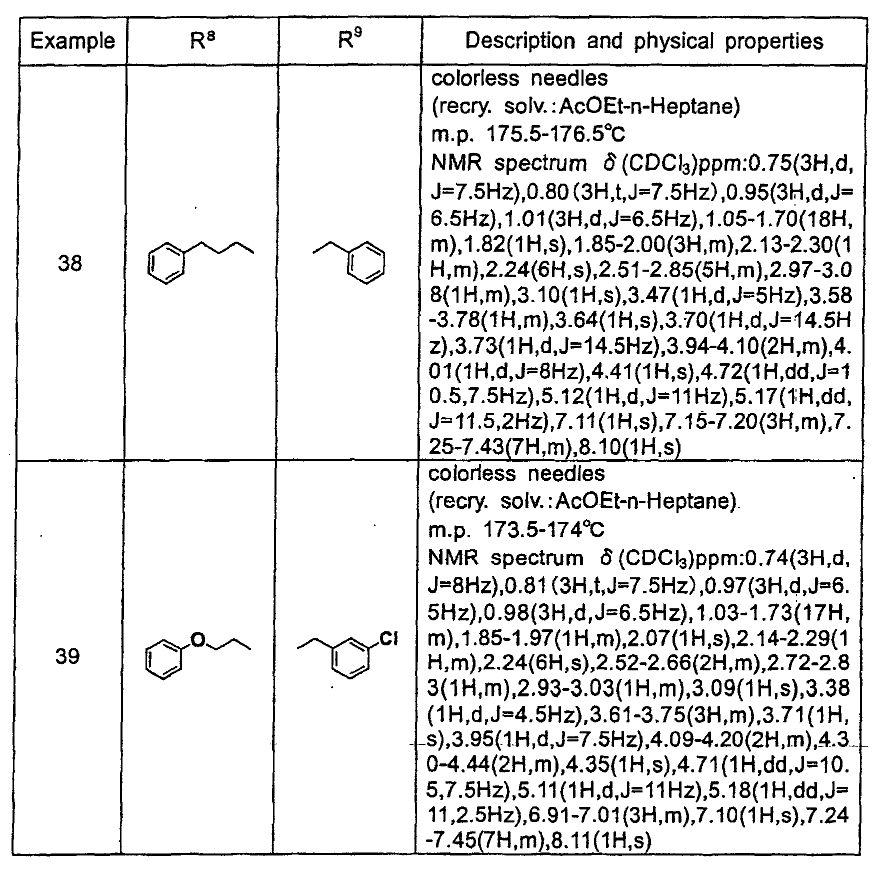

### Example 40: 4"-O-Acetyl-2'-O-benzylaminocarbonylerythromycin A 9-[O-(3-cyclohexylpropyl)oxime]

To a solution of 0.50 g of 4"-O-acetyl-2'-O-(1-imidazolylcarbonyl)erythromycin A 9-[O-(3-cyclohexylpropyl)oxime] in 5.0 ml of tetrahydrofuran, 0.06 ml of benzylamine was added. Under nitrogen atmosphere, the mixture was stirred at room temperature for 2 hours. And then, the reaction mixture was added with 0.50 ml of water, and stirred at room temperature for 17 hours. The reaction mixture was concentrated under reduced pressure, and the obtained residue was added with water and alkalified by saturated aqueous solution of sodium hydrogencarbonate, and extracted with ethyl acetate. The extract was washed successively with water and saturated brine, and dried over sodium sulfate, and the solvent was evaporated under reduced pressure. The residue was purified by column chromatography (silica gel, ethyl acetate) to obtain 0.24 g of a colorless amorphous solid.
NMR spectrum δ (CDCl₃)ppm:0.80-2.05(45H,m),0.85(3H,t,J=7.5Hz),0.96(3H,d,J=6.5 Hz),1.04(3H,d,J=6.5Hz),1.95(3H,s),2.31(6H,s),2.39(1H,d,J=15.5Hz),2.57-2.72(2H,m),2. 80-2.92(1H,m),3.12(1H,s),3.26(3H,s),3.51(1H,d,J=6.5Hz),3.58-3.75(2H,m),3.68(1H,s),3. 90-4.04(3H,m),4.26(1H,dd,J=15,4.5Hz),4.30-4.60(2H,m),4.46(1H,s),4.52(1H,d,J=7.5Hz) ,4.62-4.77(1H,m),4.66(1H,d,J=10Hz),4.92(1H,d,J=4.5Hz),5.07-5.18(1H,m),5.30-5.40(1 H,m),7.20-7.40(5H,m)

In accordance with the method of Example 40, the compounds of Examples 41 through 99 were obtained.

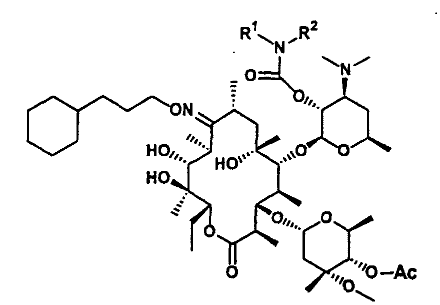

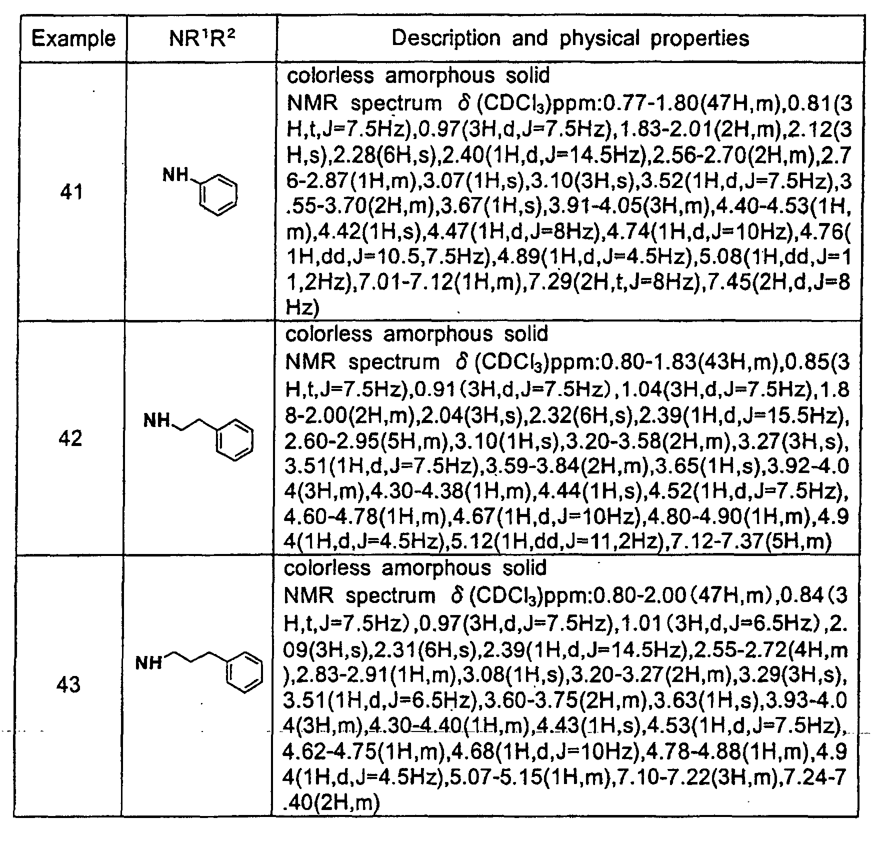

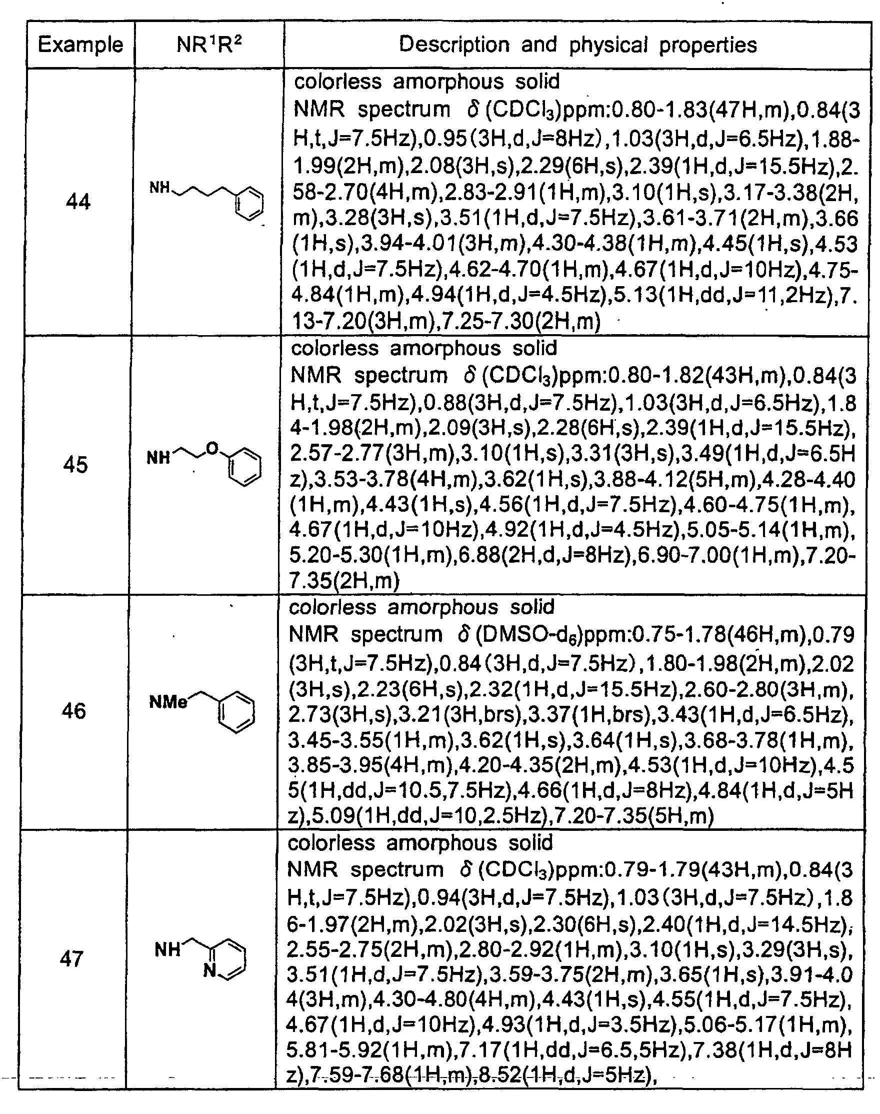

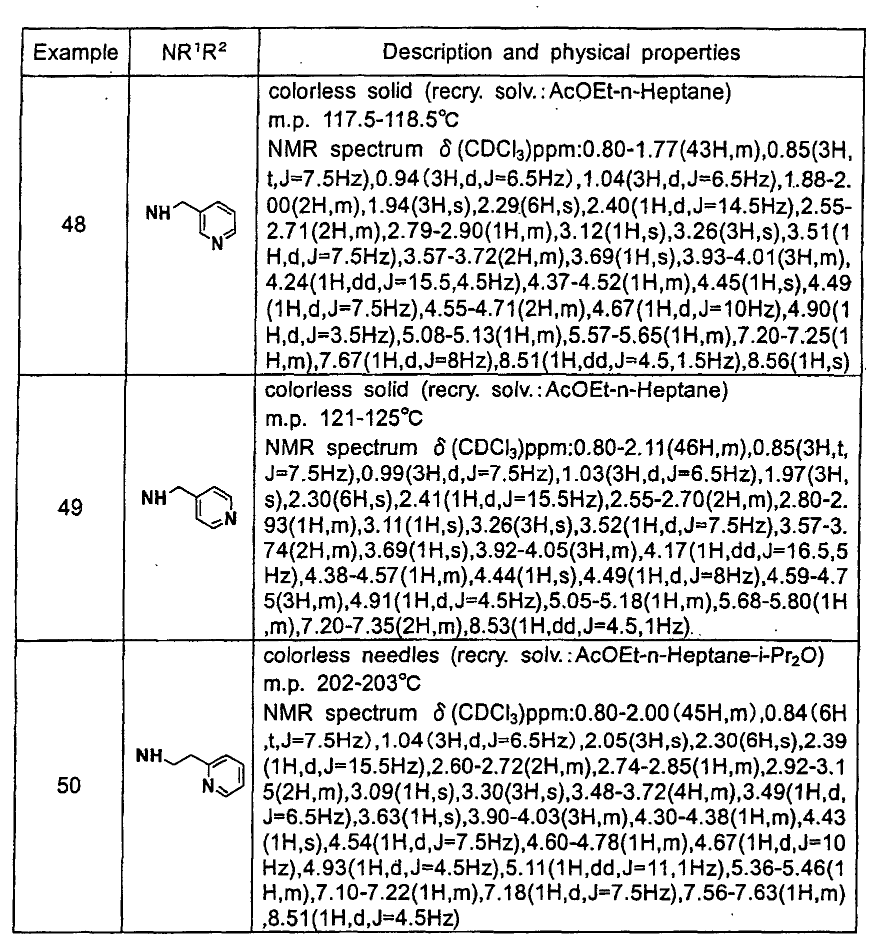

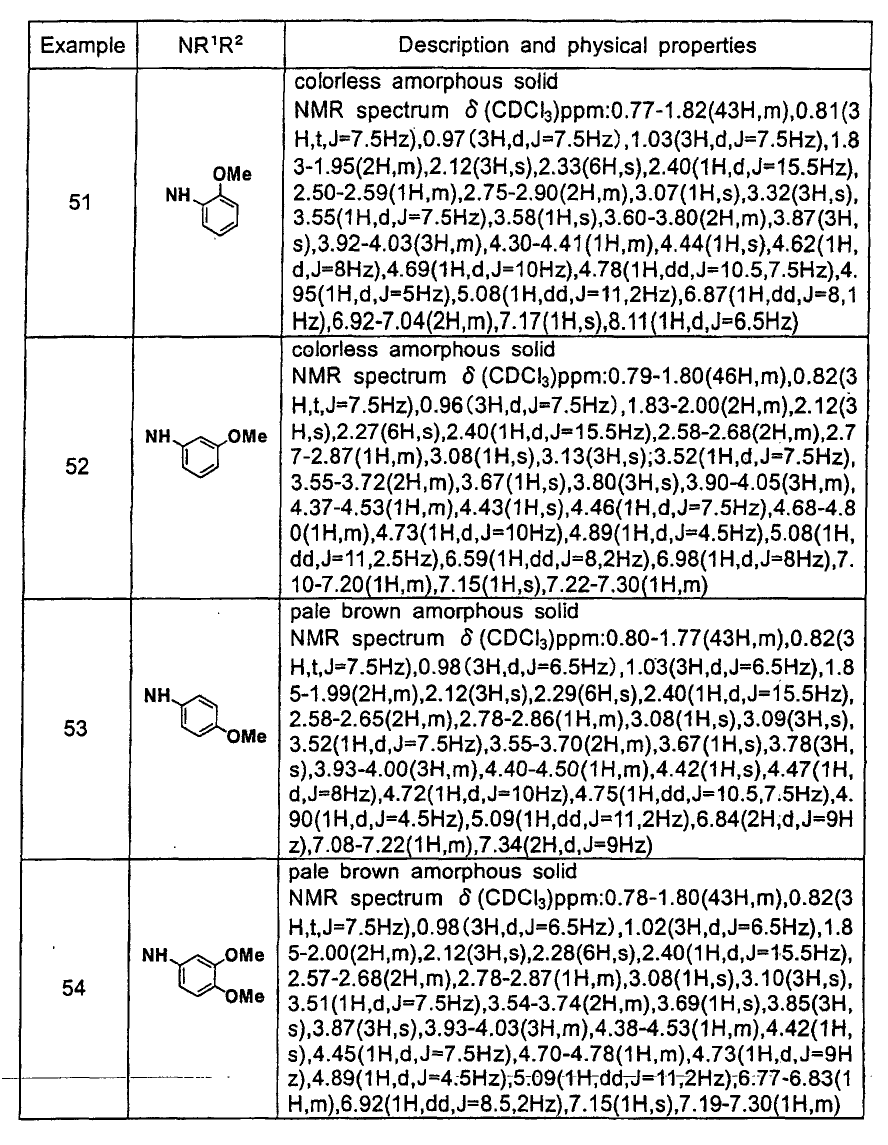

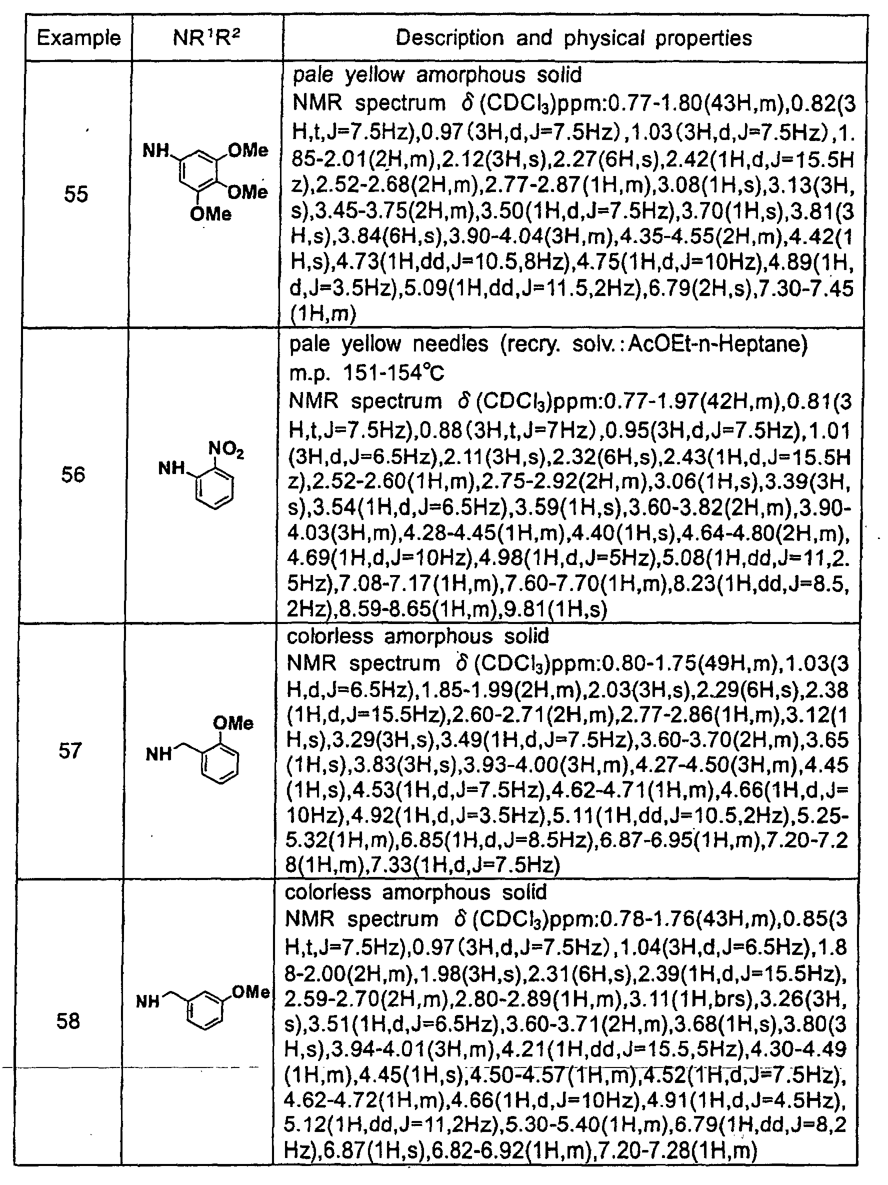

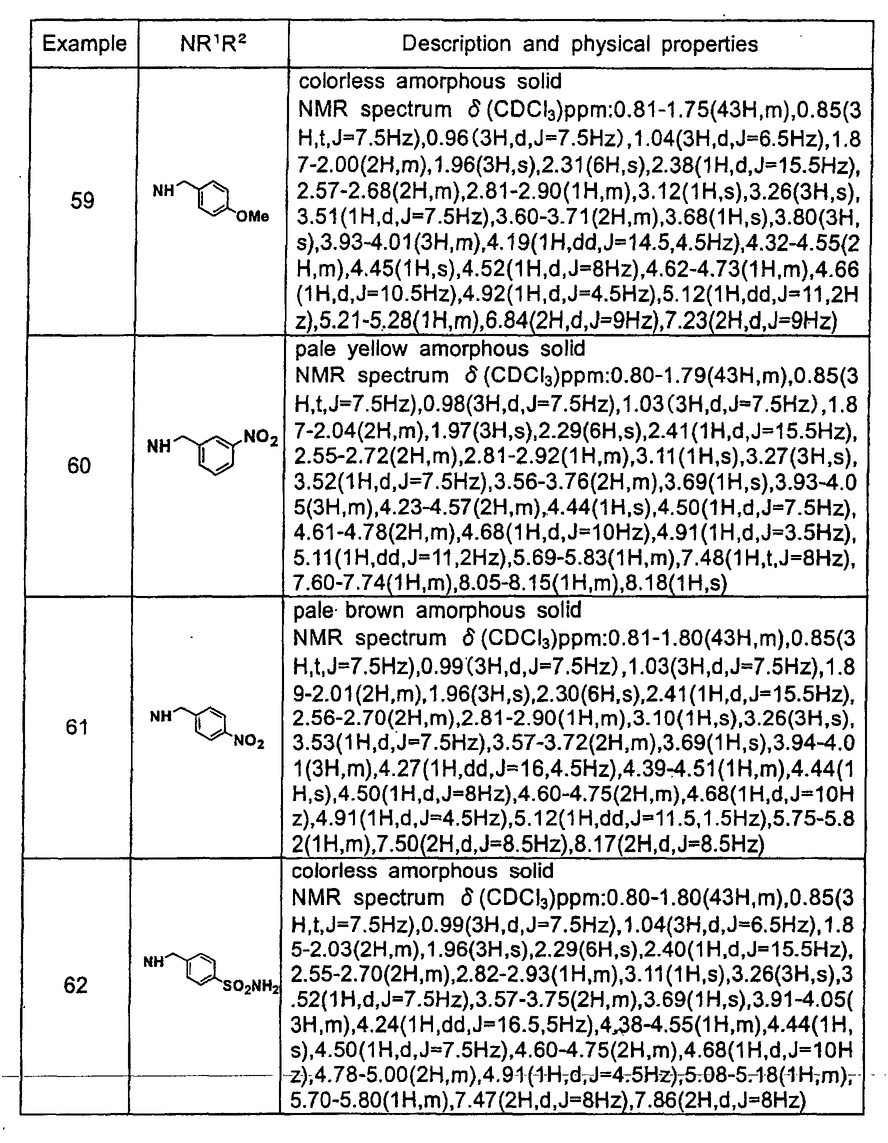

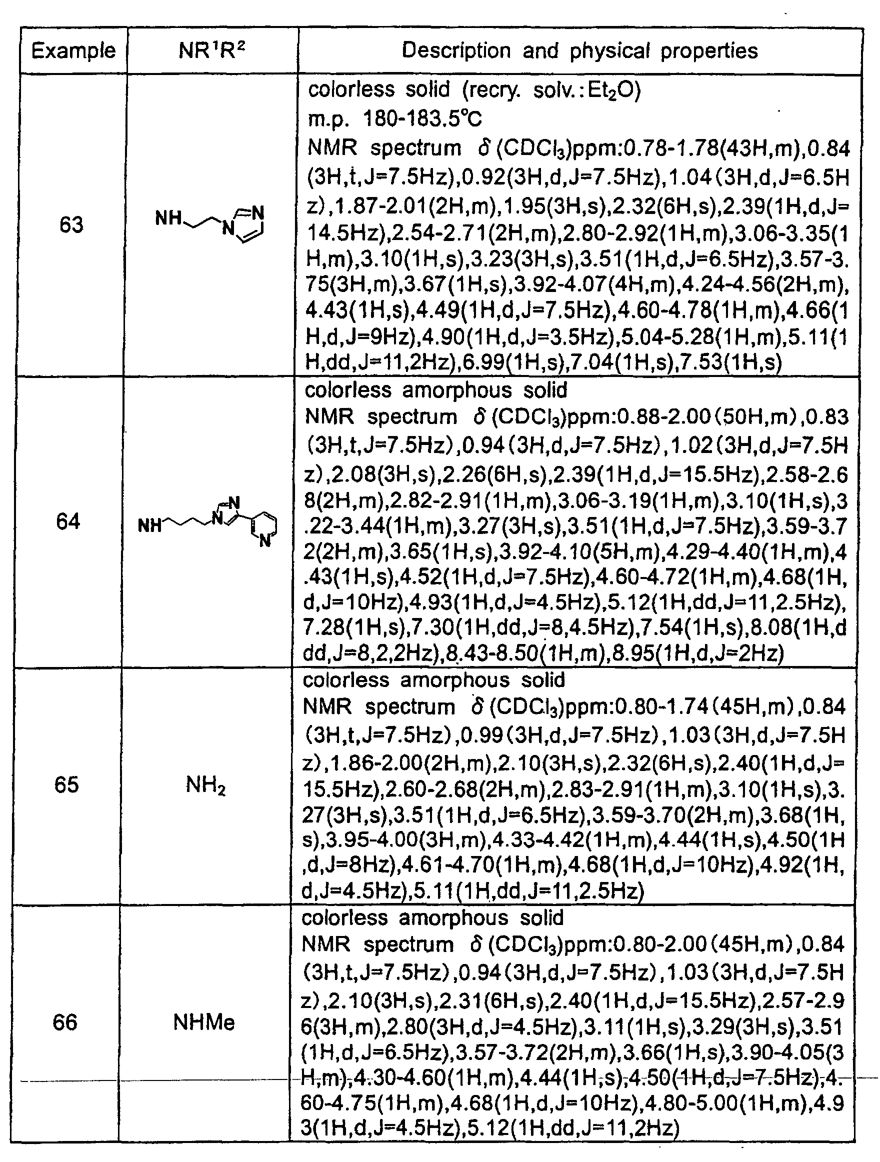

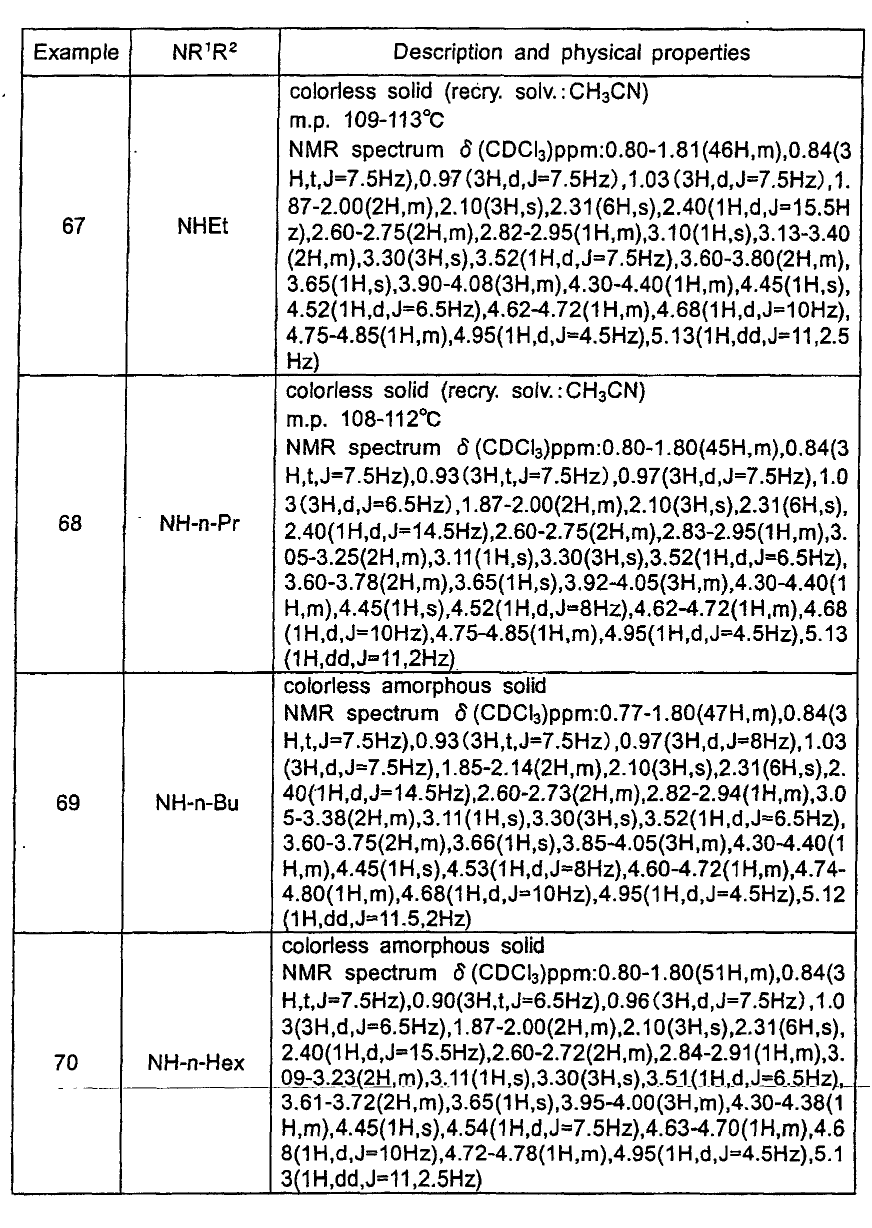

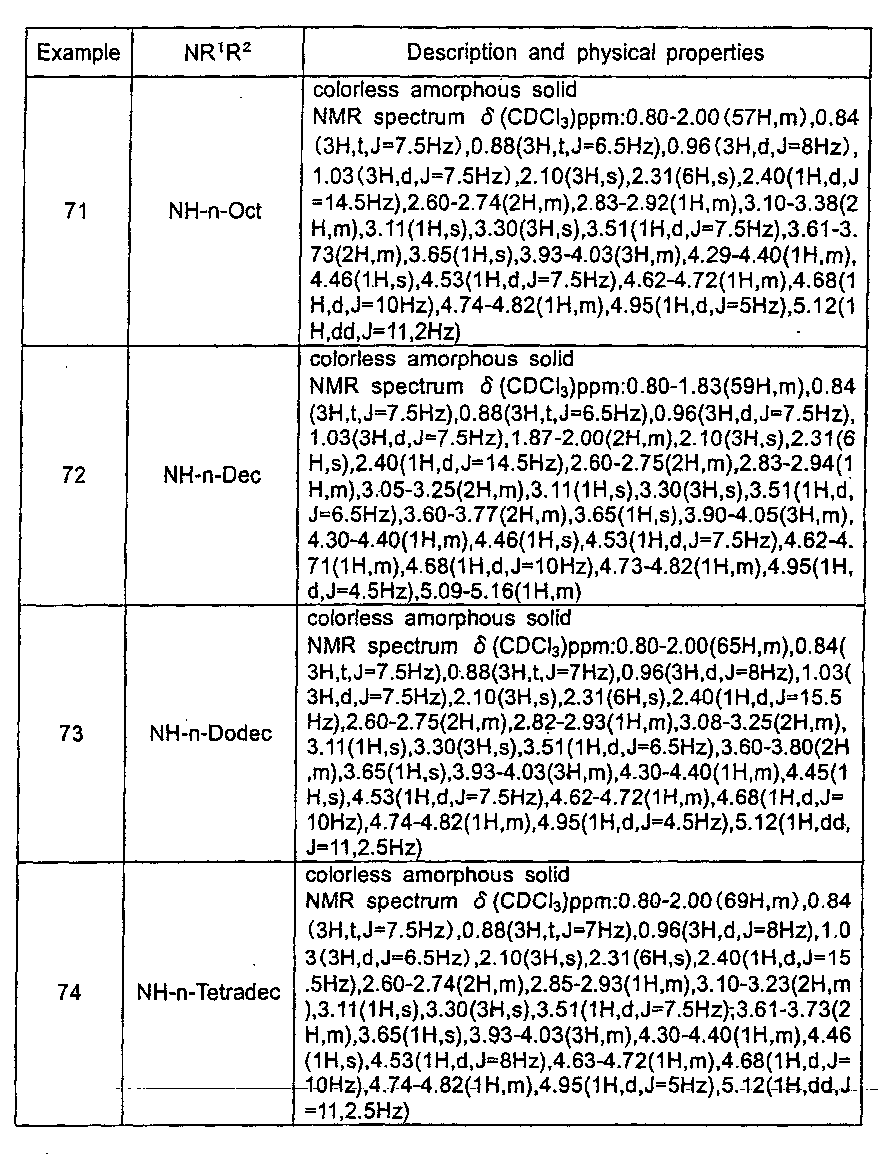

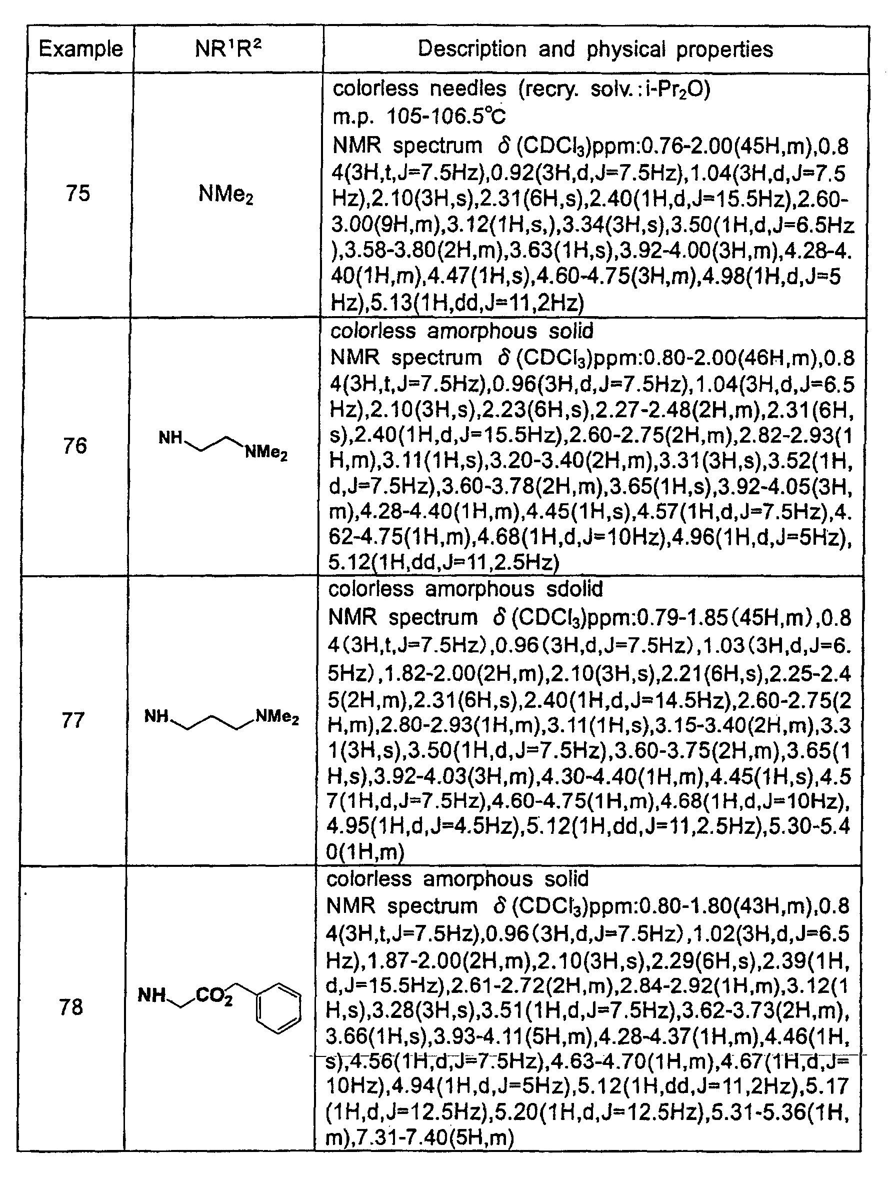

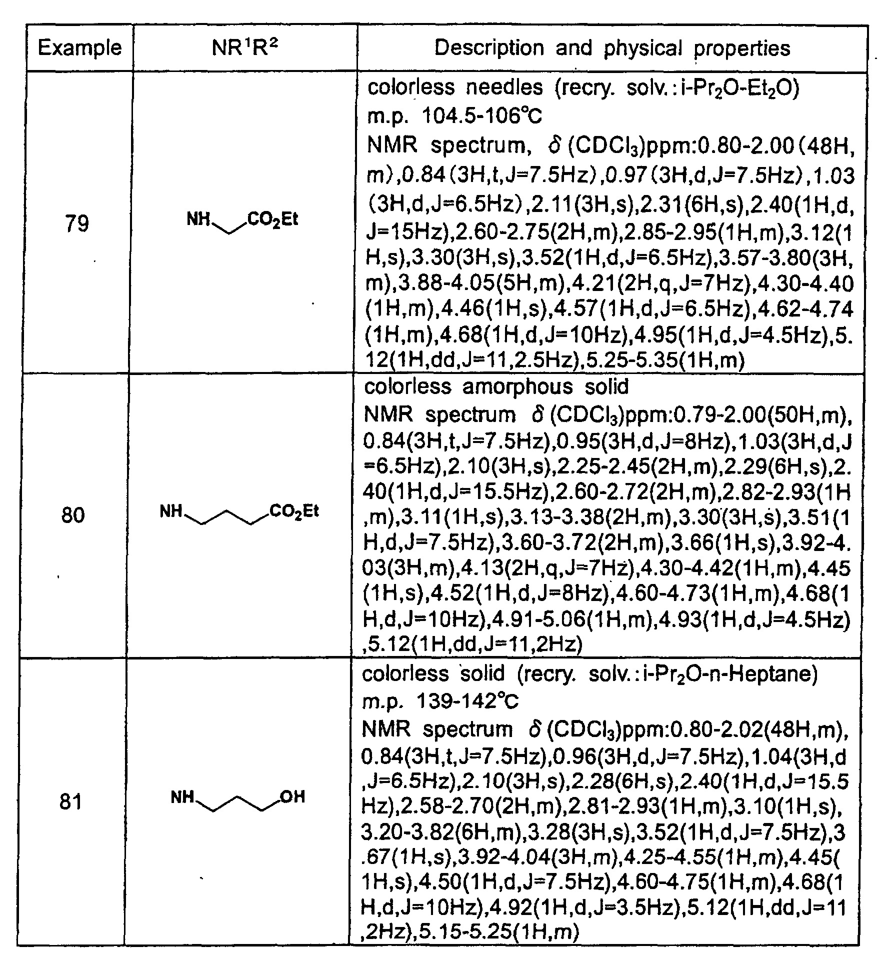

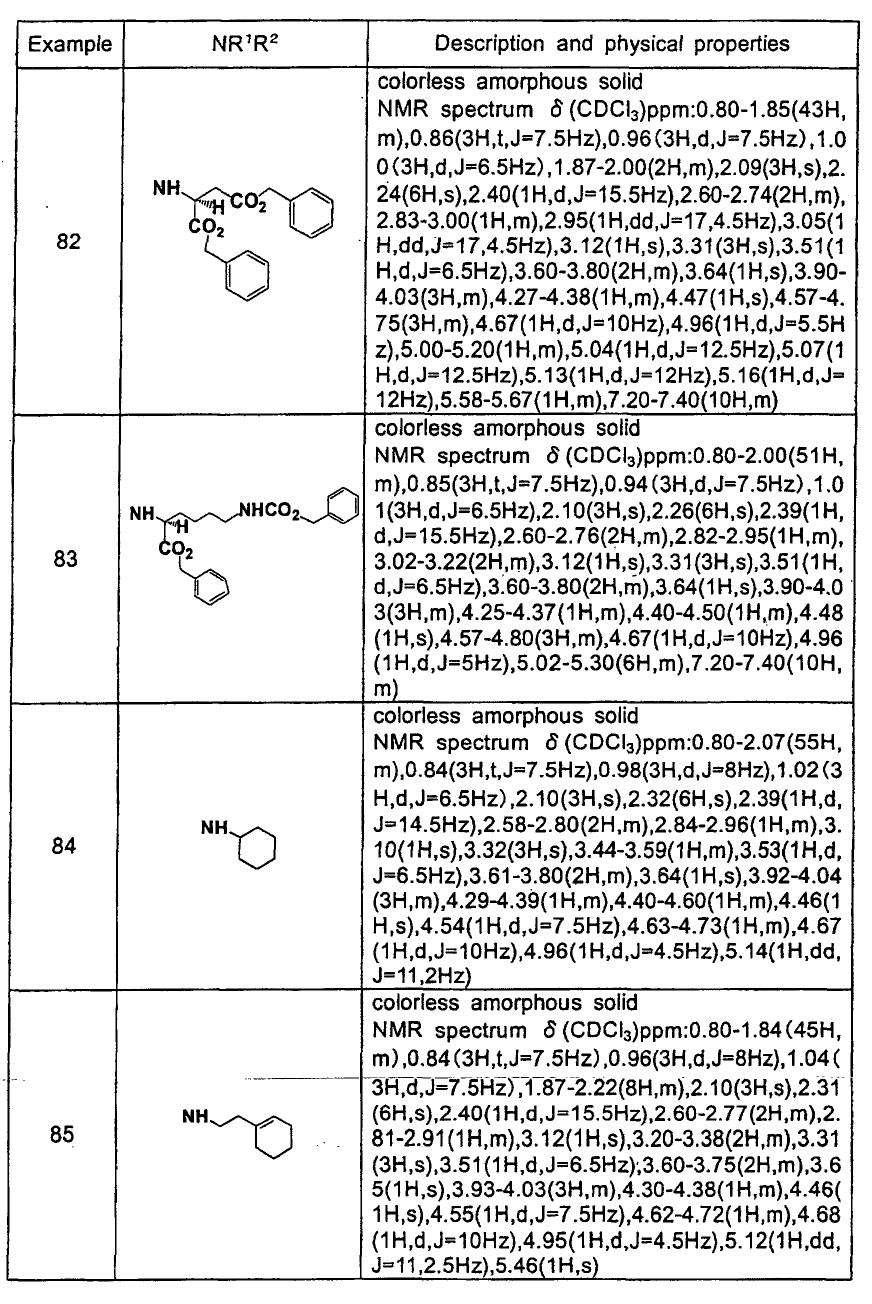

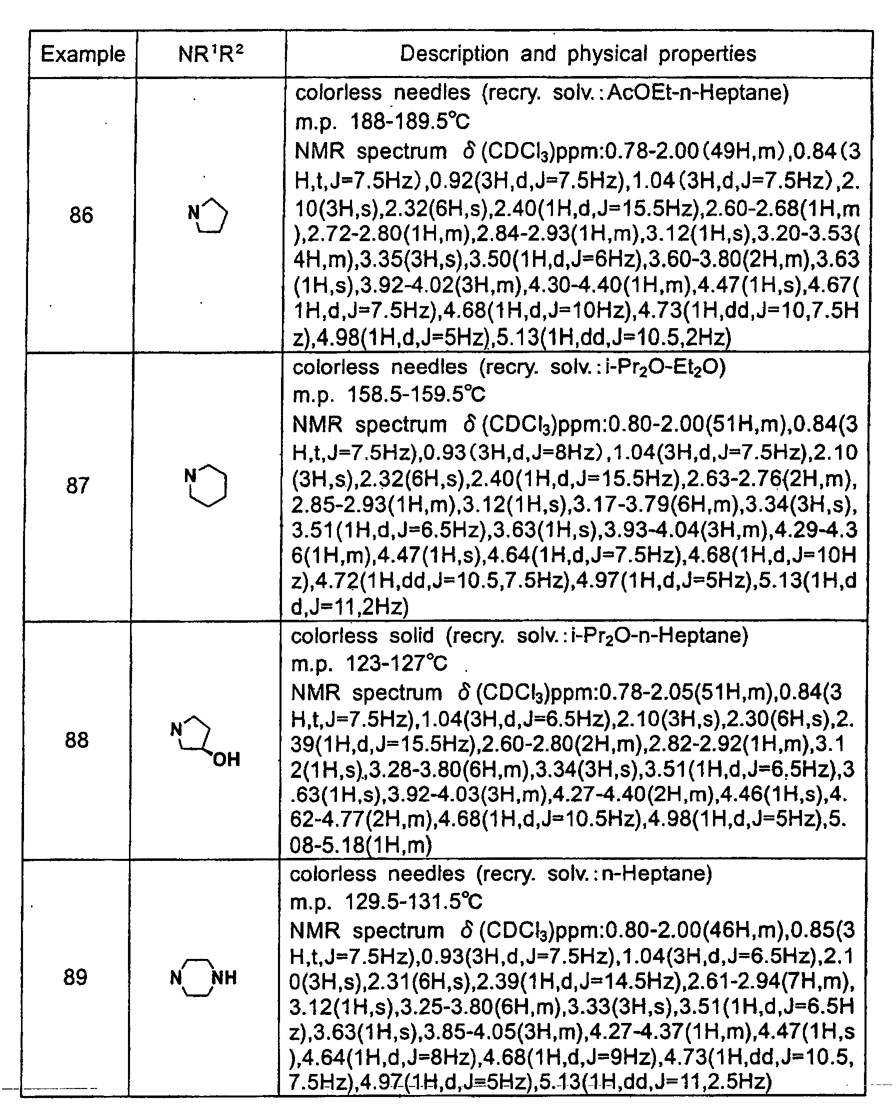

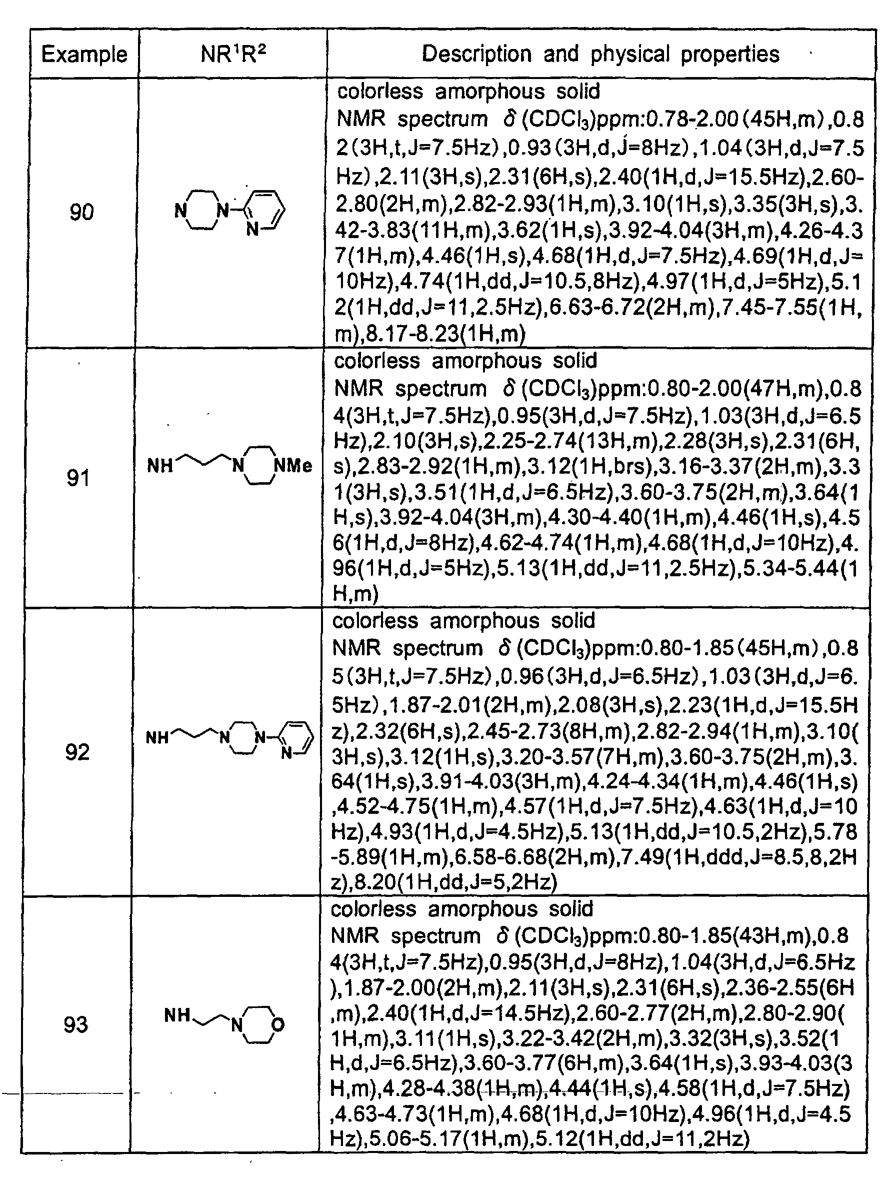

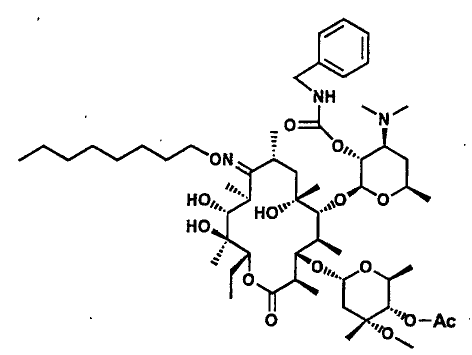

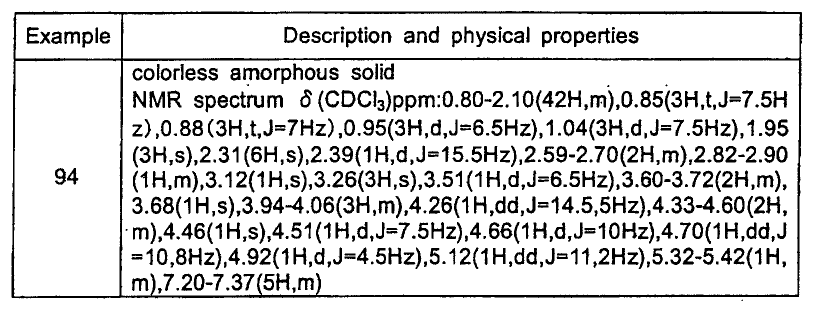

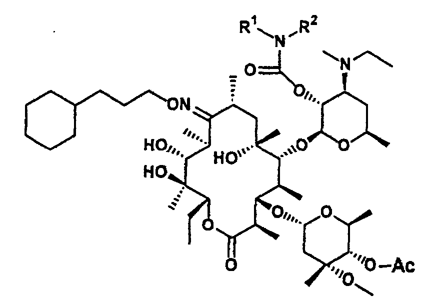

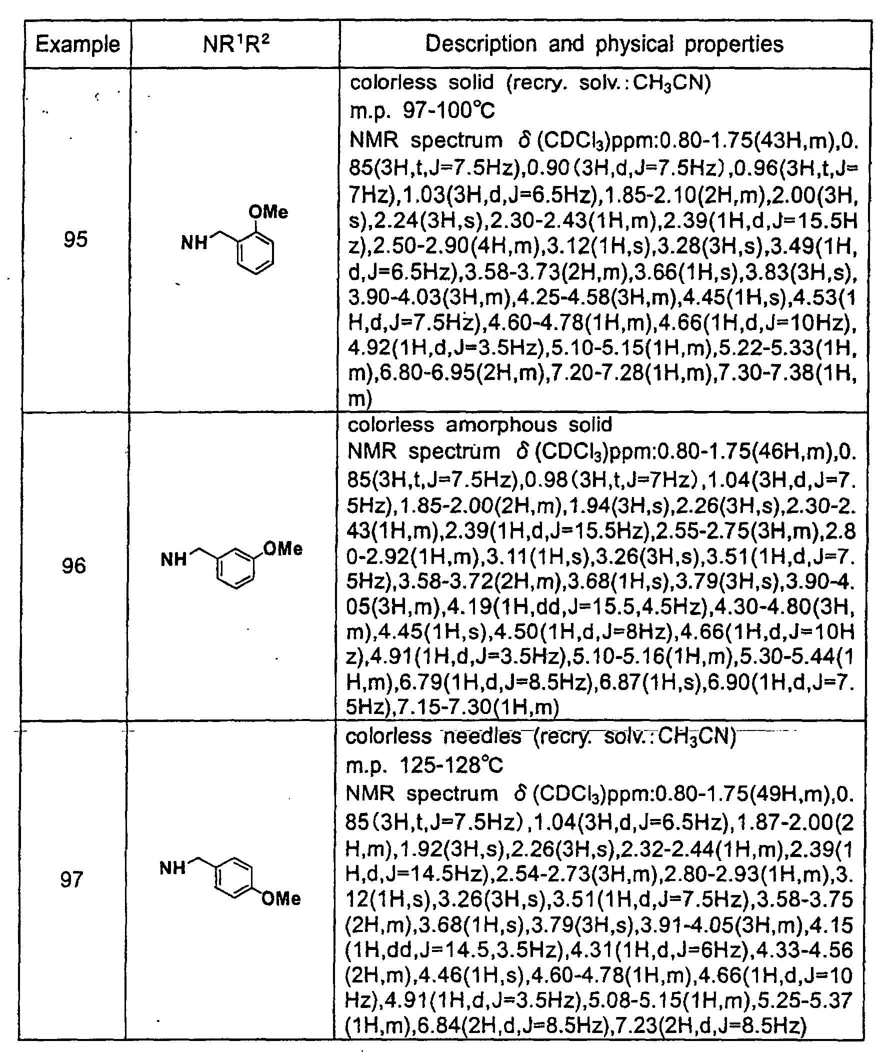

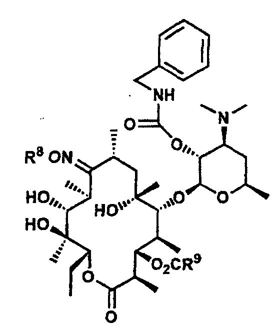

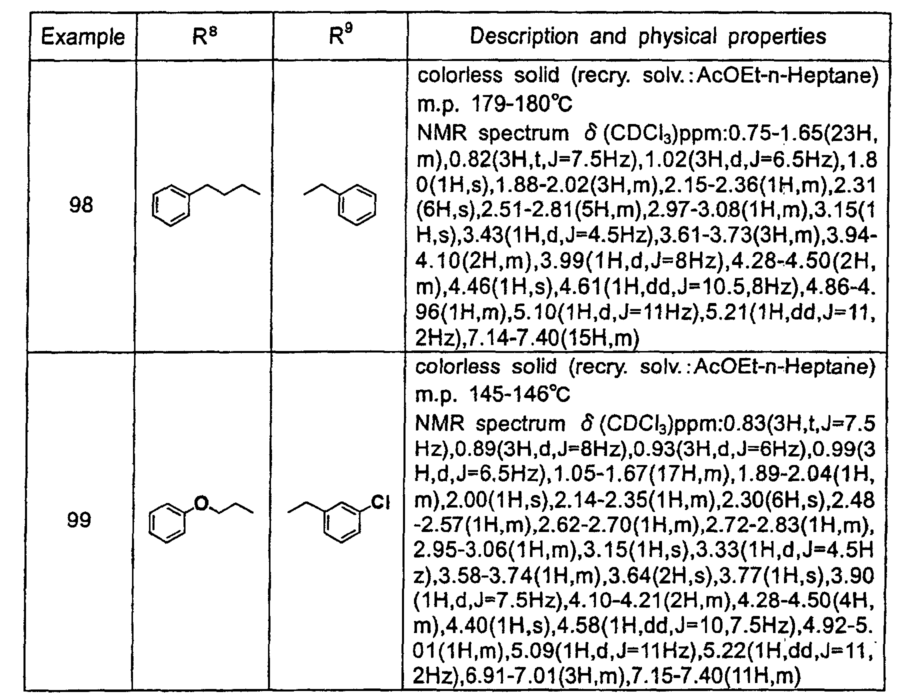

### Example 100: 4"-O-Acetyl-2'-O-benzylaminocarbonyl-3'-N-demethyl-3'-N-ethylerythromycin A 9-[O-(3-cyclohexylpropyl)oxime]

To a solution of 0.50 g of 4"-O-acetyl-3'-N-demethyl-N-ethylerythromycin A 9-[O-(3-cyclohexylpropyl)oxime] in 5.0 ml of toluene, 0.12 g of N,N'-carbonyldiimidazole was added, and the mixture was stirred at 80°C of outer temperature for 19 hours. And then, the reaction mixture was added with 0.06 g of N, N'-carbonyldiimidazole, and stirred at 80°C of outer temperature for 2.5 hours. And then, the reaction mixture was added with 0.12 ml of benzylamine, and stirred at 80°C of outer temperature for 4 hours. The reaction mixture was added with water, and extracted with ethyl acetate. The extract was washed successively with water and saturated brine, and dried over sodium sulfate, and the solvent was evaporated under reduced pressure. The residue was purified by column chromatography (silica gel, ethyl acetate : ammonia water = 50 : 0.1) to obtain 0.13 g of a pale yellowish amorphous solid.
NMR spectrum δ (CDCl₃)ppm:0.80-1.76(52H,m),0.85(3H,t,J=7.5Hz),1.86-2.01(2H,m), 1.91(3H,s),2.27(3H,s),2.32-2.45(1H,m),2.39(1H,d,J=14.5Hz),2.55-2.76(3H,m),2.81-2.94 (1H,m),3.12(1H,s),3.26(3H,s),3.51(1H,d,J=6.5Hz),3.56-3.75(2H,m),3.69(1H,s),3.89-4.04 (3H,m),4.22(1H,dd,J=15.5,4.5Hz),4.33-4.80(2H,m),4.45(1H,s),4.50(1H,d,J=7.5Hz),4.58( 1H,dd,J=15,7Hz),4.66(1H,d,J=10Hz),4.91(1H,d,J=3.5Hz),5.07-5.17(1H,m),5.33-5.47(1 H,m),7.22-7.36(5H,m)

In accordance with the method of Example 100, the compound of Example 101 was obtained.

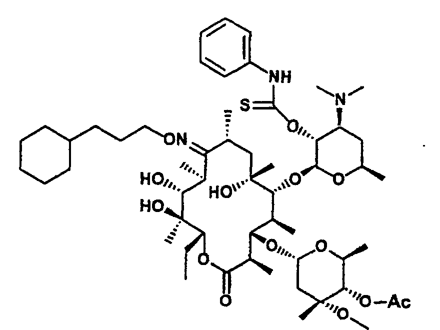

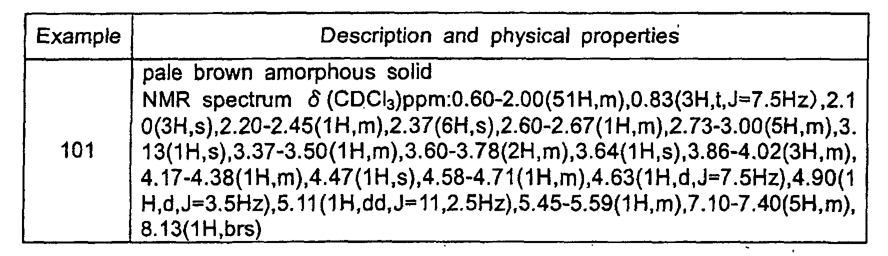

### Example 102: 4"-O-Acetyl-2'-O-(1-(S)-5-amino-1-carboxylpentylaminocarbonyl)-erythromycin A 9-[O-(3-cyclohexylpropyl)oxime]

To a solution of 0.70 g of 4"-O-acetyl-2'-O-(1-(S)-1-benzyloxycarbonyl-5-benzyloxycarbonylaminopentylaminocarbonyl)erythromycin A 9-[O-(3-cyclohexylpropyl)oxime] in 70 ml of methanol, 70 mg of 5% palladium carbon was added, and the mixture was hydrogenated at room temperature for 1.5 hours under hydrogen atmospheric condition. And then, the mixture was added with 70 mg of 5% palladium carbon and 50 ml of methanol, and hydrogenated at 50°C for 2 hours under hydrogen atmospheric condition. The catalyst was filtered off, and the solvent was evaporated to obtain a pale brown solid. Recrystallization from a mixed solvent of ethyl acetate and diisopropyl ether gave 0.19 g of a pale brown solid having the melting point of from 158 to 161°C.
NMR spectrum δ (CDCl₃)ppm:0.77-2.15(54H,m),0.84(3H,t,J=7Hz),0.95(3H,d,J=7.5 Hz),1.03(3H,d,J=6.5Hz),2.10(3H.s),2.30(6H,s),2.39(1H,d,J=14.5Hz),2.55-3.05(5H,m),3. 13(1H,brs),3.33(3H,s),3.53(1H,d,J=5.5Hz),3.58-3.80(2H,m),3.63(1H,s),3.85-4.13(4H,m) ,4.20-4.75(3H,m),4.43(1H,brs),4.66(1H,d,J=10Hz),4.92-5.00(1H,m),5.05-5.18(1H,m),5.6 2-5.85(1H,m)

### Example 103: 4"-O-Acetyl-2'-O-benzylaminocarbonylerythromycin A 9-[O-(3-phenoxypropyl)oxime] ·L-(+)-tartrate

The compound obtained in Example 6 was converted into the L-(+)-tartrate of the compound in a conventional manner. Recrystallization from acetonitrile gave colorless needles having the melting point of from 140.5 to 142°C.
NMR spectrum δ (CDCl₃)ppm:0.86(3H,t,J=7.5Hz),0.86(3H,d,J=8Hz),1.01(3H,d,J=6.5 Hz),1.06-1.78(33H,m),1.87-2.18(5H,m),2.08(3H,s),2.38(1H,d,J=15.5Hz),2.60-2.70(1H,m ),2.73-2.89(1H,m),2.79(6H,s),3.12(1H,brs),3.29(3H,s),3.48(1H,d,J=6Hz),3.60-3.77(2H, m),3.64(1H,s),3.83-3.95(1H,m),3.93(1H,d,J=9Hz),4.04(2H,t,J=6.5Hz),4.16-4.26(4H,m), 4.31(2H,s),4.36(1H,brs),4.41(1H,dd,J=15,6.5Hz),4.67(1H,d,J=10Hz),4.72(1H,d,J=7.5H z),4.83(1H,dd,J=11,7.5Hz),4.95(1H,d,J=5Hz),5.12(1H,dd,J=11,2Hz),6.18-6.25(1H,m),6. 82-6.95(2H,m),6.90(1H,d,J=8Hz),7.18-7.40(7H,m)

In order to evaluate excellent efficacy of the compounds of the present invention, their antibacterial spectrums against atypical acid-fast mycobacteria were measured. Clarithromycin, rifampicin and 4"-O-acetylerythromycin A 9-(O-methyloxime) [a compound disclosed in the Japanese Patent Unexamined Publication (KOKAI) No.63-107921/1988] were used as reference compounds. Ac in the formula represents acetyl group.

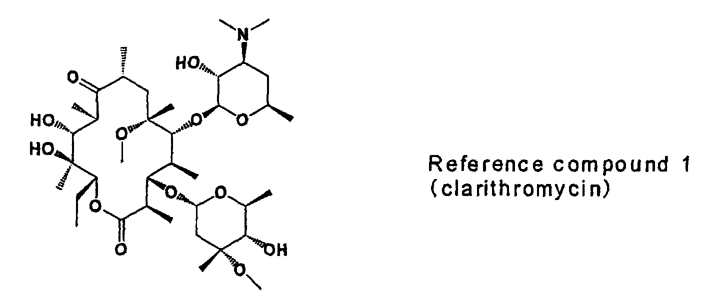

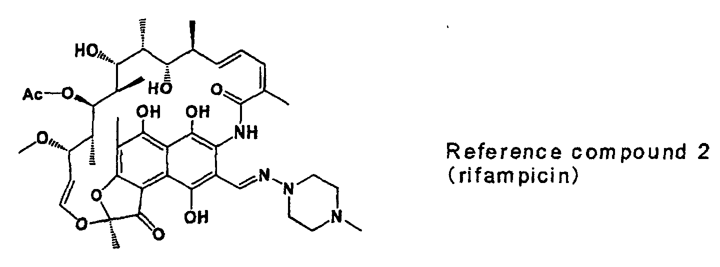

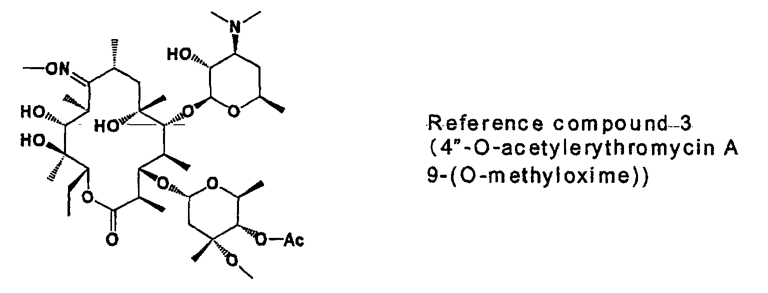

### Antibacterial activity against atypical acid-fast mycobacteria

Antibacterial activities (minimum inhibitory concentrations) against clinical isolates of atypical acid-fast mycobacteria were measured by the agar dilution method according to the standard method of the Japan Society of Chemotherapy. About 5 µl of bacterial suspension (adjusted to 10⁶ CFU/ml) were spotted on the 7H11 agar plates containing the test compounds. The minimum inhibitory concentrations were determined by the growth or no growth of the bacteria after incubation at 37°C for 7 days. The results are shown in the following table. The abbreviation M in the table represents Mycobacterium. The compounds of the present invention had more excellent antibacterial activity than the reference compounds against atypical acid-fast mycobacteria including clarithromycin-resistant strains (*M.avium* 20092 and other bacteria).

| Antibacterial spectrum (Minimum inhibitory concentration µg/ml) | | | | | | |
|---|---|---|---|---|---|---|
| Strain | Example 6 | Example 7 | Example 9 | Example 15 | Example 18 | Example 26 |
| M.avium 20034 | 0.10 | 0.10 | 0.20 | 0.10 | 0.20 | 0.10 |
| M.avium 20045 | 0.20 | 0.20 | 0.39 | 0.20 | 0.20 | 0.20 |
| M.avium 20092 | 0.10 | 0.10 | 0.20 | 0.10 | 0.20 | 0.20 |
| M.avium 20096 | 0.10 | 0.20 | 0.20 | 0.20 | 0.20 | 0.20 |
| M.intracellulare 20066 | 0.20 | 0.10 | 0.20 | 0.20 | 0.20 | 0.20 |
| M.intracellulare 20067 | 0.20 | 0.20 | 0.39 | 0.20 | 0.20 | 0.20 |
| M.intracellulare 20073 | 0.20 | 0.20 | 0.39 | 0.10 | 0.39 | 0.10 |
| M.intracellulare 20075 | 0.20 | 0.20 | 0.39 | 0.20 | 0.20 | 0.20 |

| Antibacterial spectrum (Minimum inhibitory concentration µg/ml) | | | | | | |
|---|---|---|---|---|---|---|
| Strain | Example 40 | Example 49 | Example 94 | Reference compound 1 | Reference compound 2 | Reference compound 3 |
| M.avium 20034 | 0.39 | 0.78 | 0.39 | 3.13 | 12.5 | >50 |
| M.avium 20045 | 0.78 | 0.78 | 0.78 | 1.56 | 3.13 | 12.5 |
| M.avium 20092 | 0.78 | 0.78 | 0.39 | >50 | 50 | >50 |
| M.avium 20096 | 0.78 | 0.78 | 0.39 | >50 | 3.13 | >50 |
| M.intracellulare 20066 | ≦0.20 | 1.56 | 0.39 | 3.13 | 3.13 | 12.5 |
| M.intracellulare 20067 | 0.78 | 1.56 | 0.39 | 1.56 | 1.56 | 6.25 |
| M.intracellulare 20073 | 0.78 | 1.56 | 0.39 | 1.56 | 3.13 | 12.5 |
| M.intracellulare 20075 | 0.78 | 1.56 | 0.39 | 3.13 | 3.13 | 12.5 |

### Industrial Applicability

The novel erythromycin derivatives and salts thereof have excellent antibacterial activity against atypical acid-fast mycobacteria including m

## Claims

1. A novel erythromycin derivative represented by the following general formula or a salt thereof: 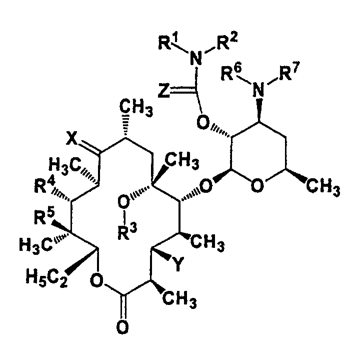 wherein, R¹ and R² independently represent hydrogen atom, an alkyl group which may be substituted, an alkenyl group which may be substituted, an alkynyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, or R¹ and R² may combine together with the nitrogen atom to which they bind to form a saturated or unsaturated heterocyclic group which may further contain one or more heteroatoms selected from the group consisting of oxygen atom, sulfur atom, and nitrogen atom and which may be substituted, R³ representshydrogen atom or methyl group, R⁵ represents hydrogen atom or hydroxyl group when R⁴ represents hydroxyl group, or R⁴ and R⁵ may combine together with two carbon atoms on the ring to which each of them binds to form a heterocyclic group represented by the following formula: 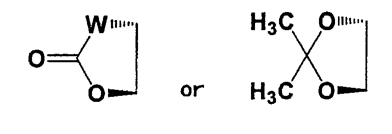 R⁶ and R⁷ independently represent hydrogen atom, an alkyl group which may be substituted, an alkenyl group which may be substituted, an alkynyl group which may be substituted, a cycloalkyl group which may be substituted, an alkyl group substituted with a saturated or unsaturated homocyclic group which may be substituted, or an alkyl group substituted with a saturated or unsaturated heterocyclic group which may be substituted, or R⁶ and R⁷ may bind together with the nitrogen atom to which they bind to form a saturated heterocyclic group which may further contain one or more heteroatoms selected from the group consisting of oxygen atom, sulfur atom, and nitrogen atom and which may be substituted, X represents NH or a group represented by N-O-R⁸, Y represents a group represented by O-C(=O)-R⁹ or O-C(=O)-U-R¹⁰, or represents an oxy group substituted with a heterocyclic group represented by the following formula: 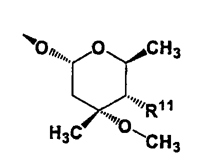 Z represents oxygen atom or sulfur atom, W represents oxygen atom or nitrogen atom which may be substituted, R⁸ and R⁹ independently represents hydrogen atom, an alkyl group which may be substituted, an alkenyl group which may be substituted, an alkynyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, R¹⁰ represents an alkyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, R¹¹ represents hydrogen atom, hydroxyl group, or a group represented by O-C(=O)-R¹² or O-C(=O)-V-R¹³, R¹² and R¹³ independently represent an alkyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, U and V independently represent oxygen atom or a group represented by NH.

2. The compound or the salt thereof according to claim 1, wherein R³ is hydrogen atom.

3. The compound or the salt thereof according to claim 1 or claim 2, wherein Y is the oxy group substituted by a heterocyclic group represented by the following formula: 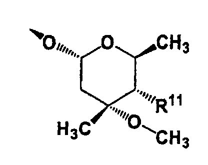 wherein R¹¹ represents hydrogen atom, hydroxyl group, or a group represented by O-C(=O)-R¹² or O-C(=O)-V-R¹³, R¹² and R¹³ independently represent an alkyl group which may be substituted, a saturated or unsaturated homocyclic group which may be substituted, a saturated or unsaturated heterocyclic group which may be substituted, an alkyl group substituted with a saturated or unsaturated homocyclic group which may be substituted, or an alkyl group substituted with a saturated or unsaturated heterocyclic group which may be substituted, V represents oxygen atom or a group represented by NH.

4. The compound or the salt thereof according to any one of claims 1 to 3, wherein R¹ is hydrogen atom.

5. A medicament comprising the compound according to any one of claims 1 to 4 or a pharmacologically acceptable salt thereof as an active ingreident.

6. The medicament according to claim 5, which is an antibacterial agent.

7. The medicament according to claim 5, which is used for therapeutic and/or preventive treatment of an atypical acid-fast mycobacteriosis.

8. A use of the compound according to any one of claims 1 to 4 or a pharmacologically acceptable salt thereof for manufacture of the medicament according to any one of claims 5 to 7.

9. A method for therapeutic treatment of an infectious disease which comprises the step of administering to a mammal including a human a therapeutically effective amount of the compound according to any one of claims 1 to 4 or a pharmacologically acceptable salt thereof.

10. The method according to claim 9, wherein the infectious disease is an atypical acid-fast mycobacteriosis.
